# EUROPÄISCHE PATENTSCHRIFT

(11) **EP 1 572 747 B1**
(45) Veröffentlichungstag und Bekanntmachung des Hinweises auf die Patenterteilung: **02.07.2008**
(21) Anmeldenummer: 03788853.4
(22) Anmeldetag: 01.12.2003
(51) Int. Cl.: C07K 16/30

(54) **TUMORSPEZIFISCHE ERKENNUNGSMOLEKÜLE**
TUMOR-SPECIFIC RECOGNITION MOLECULES
MOLECULES DE RECONNAISSANCE SPECIFIQUES DE TUMEURS

(30) Priorität: 29.11.2002 DE 10256900
(43) Veröffentlichungstag der Anmeldung: 14.09.2005
(73) Patentinhaber: Nemod Biotherapeutics GmbH & Co. KG, 13125 Berlin (DE)
(72) Erfinder: GOLETZ, Steffen, 16548 Glienicke-Nordbahn (DE); DANIELCZYK, Antje, 15752 Kolberg (DE); KARSTEN, Uwe, 16341 Panketal (DE); RAVN, Peter, 13125 Berlin (DE); STAHN, Renate, 13125 Berlin (DE); CHRISTENSEN, Peter, Astrup, 13125 Berlin (DE)
(74) Vertreter: Jaenichen, Hans-Rainer
(86) Internationale Anmeldenummer: PCT/DE2003/003994
(87) Internationale Veröffentlichungsnummer: WO 2004/050707

(56) Entgegenhaltungen:
- DE-A- 4 329 004
- KARSTEN UWE ET AL: "A New Monoclonal Antibody (A78-G/A7) to the Thomsen-Friedenreich Pan-Tumor Antigen" HYBRIDOMA, Bd. 14, Nr. 1, 1995, Seiten 37-44, XP009034408 ISSN: 0272-457X
- PRICE M R ET AL: "SUMMARY REPORT ON THE ISOBM TD-4 WORKSHOP: ANALYSIS OF 56 MONOCLONAL ANTIBODIES AGAINST THE MUC1 MUCIN" TUMOR BIOLOGY, KARGER, BASEL, CH, Bd. 19, Nr. SUPPL 1, 1998, Seiten 1-20, XP002071245 ISSN: 1010-4283
- JESCHKE U ET AL: "Expression of the Thomsen-Friedenreich antigen and of its putative carrier protein mucin 1 in the human placenta and in trophoblast cells in vitro" HISTOCHEMISTRY AND CELL BIOLOGY, Bd. 117, Nr. 3, März 2002 (2002-03), Seiten 219-226, XP002290192 ISSN: 0948-6143
- SCHNEIDER F ET AL: "Overexpression of sialyltransferase CMP-sialic acid:Galbeta1,3GalNAc-R alpha6-Sialyltransferase is related to poor patient survival in human colorectal carcinomas" CANCER RESEARCH, AMERICAN ASSOCIATION FOR CANCER RESEARCH, BALTIMORE, MD, US, Bd. 61, Nr. 11, 1. Juni 2001 (2001-06-01), Seiten 4605-4611, XP002232470 ISSN: 0008-5472

### SEQUENZPROTOKOLL

<110> Nemod Biotherapeutics GmbH & Co. KG
<120> Tumorspezifische Erkennungsmoleküle
<130> P159602PC-La
<140> PCT/DE03/03994
   <141> 2003-12-01
<150> DE 102 56 900.2
   <151> 2002-11-29
<160> 113
<170> PatentIn Ver. 2.1
<210> 1
   <211> 5
   <212> PRT
   <213> Künstliche Sequenz
<220>
   <223> Beschreibung der künstlichen Sequenz: CDR-Sequenz
<400> 1
   Asn Tyr Trp Leu Gly
    1               5
<210> 2
   <211> 17
   <212> PRT
   <213> Künstliche Sequenz
<220>
   <223> Beschreibung der künstlichen Sequenz: CDR-Sequenz
<400> 2
   Asp Ile Tyr Pro Gly Gly Gly Tyr Thr Asn Tyr Asn Glu Lys Phe Lys
   1 5 10 15
   Gly
<210> 3
   <211> 17
   <212> PRT
   <213> Künstliche Sequenz
<220>
   <223> Beschreibung der künstlichen Sequenz: CDR-Sequenz
<400> 3
   Asp Ile Tyr Pro Gly Gly Ser Tyr Thr Asn Tyr Asn Glu Lys Phe Lys
   1 5 10 15
   Gly
<210> 4
   <211> 10
   <212> PRT
   <213> Künstliche Sequenz
<220>
   <223> Beschreibung der künstlichen Sequenz: CDR-Sequenz
<400> 4
   Tyr Asp Ala Ala Gly Pro Trp Phe Ala Tyr
   1 5 10
<210> 5
   <211> 10
   <212> PRT
   <213> Künstliche Sequenz
<220>
   <223> Beschreibung der künstlichen Sequenz: CDR-Sequenz
<400> 5
   Tyr Asp Ala Ala Gly Pro Gly Phe Ala Tyr
   1 5 10
<210> 6
   <211> 8
   <212> PRT
   <213> Künstliche Sequenz
<220>
   <223> Beschreibung der künstlichen Sequenz: CDR-Sequenz
<400> 6
   Tyr Asp Asn His Tyr Phe Asp Tyr
   1 5
<210> 7
   <211> 16
   <212> PRT
   <213> Künstliche Sequenz
<220>
   <223> Beschreibung der künstlichen Sequenz: CDR-Sequenz
<400> 7
   Arg Ser Ser Gln Ser Ile Val His Ser Asn Gly Asn Thr Tyr Leu Glu
   1 5 10 15
<210> 8
   <211> 16
   <212> PRT
   <213> Künstliche Sequenz
<220>
   <223> Beschreibung der künstlichen Sequenz: CDR-Sequenz
<400> 8
   Arg Ser Ser Gln Ser Leu Leu His Ser Asn Gly Asn Thr Tyr Leu His
   1 5 10 15
<210> 9
   <211> 16
   <212> PRT
   <213> Künstliche Sequenz
<220>
   <223> Beschreibung der künstlichen Sequenz: CDR-Sequenz
<400> 9
   Lys Ser Ser Gln Ser Leu Leu His Ser Asp Gly Lys Thr Tyr Leu Tyr
   1 5 10 15
<210> 10
   <211> 7
   <212> PRT
   <213> Künstliche Sequenz
<220>
   <223> Beschreibung der künstlichen Sequenz: CDR-Sequenz '
<400> 10
   Lys Val Ser Asn Arg Phe Ser
   1 5
<210> 11
   <211> 7
   <212> PRT
   <213> Künstliche Sequenz
<220>
   <223> Beschreibung der künstlichen Sequenz: CDR-Sequenz
<400> 11
   Glu Val Ser Ser Arg Phe Ser
   1 5
<210> 12
   <211> 9
   <212> PRT
   <213> Künstliche Sequenz
<220>
   <223> Beschreibung der künstlichen Sequenz: CDR-Sequenz
<400> 12
   Phe Gln Gly Ser His Val Pro Tyr Thr
   1 5
<210> 13
   <211> 9
   <212> PRT
   <213> Künstliche Sequenz
<220>
   <223> Beschreibung der künstlichen Sequenz: CDR-Sequenz
<400> 13
   Ser Gln Ser Thr His Val Pro Tyr Thr
   1 5
<210> 14
   <211> 5
   <212> PRT
   <213> Künstliche Sequenz
<220>
   <223> Beschreibung der künstlichen Sequenz: CDR-Sequenz (canonical structure variant)
<400> 14
   Asn Tyr Trp Ile Gly
   1               5
<210> 15
   <211> 5
   <212> PRT
   <213> Künstliche Sequenz
<220>
   <223> Beschreibung der künstlichen Sequenz: CDR-Sequenz (canonical structure variant)
<400> 15
   Asn Tyr Trp Met Gly
   1               5
<210> 16
   <211> 5
   <212> PRT
   <213> Künstliche Sequenz
<220>
   <223> Beschreibung der künstlichen Sequenz: CDR-Sequenz (canonical structure variant)
<400> 16
   Asn Tyr Trp Trp Gly
   1               5
<210> 17
   <211> 5
   <212> PRT
   <213> Künstliche Sequenz
<220>
   <223> Beschreibung der künstlichen Sequenz: CDR-Sequenz (canonical structure variant)
<400> 17
   Asn Tyr Trp Val Gly
   1               5
<210> 18
   <211> 17
   <212> PRT
   <213> Künstliche Sequenz
<220>
   <223> Beschreibung der künstlichen Sequenz: CDR-Sequenz. (canonical structure variant)
<400> 18
   Asp Ile Tyr Pro Gly Gly Asp Tyr Thr Asn Tyr Asn Glu Lys Phe Lys
   1 5 10 15
   Gly
<210> 19
   <211> 17
   <212> PRT
   <213> Künstliche Sequenz
<220>
   <223> Beschreibung der künstlichen Sequenz: CDR-Sequenz (canonical structure variant)
<400> 19
   Asp Ile Tyr Pro Gly Gly Asn Tyr Thr Asn Tyr Asn Glu Lys Phe Lys
   1 5 10 15
   Gly
<210> 20
   <211> 17
   <212> PRT
   <213> Künstliche Sequenz
<220>
   <223> Beschreibung der künstlichen Sequenz: CDR-Sequenz (canonical structure variant)
<400> 20
   Asp Ile Tyr Thr Gly Gly Gly Tyr Thr Asn Tyr Asn Glu Lys Phe Lys
   1 5 10 15
   Gly
<210> 21
   <211> 17
   <212> PRT
   <213> Künstliche Sequenz
<220>
   <223> Beschreibung der künstlichen Sequenz: CDR-Sequenz (canonical structure variant)
<400> 21
   Asp Ile Tyr Thr Gly Gly Asp Tyr Thr Asn Tyr Asn Glu Lys Phe Lys
   1 5 10 15
   Gly
<210> 22
   <211> 17
   <212> PRT
   <213> Künstliche Sequenz
<220>
   <223> Beschreibung der künstlichen Sequenz: CDR-Sequenz (canonical structure variant)
<400> 22
   Asp Ile Tyr Thr Gly Gly Asn Tyr Thr Asn Tyr Asn Glu Lys Phe Lys
   1 5 10 15
   Gly
<210> 23
   <211> 17
   <212> PRT
   <213> Künstliche Sequenz
<220>
   <223> Beschreibung der künstlichen Sequenz: CDR-Sequenz (canonical structure variant)
<400> 23
   Asp Ile Tyr Thr Gly Gly Ser Tyr Thr Asn Tyr Asn Glu Lys Phe Lys
   1 5 10 15
   Gly
<210> 24
   <211> 17
   <212> PRT
   <213> Künstliche Sequenz
<220>
   <223> Beschreibung der künstlichen Sequenz: CDR-Sequenz (canonical structure variant)
<400> 24
   Asp Ile Tyr Ala Gly Gly Gly Tyr Thr Asn Tyr Asn Glu Lys Phe Lys
   1 5 10 15
   Gly
<210> 25
   <211> 17
   <212> PRT
   <213> Künstliche Sequenz
<220>
   <223> Beschreibung der künstlichen Sequenz: CDR-Sequenz (canonical structure variant)
<400> 25
   Asp Ile Tyr Ala Gly Gly Asp Tyr Thr Asn Tyr Asn Glu Lys Phe Lys
   1 5 10 15
   Gly
<210> 26
   <211> 17
   <212> PRT
   <213> Künstliche Sequenz
<220>
   <223> Beschreibung der künstlichen Sequenz: CDR-Sequenz (canonical structure variant)
<400> 26
   Asp Ile Tyr Ala Gly Gly Asp Tyr Thr Asn Tyr Asn Glu Lys Phe Lys
   1 5 10 15
   Gly
<210> 27
   <211> 17
   <212> PRT
   <213> Künstliche Sequenz
<220>
   <223> Beschreibung der künstlichen Sequenz: CDR-Sequenz (canonical structure variant)
<400> 27
   Asp Ile Tyr Ala Gly Gly Ser Tyr Thr Asn Tyr Asn Glu Lys Phe Lys
   1 5 10 15
   Gly
<210> 28
   <211> 16
   <212> PRT
   <213> Künstliche Sequenz
<220>
   <223> Beschreibung der künstlichen Sequenz: CDR-Sequenz (canonical structure variant)
<400> 28
   Arg Pro Ser Gln Ser Ile Val His Ser Asn Gly Asn Thr Tyr Leu Glu
   1 5 10 15
<210> 29
   <211> 16
   <212> PRT
   <213> Künstliche Sequenz
<220>
   <223> Beschreibung der künstlichen Sequenz: CDR-Sequenz (canonical structure variant)
<400> 29
   Arg Pro Ser Gln Ser Ile Val His Ser Asn Gly Asn Thr Tyr Leu Glu
   1 5 10 15
<210> 30
   <211> 16
   <212> PRT
   <213> Künstliche Sequenz
<220>
   <223> Beschreibung der künstlichen Sequenz: CDR-Sequenz (canonical structure variant)
<400> 30
   Arg Ser Ser Gln Ser Ile Val His Ser Asn Gly Asn Thr Tyr Phe Glu
   1 5 10 15
<210> 31
   <211> 16
   <212> PRT
   <213> Künstliche Sequenz
<220>
   <223> Beschreibung der künstlichen Sequenz: CDR-Sequenz (canonical structure variant)
<400> 31
   Arg Pro Ser Gln Ser Leu Val His Ser Asn Gly Asn Thr Tyr Leu Glu
   1               5                   10                  15
<210> 32
   <211> 16
   <212> PRT
   <213> Künstliche Sequenz
<220>
   <223> Beschreibung der künstlichen Sequenz: CDR-Sequenz (canonical structure variant)
<400> 32
   Arg Pro Ser Gln Ser Ile Val His Ser Asn Gly Asn Thr Tyr Phe Glu
   1               5                   10                  15
<210> 33
   <211> 16
   <212> PRT
   <213> Künstliche Sequenz
<220>
   <223> Beschreibung der künstlichen Sequenz: CDR-Sequenz (canonical structure variant)
<400> 33
   Arg Ser Ser Gln Ser Leu Val His Ser Asn Gly Asn Thr Tyr Phe Glu
   1               5                   10                  15
<210> 34
   <211> 16
   <212> PRT
   <213> Künstliche Sequenz
<220>
   <223> Beschreibung der künstlichen Sequenz: CDR-Sequenz (canonical structure variant)
<400> 34
   Arg Pro Ser Gln Ser Leu Leu His Ser Asn Gly Asn Thr Tyr Leu His
   1               5                   10                  15
<210> 35
   <211> 16
   <212> PRT
   <213> Künstliche Sequenz
<220>
   <223> Beschreibung der künstlichen Sequenz: CDR-Sequenz (canonical structure variant)
<400> 35
   Arg Ser Ser Gln Ser Ile Leu His Ser Asn Gly Asn Thr Tyr Leu His
   1 5 10 15
<210> 36
   <211> 16
   <212> PRT
   <213> Künstliche Sequenz
<220>
   <223> Beschreibung der künstlichen Sequenz: CDR-Sequenz (canonical structure variant)
<400> 36
   Arg Ser Ser Gln Ser Leu Leu His Ser Asn Gly Asn Thr Tyr Phe His
   1 5 10 15
<210> 37
   <211> 16
   <212> PRT
   <213> Künstliche Sequenz
<220>
   <223> Beschreibung der künstlichen Sequenz: CDR-Sequenz (canonical structure variant)
<400> 37
   Arg Pro Ser Gln Ser Ile Leu His Ser Asn Gly Asn Thr Tyr Leu His
   1 5 10 15
<210> 38
   <211> 16
   <212> PRT
   <213> Künstliche Sequenz
<220>
   <223> Beschreibung der künstlichen Sequenz: CDR-Sequenz (canonical structure variant)
<400> 38
   Arg Pro Ser Gln Ser Leu Leu His Ser Asn Gly Asn Thr Tyr Phe His
   1 5 10 15
<210> 39
   <211> 16
   <212> PRT
   <213> Künstliche Sequenz
<220>
   <223> Beschreibung der künstlichen Sequenz: CDR-Sequenz (canonical structure variant)
<400> 39
   Arg Ser Ser Gln Ser Ile Leu His Ser Asn Gly Asn Thr Tyr Phe His
   1               5                   10                  15
<210> 40
   <211> 16
   <212> PRT
   <213> Künstliche Sequenz
<220>
   <223> Beschreibung der künstlichen Sequenz: CDR-Sequenz (canonical structure variant)
<400> 40
   Lys Pro Ser Gln Ser Leu Leu His Ser Asp Gly Lys Thr Tyr Leu Tyr
   1               5                   10                  15
<210> 41
   <211> 16
   <212> PRT
   <213> Künstliche Sequenz
<220>
   <223> Beschreibung der künstlichen Sequenz: CDR-Sequenz (canonical structure variant)
<400> 41
   Lys Ser Ser Gln Ser Ile Leu His Ser Asp Gly Lys Thr Tyr Leu Tyr
   1               5                   10                  15
<210> 42
   <211> 16
   <212> PRT
   <213> Künstliche Sequenz
<220>
   <223> Beschreibung der künstlichen Sequenz: CDR-Sequenz (canonical structure variant)
<400> 42
   Lys Ser Ser Gln Ser Leu Leu His Ser Asp Gly Lys Thr Tyr Phe Tyr
   1 5 10 15
<210> 43
   <211> 16
   <212> PRT
   <213> Künstliche Sequenz
<220>
   <223> Beschreibung der künstlichen Sequenz: CDR-Sequenz (canonical structure variant)
<400> 43
   Lys Pro Ser Gln Ser Ile Leu His Ser Asp Gly Lys Thr Tyr Leu Tyr
   1 5 10 15
<210> 44
   <211> 16
   <212> PRT
   <213> Künstliche Sequenz
<220>
   <223> Beschreibung der künstlichen Sequenz: CDR-Sequenz (canonical structure variant)
<400> 44
   Lys Pro Ser Gln Ser Ile Leu His Ser Asp Gly Lys Thr Tyr Leu Tyr
   1 5 10 15
<210> 45
   <211> 16
   <212> PRT
   <213> Künstliche Sequenz
<220>
   <223> Beschreibung der künstlichen Sequenz: CDR-Sequenz (canonical structure variant)
<400> 45
   Lys Ser Ser Gln Ser Ile Leu His Ser Asp Gly Lys Thr Tyr Phe Tyr
   1 5 10 15
<210> 46
   <211> 119
   <212> PRT
   <213> Künstliche Sequenz
<220>
   <223> Beschreibung der künstlichen Sequenz: variable schwere Ketten VH
<400> 46
   Gln Val Gln Leu Lys Glu Ser Gly Ala Glu Leu Val Arg Pro Gly Thr
    1               5                  10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser
           115
<210> 47
   <211> 117
   <212> PRT
   <213> Künstliche Sequenz
<220>
   <223> Beschreibung der künstlichen Sequenz: variable schwere Ketten VH
<400> 47
   Gln Val Gln Leu Lys Gln Ser Gly Ala Glu Leu Val Arg Pro Gly Thr
    1               5                  10                  15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Ser Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Tyr Asp Asn His Tyr Phe Asp Tyr Trp Gly Gln Gly Thr Thr
   100 105 110
   Leu Thr Val Ser Ser
   115
<210> 48
   <211> 119
   <212> PRT
   <213> Künstliche Sequenz
<220>
   <223> Beschreibung der künstlichen Sequenz: variable schwere Ketten VH
<400> 48
   Gln Val Gln Leu Lys Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Leu Thr Val Ser Ser
   115
<210> 49
   <211> 119
   <212> PRT
   <213> Künstliche Sequenz
<220>
   <223> Beschreibung der künstlichen Sequenz: variable schwere Ketten VH
<400> 49
   Glu Val Lys Leu Val Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Ser Val Thr Val Ser Ser
   115
<210> 50
   <211> 119
   <212> PRT
   <213> Künstliche Sequenz
<220>
   <223> Beschreibung der künstlichen Sequenz: variable schwere Ketten VH
<400> 50
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 51
   <211> 119
   <212> PRT
   <213> Künstliche Sequenz
<220>
   <223> Beschreibung der künstlichen Sequenz: variable schwere Ketten VH
<400> 51
   Glu Val Lys Leu Val Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 52
   <211> 119
   <212> PRT
   <213> Künstliche Sequenz
<220>
   <223> Beschreibung der künstlichen Sequenz: variable schwere Ketten VH
<400> 52
   Gln Val Gln Leu Lys Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ala
   115
<210> 53
   <211> 119
   <212> PRT
   <213> Künstliche Sequenz
<220>
   <223> Beschreibung der künstlichen Sequenz: variable schwere Ketten VH
<400> 53
   Gln Val Gln Leu Lys Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 54
   <211> 119
   <212> PRT
   <213> Künstliche Sequenz
<220>
   <223> Beschreibung der künstlichen Sequenz: variable schwere Ketten VH
<400> 54
   Gln Val Thr Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Ser Val Thr Val Ser Ser
   115
<210> 55
   <211> 119
   <212> PRT
   <213> Künstliche Sequenz
<220>
   <223> Beschreibung der künstlichen Sequenz: variable schwere Ketten VH
<400> 55
   Gln Val Gln Leu Lys Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Ser Val Thr Val Ser Ser
   115
<210> 56
   <211> 119
   <212> PRT
   <213> Künstliche Sequenz
<220>
   <223> Beschreibung der künstlichen Sequenz: variable schwere Ketten VH
<400> 56
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 57
   <211> 119
   <212> PRT
   <213> Künstliche Sequenz
<220>
   <223> Beschreibung der künstlichen Sequenz: variable schwere Ketten VH
<400> 57
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 58
   <211> 119
   <212> PRT
   <213> Künstliche Sequenz
<220>
   <223> Beschreibung der künstlichen Sequenz: variable schwere Ketten VH
<400> 58
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Arg Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 59
   <211> 119
   <212> PRT
   <213> Künstliche Sequenz
<220>
   <223> Beschreibung der künstlichen Sequenz: variable schwere Ketten VH
<400> 59
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Glu Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 60
   <211> 119
   <212> PRT
   <213> Künstliche Sequenz
<220>
   <223> Beschreibung der künstlichen Sequenz: variable schwere Ketten VH
<400> 60
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Lys Gln Arg Pro Gly Gln Gly Leu Glu Arg Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 61
   <211> 119
   <212> PRT
   <213> Künstliche Sequenz
<220>
   <223> Beschreibung der künstlichen Sequenz: variable schwere Ketten VH
<400> 61
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 62
   <211> 119
   <212> PRT
   <213> Künstliche Sequenz
<220>
   <223> Beschreibung der künstlichen Sequenz: variable schwere Ketten VH
<400> 62
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 63
   <211> 119
   <212> PRT
   <213> Künstliche Sequenz
<220>
   <223> Beschreibung der künstlichen Sequenz: variable schwere Ketten VH
<400> 63
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 64 ,
   <211> 119
   <212> PRT
   <213> Künstliche Sequenz
<220>
   <223> Beschreibung der künstlichen Sequenz: variable schwere Ketten VH
<400> 64
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Thr Ser Glu Asp Ser Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 65
   <211> 119
   <212> PRT
   <213> Künstliche Sequenz
<220>
   <223> Beschreibung der künstlichen Sequenz: variable schwere Ketten VH
<400> 65
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 66
   <211> 119
   <212> PRT
   <213> Künstliche Sequenz
<220>
   <223> Beschreibung der künstlichen Sequenz: variable schwere Ketten VH
<400> 66
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 67
   <211> 119
   <212> PRT
   <213> Künstliche Sequenz
<220>
   <223> Beschreibung der künstlichen Sequenz: variable schwere Ketten VH
<400> 67
   Gln Val Gln Leu Val Gln Ser Gly Ala Glu Val Lys Lys Pro Gly Ala
   1 5 10 15
   Ser Val Lys Val Pro Cys Lys Ala Ser Gly Tyr Thr Phe Thr Asn Tyr
   20 25 30
   Trp Leu Gly Trp Val Lys Gln Arg Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 68
   <211> 119
   <212> PRT
   <213> Künstliche Sequenz
<220>
   <223> Beschreibung der künstlichen Sequenz: variable schwere Ketten VH
<400> 68
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Lys Gln Arg Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 69
   <211> 119
   <212> PRT
   <213> Künstliche Sequenz
<220>
   <223> Beschreibung der künstlichen Sequenz: variable schwere Ketten VH
<400> 69
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 70
   <211> 119
   <212> PRT
   <213> Künstliche Sequenz
<220>
   <223> Beschreibung der künstlichen Sequenz: variable schwere Ketten VH
<400> 70
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 71
   <211> 119
   <212> PRT
   <213> Künstliche Sequenz
<220>
   <223> Beschreibung der künstlichen Sequenz: variable schwere Ketten VH
<400> 71
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Lys Gln Arg Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Thr Ser Glu Asp Ser Ala Val Tyr Tyr Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 72
   <211> 119
   <212> PRT
   <213> Künstliche Sequenz
<220>
   <223> Beschreibung der künstlichen Sequenz: variable schwere Ketten VH
<400> 72
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 73
   <211> 119
   <212> PRT
   <213> Künstliche Sequenz
<220>
   <223> Beschreibung der künstlichen Sequenz: variable schwere Ketten VH
<400> 73
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Tyr Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 74
   <211> 119
   <212> PRT
   <213> Künstliche Sequenz
<220>
   <223> Beschreibung der künstlichen Sequenz: variable schwere Ketten VH
<400> 74
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Thr Ser Thr Ala Tyr
   65 70 75 80
   Met Glu Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Ser
   115
<210> 75
   <211> 119
   <212> PRT
   <213> Künstliche Sequenz
<220>
   <223> Beschreibung der künstlichen Sequenz: variable schwere Ketten VH
<400> 75
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Arg Val Thr Ile Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 76
   <211> 119
   <212> PRT
   <213> Künstliche Sequenz
<220>
   <223> Beschreibung der künstlichen Sequenz: variable schwere Ketten VH
<400> 76
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Arg Val Thr Ile Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 77
   <211> 119
   <212> PRT
   <213> Künstliche Sequenz
<220>
   <223> Beschreibung der künstlichen Sequenz: variable schwere Ketten VH
<400> 77
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Lys Gln Arg Pro Gly Gln Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Arg Val Thr Ile Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 78
   <211> 119
   <212> PRT
   <213> Künstliche Sequenz
<220>
   <223> Beschreibung der künstlichen Sequenz: variable schwere Ketten VH
<400> 78
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Trp Leu Gly Trp Val Arg Gln Ala Pro Gly Gln Gly Leu Glu Trp Met
   35 40 45
   Gly Asp Ile Tyr Pro Gly Gly Gly Tyr Thr Asn Tyr Asn Glu Lys Phe
   50 55 60
   Lys Gly Arg Val Thr Ile Thr Ala Asp Thr Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Tyr Tyr Asp Ala Ala Gly Pro Trp Phe Ala Tyr Trp Gly Gln Gly
   100 105 110
   Thr Leu Val Thr Val Ser Ser
   115
<210> 79
   <211> 119
   <212> PRT
   <213> Künstliche Sequenz
<220>
   <223> Beschreibung der künstlichen Sequenz: variable schwere Ketten VH
<400> 79
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Met
           35                  40                  45
   Gly Asp Ile Tyr Pro Gly Gly Gly Tyr Thr Asn Tyr Asn Glu Lys Phe
       50                  55                  60
   Lys Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 80
   <211> 114
   <212> PRT
   <213> Künstliche Sequenz
<220>
   <223> Beschreibung der künstlichen Sequenz: variable leichte Ketten
<400> 80
   Asp Ile Gln Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg Ala
<210> 81
   <211> 114
   <212> PRT
   <213> Künstliche Sequenz
<220>
   <223> Beschreibung der künstlichen Sequenz: variable leichte Ketten
<400> 81
   Asp Ile Val Ile Thr Gln Thr Pro Leu Ser Leu Pro Val Ser Leu Gly
   1 5 10 15
   Asp Gln Ala Ser Ile Ser Cys Arg Ser Ser Gln Ser Leu Leu His Ser
   20 25 30
   Asn Gly Asn Thr Tyr Leu His Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Phe Cys Ser Gln Ser
   85 90 95
   Thr His Val Pro Tyr Thr Phe Gly Gly Gly Thr Lys Leu Glu Ile Lys
   100 105 110
   Arg Ala
<210> 82
   <211> 114
   <212> PRT
   <213> Künstliche Sequenz
<220>
   <223> Beschreibung der künstlichen Sequenz: variable leichte Ketten
<400> 82
   Asp Ile Gln Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Leu Lys
   100 105 110
   Arg Ala
<210> 83
   <211> 114
   <212> PRT
   <213> Künstliche Sequenz
<220>
   <223> Beschreibung der künstlichen Sequenz: variable leichte Ketten
<400> 83
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg Ala
<210> 84
   <211> 114
   <212> PRT
   <213> Künstliche Sequenz
<220>
   <223> Beschreibung der künstlichen Sequenz: variable leichte Ketten
<400> 84
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Leu Lys
   100 105 110
   Arg Ala
<210> 85
   <211> 114
   <212> PRT
   <213> Künstliche Sequenz
<220>
   <223> Beschreibung der künstlichen Sequenz: variable leichte Ketten
<400> 85
   Asp Ile Gln Met Thr Gln Ser Pro Leu Ser Leu Pro Val Thr Pro Gly
   1 5 10 15
   Glu Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gly Gly Thr Lys Val Glu Ile Lys
   100 105 110
   Arg Ala
<210> 86
   <211> 114
   <212> PRT
   <213> Künstliche Sequenz
<220>
   <223> Beschreibung der künstlichen Sequenz: variable leichte Ketten
<400> 86
   Asp Ile Gln Met Thr Gln Ser Pro Leu Ser Leu Pro Val Thr Pro Gly
   1 5 10 15
   Glu Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 87
   <211> 114
   <212> PRT
   <213> Künstliche Sequenz
<220>
   <223> Beschreibung der künstlichen Sequenz: variable leichte Ketten
<400> 87
   Asp Ile Gln Met Thr Gln Ser Pro Leu Ser Leu Pro Val Thr Pro Gly
   1 5 10 15
   Glu Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gly Gly Thr Lys Val Glu Ile Lys
   100 105 110
   Arg Ala
<210> 88
   <211> 114
   <212> PRT
   <213> Künstliche Sequenz
<220>
   <223> Beschreibung der künstlichen Sequenz: variable leichte Ketten
<400> 88
   Asp Ile Gln Met Thr Gln Ser Pro Leu Ser Leu Pro Val Thr Pro Gly
   1 5 10 15
   Glu Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 89
   <211> 114
   <212> PRT
   <213> Künstliche Sequenz
<220>
   <223> Beschreibung der künstlichen Sequenz: variable leichte Ketten
<400> 89
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gly Gly Thr Lys Val Glu Ile Lys
   100 105 110
   Arg Ala
<210> 90
   <211> 114
   <212> PRT
   <213> Künstliche Sequenz
<220>
   <223> Beschreibung der künstlichen Sequenz: variable leichte Ketten
<400> 90
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 91
   <211> 114
   <212> PRT
   <213> Künstliche Sequenz
<220>
   <223> Beschreibung der künstlichen Sequenz: variable leichte Ketten
<400> 91
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gly Gly Thr Lys Val Glu Ile Lys
               100                 105                 110
   Arg Ala
<210> 92
   <211> 114
   <212> PRT
   <213> Künstliche Sequenz
<220>
   <223> Beschreibung der künstlichen Sequenz: variable leichte Ketten
<400> 92
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 93
   <211> 114
   <212> PRT
   <213> Künstliche Sequenz
<220>
   <223> Beschreibung der künstlichen Sequenz: variable leichte Ketten
<400> 93
   Asp Ile Val Met Thr Gln Thr Pro Leu Ser Leu Pro Val Thr Pro Gly
   1 5 10 15
   Gln Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 94
   <211> 114
   <212> PRT
   <213> Künstliche Sequenz
<220>
   <223> Beschreibung der künstlichen Sequenz: variable leichte Ketten
<400> 94
   Asp Ile Val Met Thr Gln Thr Pro Leu Ser Leu Ser Val Thr Pro Gly
   1               5                   10                  15
   Gln Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 95
   <211> 276
   <212> PRT
   <213> Künstliche Sequenz
<220>
   <223> Beschreibung der künstlichen Sequenz: single chain Fv Formate
<400> 95
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Gly Gly Gly Gly Ser Gly
   115 120 125
   Gly Gly Gly Ser Gly Gly Ser Ala Arg Asp Ile Gln Met Thr Gln Thr
   130 135 140
   Pro Leu Ser Leu Pro Val Ser Leu Gly Asp Gln Ala Ser Ile Ser Cys
   145 150 155 160
   Arg Ser Ser Gln Ser Ile Val His Ser Asn Gly Asn Thr Tyr Leu Glu
   165 170 175
   Trp Tyr Leu Gln Lys Pro Gly Gln Ser Pro Lys Leu Leu Ile Tyr Lys
   180 185 190
   Val Ser Asn Arg Phe Ser Gly Val Pro Asp Arg Phe Ser Gly Ser Gly
   195 200 205
   Ser Gly Thr Asp Phe Thr Leu Lys Ile Ser Arg Val Glu Ala Glu Asp
   210 215 220
   Leu Gly Val Tyr Tyr Cys Phe Gln Gly Ser His Val Pro Tyr Thr Phe
   225 230 235 240
   Gly Gly Gly Thr Lys Leu Glu Ile Lys Arg Ala Ala Ala His His His
   245 250 255
   His His His Gly Ala Ala Glu Gln Lys Leu Ile Ser Glu Glu Asp Leu
   260 265 270
   Asn Gly Ala Ala
   275
<210> 96
   <211> 267
   <212> PRT
   <213> Künstliche Sequenz
<220>
   <223> Beschreibung der künstlichen Sequenz: single chain Fv Formate
<400> 96
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Gly Ser Gly Ser Ser Ala
   115 120 125
   Asp Ile Gln Met Thr Gln Thr Pro Leu Ser Leu Pro Val Ser Leu Gly
   130 135 140
   Asp Gln Ala Ser Ile Ser Cys Arg Ser Ser Gln Ser Ile Val His Ser
   145 150 155 160
   Asn Gly Asn Thr Tyr Leu Glu Trp Tyr Leu Gln Lys Pro Gly Gln Ser
   165 170 175
   Pro Lys Leu Leu Ile Tyr Lys Val Ser Asn Arg Phe Ser Gly Val Pro
   180 185 190
   Asp Arg Phe Ser Gly Ser Gly Ser Gly Thr Asp Phe Thr Leu Lys Ile
   195 200 205
   Ser Arg Val Glu Ala Glu Asp Leu Gly Val Tyr Tyr Cys Phe Gln Gly
   210 215 220
   Ser His Val Pro Tyr Thr Phe Gly Gly Gly Thr Lys Leu Glu Ile Lys
   225 230 235 240
   Arg Ala Ala Ala His His His His His His Gly Ala Ala Glu Gln Lys
   245 250 255
   Leu Ile Ser Glu Glu Asp Leu Asn Gly Ala Ala
   260 265
<210> 97
   <211> 266
   <212> PRT
   <213> Künstliche Sequenz
<220>
   <223> Beschreibung der künstlichen Sequenz: single chain Fv Formate
<400> 97
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Gly Gly Ser Ser Ala Asp
   115 120 125
   Ile Gln Met Thr Gln Thr Pro Leu Ser Leu Pro Val Ser Leu Gly Asp
   130 135 140
   Gln Ala Ser Ile Ser Cys Arg Ser Ser Gln Ser Ile Val His Ser Asn
   145 150 155 160
   Gly Asn Thr Tyr Leu Glu Trp Tyr Leu Gln Lys Pro Gly Gln Ser Pro
   165 170 175
   Lys Leu Leu Ile Tyr Lys Val Ser Asn Arg Phe Ser Gly Val Pro Asp
   180 185 190
   Arg Phe Ser Gly Ser Gly Ser Gly Thr Asp Phe Thr Leu Lys Ile Ser
   195 200 205
   Arg Val Glu Ala Glu Asp Leu Gly Val Tyr Tyr Cys Phe Gln Gly Ser
   210 215 220
   His Val Pro Tyr Thr Phe Gly Gly Gly Thr Lys Leu Glu Ile Lys Arg
   225 230 235 240
   Ala Ala Ala His His His His His His Gly Ala Ala Glu Gln Lys Leu
   245 250 255
   Ile Ser Glu Glu Asp Leu Asn Gly Ala Ala
   260 265
<210> 98
   <211> 265
   <212> PRT
   <213> Künstliche Sequenz
<220>
   <223> Beschreibung der künstlichen Sequenz: single chain Fv Formate
<400> 98
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Gly Ser Ser Ala Asp Ile
   115 120 125
   Gln Met Thr Gln Thr Pro Leu Ser Leu Pro Val Ser Leu Gly Asp Gln
   130 135 140
   Ala Ser Ile Ser Cys Arg Ser Ser Gln Ser Ile Val His Ser Asn Gly
   145 150 155 160
   Asn Thr Tyr Leu Glu Trp Tyr Leu Gln Lys Pro Gly Gln Ser Pro Lys
   165 170 175
   Leu Leu Ile Tyr Lys Val Ser Asn Arg Phe Ser Gly Val Pro Asp Arg
   180 185 190
   Phe Ser Gly Ser Gly Ser Gly Thr Asp Phe Thr Leu Lys Ile Ser Arg
   195 200 205
   Val Glu Ala Glu Asp Leu Gly Val Tyr Tyr Cys Phe Gln Gly Ser His
   210 215 220
   Val Pro Tyr Thr Phe Gly Gly Gly Thr Lys Leu Glu Ile Lys Arg Ala
   225 230 235 240
   Ala Ala His His His His His His Gly Ala Ala Glu Gln Lys Leu Ile
   245 250 255
   Ser Glu Glu Asp Leu Asn Gly Ala Ala
   260 265
<210> 99
   <211> 264
   <212> PRT
   <213> Künstliche Sequenz
<220>
   <223> Beschreibung der künstlichen Sequenz: single chain Fv Formate
<400> 99
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Ser Ser Ala Asp Ile Gln
   115 120 125
   Met Thr Gln Thr Pro Leu Ser Leu Pro Val Ser Leu Gly Asp Gln Ala
   130 135 140
   Ser Ile Ser Cys Arg Ser Ser Gln Ser Ile Val His Ser Asn Gly Asn
   145 150 155 160
   Thr Tyr Leu Glu Trp Tyr Leu Gln Lys Pro Gly Gln Ser Pro Lys Leu
   165 170 175
   Leu Ile Tyr Lys Val Ser Asn Arg Phe Ser Gly Val Pro Asp Arg Phe
   180 185 190
   Ser Gly Ser Gly Ser Gly Thr Asp Phe Thr Leu Lys Ile Ser Arg Val
   195 200 205
   Glu Ala Glu Asp Leu Gly Val Tyr Tyr Cys Phe Gln Gly Ser His Val
   210 215 220
   Pro Tyr Thr Phe Gly Gly Gly Thr Lys Leu Glu Ile Lys Arg Ala Ala
   225 230 235 240
   Ala His His His His His His Gly Ala Ala Glu Gln Lys Leu Ile Ser
   245 250 255
   Glu Glu Asp Leu Asn Gly Ala Ala
   260
<210> 100
   <211> 263
   <212> PRT
   <213> Künstliche Sequenz
<220>
   <223> Beschreibung der künstlichen Sequenz: single chain Fv Formate
<400> 100
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Ser Ala Asp Ile Gln Met
   115 120 125
   Thr Gln Thr Pro Leu Ser Leu Pro Val Ser Leu Gly Asp Gln Ala Ser
   130 135 140
   Ile Ser Cys Arg Ser Ser Gln Ser Ile Val His Ser Asn Gly Asn Thr
   145 150 155 160
   Tyr Leu Glu Trp Tyr Leu Gln Lys Pro Gly Gln Ser Pro Lys Leu Leu
   165 170 175
   Ile Tyr Lys Val Ser Asn Arg Phe Ser Gly Val Pro Asp Arg Phe Ser
   180 185 190
   Gly Ser Gly Ser Gly Thr Asp Phe Thr Leu Lys Ile Ser Arg Val Glu
   195 200 205
   Ala Glu Asp Leu Gly Val Tyr Tyr Cys Phe Gln Gly Ser His Val Pro
   210 215 220
   Tyr Thr Phe Gly Gly Gly Thr Lys Leu Glu Ile Lys Arg Ala Ala Ala
   225 230 235 240
   His His His His His His Gly Ala Ala Glu Gln Lys Leu Ile Ser Glu
   245 250 255
   Glu Asp Leu Asn Gly Ala Ala
   260
<210> 101
   <211> 262
   <212> PRT
   <213> Künstliche Sequenz
<220>
   <223> Beschreibung der künstlichen Sequenz: single chain Fv Formate
<400> 101
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Ala Asp Ile Gln Met Thr
   115 120 125
   Gln Thr Pro Leu Ser Leu Pro Val Ser Leu Gly Asp Gln Ala Ser Ile
   130 135 140
   Ser Cys Arg Ser Ser Gln Ser Ile Val His Ser Asn Gly Asn Thr Tyr
   145 150 155 160
   Leu Glu Trp Tyr Leu Gln Lys Pro Gly Gln Ser Pro Lys Leu Leu Ile
   165 170 175
   Tyr Lys Val Ser Asn Arg Phe Ser Gly Val Pro Asp Arg Phe Ser Gly
   180 185 190
   Ser Gly Ser Gly Thr Asp Phe Thr Leu Lys Ile Ser Arg Val Glu Ala
   195 200 205
   Glu Asp Leu Gly Val Tyr Tyr Cys Phe Gln Gly Ser His Val Pro Tyr
   210 215 220
   Thr Phe Gly Gly Gly Thr Lys Leu Glu Ile Lys Arg Ala Ala Ala His
   225 230 235 240
   His His His His His Gly Ala Ala Glu Gln Lys Leu Ile Ser Glu Glu
   245 250 255
   Asp Leu Asn Gly Ala Ala
   260
<210> 102
   <211> 261
   <212> PRT
   <213> Künstliche Sequenz
<220>
   <223> Beschreibung der künstlichen Sequenz: single chain Fv Formate
<400> 102
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ala Asp Ile Gln Met Thr Gln
           115                 120                 125
   Thr Pro Leu Ser Leu Pro Val Ser Leu Gly Asp Gln Ala Ser Ile Ser
       130                 135                 140
   Cys Arg Ser Ser Gln Ser Ile Val His Ser Asn Gly Asn Thr Tyr Leu
   145                 150                 155                 160
   Glu Trp Tyr Leu Gln Lys Pro Gly Gln Ser Pro Lys Leu Leu Ile Tyr
                   165                 170                 175
   Lys Val Ser Asn Arg Phe Ser Gly Val Pro Asp Arg Phe Ser Gly Ser
               180                 185                 190
   Gly Ser Gly Thr Asp Phe Thr Leu Lys Ile Ser Arg Val Glu Ala Glu
           195                 200                 205
   Asp Leu Gly Val Tyr Tyr Cys Phe Gln Gly Ser His Val Pro Tyr Thr
       210                 215                 220
   Phe Gly Gly Gly Thr Lys Leu Glu Ile Lys Arg Ala Ala Ala His His
   225                 230                 235                 240
   His His His His Gly Ala Ala Glu Gln Lys Leu Ile Ser Glu Glu Asp
                   245                 250                 255
   Leu Asn Gly Ala Ala
   260
<210> 103
   <211> 260
   <212> PRT
   <213> Künstliche Sequenz
<220>
   <223> Beschreibung der künstlichen Sequenz: single chain Fv Formate
<400> 103
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ala Asp Ile Gln Met Thr Gln Thr
   115 120 125
   Pro Leu Ser Leu Pro Val Ser Leu Gly Asp Gln Ala Ser Ile Ser Cys
   130 135 140
   Arg Ser Ser Gln Ser Ile Val His Ser Asn Gly Asn Thr Tyr Leu Glu
   145 150 155 160
   Trp Tyr Leu Gln Lys Pro Gly Gln Ser Pro Lys Leu Leu Ile Tyr Lys
   165 170 175
   Val Ser Asn Arg Phe Ser Gly Val Pro Asp Arg Phe Ser Gly Ser Gly
   180 185 190
   Ser Gly Thr Asp Phe Thr Leu Lys Ile Ser Arg Val Glu Ala Glu Asp
   195 200 205
   Leu Gly Val Tyr Tyr Cys Phe Gln Gly Ser His Val Pro Tyr Thr Phe
   210 215 220
   Gly Gly Gly Thr Lys Leu Glu Ile Lys Arg Ala Ala Ala His His His
   225 230 235 240
   His His His Gly Ala Ala Glu Gln Lys Leu Ile Ser Glu Glu Asp Leu
   245 250 255
   Asn Gly Ala Ala
   260
<210> 104
   <211> 259
   <212> PRT
   <213> Künstliche Sequenz
<220>
   <223> Beschreibung der künstlichen Sequenz: single chain Fv Formate
<400> 104
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Asp Ile Gln Met Thr Gln Thr Pro
   115 120 125
   Leu Ser Leu Pro Val Ser Leu Gly Asp Gln Ala Ser Ile Ser Cys Arg
   130 135 140
   Ser Ser Gln Ser Ile Val His Ser Asn Gly Asn Thr Tyr Leu Glu Trp
   145 150 155 160
   Tyr Leu Gln Lys Pro Gly Gln Ser Pro Lys Leu Leu Ile Tyr Lys Val
   165 170 175
   Ser Asn Arg Phe Ser Gly Val Pro Asp Arg Phe Ser Gly Ser Gly Ser
   180 185 190
   Gly Thr Asp Phe Thr Leu Lys Ile Ser Arg Val Glu Ala Glu Asp Leu
   195 200 205
   Gly Val Tyr Tyr Cys Phe Gln Gly Ser His Val Pro Tyr Thr Phe Gly
   210 215 220
   Gly Gly Thr Lys Leu Glu Ile Lys Arg Ala Ala Ala His His His His
   225 230 235 240
   His His Gly Ala Ala Glu Gln Lys Leu Ile Ser Glu Glu Asp Leu Asn
   245 250 255
   Gly Ala Ala
<210> 105
   <211> 258
   <212> PRT
   <213> Künstliche Sequenz
<220>
   <223> Beschreibung der künstlichen Sequenz: single chain Fv Formate
<400> 105
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Asp Ile Gln Met Thr Gln Thr Pro Leu
   115 120 125
   Ser Leu Pro Val Ser Leu Gly Asp Gln Ala Ser Ile Ser Cys Arg Ser
   130 135 140
   Ser Gln Ser Ile Val His Ser Asn Gly Asn Thr Tyr Leu Glu Trp Tyr
   145 150 155 160
   Leu Gln Lys Pro Gly Gln Ser Pro Lys Leu Leu Ile Tyr Lys Val Ser
   165 170 175
   Asn Arg Phe Ser Gly Val Pro Asp Arg Phe Ser Gly Ser Gly Ser Gly
   180 185 190
   Thr Asp Phe Thr Leu Lys Ile Ser Arg Val Glu Ala Glu Asp Leu Gly
   195 200 205
   Val Tyr Tyr Cys Phe Gln Gly Ser His Val Pro Tyr Thr Phe Gly Gly
   210 215 220
   Gly Thr Lys Leu Glu Ile Lys Arg Ala Ala Ala His His His His His
   225 230 235 240
   His Gly Ala Ala Glu Gln Lys Leu Ile Ser Glu Glu Asp Leu Asn Gly
   245 250 255
   Ala Ala
<210> 106
   <211> 257
   <212> PRT
   <213> Künstliche Sequenz
<220>
   <223> Beschreibung der künstlichen Sequenz: single chain Fv Formate
<400> 106
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Asp Ile Gln Met Thr Gln Thr Pro Leu Ser
           115                 120                 125
   Leu Pro Val Ser Leu Gly Asp Gln Ala Ser Ile Ser Cys Arg Ser Ser
       130                 135                 140
   Gln Ser Ile Val His Ser Asn Gly Asn Thr Tyr Leu Glu Trp Tyr Leu
   145                 150                 155                 160
   Gln Lys Pro Gly Gln Ser Pro Lys Leu Leu Ile Tyr Lys Val Ser Asn
                   165                 170                 175
   Arg Phe Ser Gly Val Pro Asp Arg Phe Ser Gly Ser Gly Ser Gly Thr
               180                 185                 190
   Asp Phe Thr Leu Lys Ile Ser Arg Val Glu Ala Glu Asp Leu Gly Val
           195                 200                 205
   Tyr Tyr Cys Phe Gln Gly Ser His Val Pro Tyr Thr Phe Gly Gly Gly
   210 215 220
   Thr Lys Leu Glu Ile Lys Arg Ala Ala Ala His His His His His His
   225 230 235 240
   Gly Ala Ala Glu Gln Lys Leu Ile Ser Glu Glu Asp Leu Asn Gly Ala
   245 250 255
   Ala
<210> 107
   <211> 219
   <212> PRT
   <213> Künstliche Sequenz
<220>
   <223> Beschreibung der künstlichen Sequenz: muriner Antikörper
<400> 107
   Asp Ile Val Ile Thr Gln Thr Pro Leu Ser Leu Pro Val Ser Leu Gly
   1 5 10 15
   Asp Gln Ala Ser Ile Ser Cys Arg Ser Ser Gln Ser Leu Leu His Ser
   20 25 30
   Asn Gly Asn Thr Tyr Leu His Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Phe Cys Ser Gln Ser
   85 90 95
   Thr His Val Pro Tyr Thr Phe Gly Gly Gly Thr Lys Leu Glu Ile Lys
   100 105 110
   Arg Ala Asp Ala Ala Pro Thr Val Ser Ile Phe Pro Pro Ser Ser Glu
   115 120 125
   Gln Leu Thr Ser Gly Gly Ala Ser Val Val Cys Phe Leu Asn Asn Phe
   130 135 140
   Tyr Pro Lys Asp Ile Asn Val Lys Trp Lys Ile Asp Gly Ser Glu Arg
   145 150 155 160
   Gln Asn Gly Val Leu Asn Ser Trp Thr Asp Gln Asp Ser Lys Asp Ser
   165 170 175
   Thr Tyr Ser Met Ser Ser Thr Leu Thr Leu Thr Lys Asp Glu Tyr Glu
   180 185 190
   Arg His Asn Ser Tyr Thr Cys Glu Ala Thr His Lys Thr Ser Thr Ser
   195 200 205
   Pro Ile Val Lys Ser Phe Asn Arg Asn Glu Cys
   210 215
<210> 108
   <211> 219
   <212> PRT
   <213> Künstliche Sequenz
<220>
   <223> Beschreibung der künstlichen Sequenz: muriner Antikörper
<400> 108
   Asp Ile Gln Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg Ala Asp Ala Ala Pro Thr Val Ser Ile Phe Pro Pro Ser Ser Glu
   115 120 125
   Gln Leu Thr Ser Gly Gly Ala Ser Val Val Cys Phe Leu Asn Asn Phe
   130 135 140
   Tyr Pro Lys Asp Ile Asn Val Lys Trp Lys Ile Asp Gly Ser Glu Arg
   145 150 155 160
   Gln Asn Gly Val Leu Asn Ser Trp Thr Asp Gln Asp Ser Lys Asp Ser
   165 170 175
   Thr Tyr Ser Met Ser Ser Thr Leu Thr Leu Thr Lys Asp Glu Tyr Glu
   180 185 190
   Arg His Asn Ser Tyr Thr Cys Glu Ala Thr His Lys Thr Ser Thr Ser
   195 200 205
   Pro Ile Val Lys Ser Phe Asn Arg Asn Glu Cys
   210 215
<210> 109
   <211> 571
   <212> PRT
   <213> Künstliche Sequenz
<220>
   <223> Beschreibung der künstlichen Sequenz: muriner Antikörper
<400> 109
   Gln Val Gln Leu Lys Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Ser Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Tyr Asp Asn His Tyr Phe Asp Tyr Trp Gly Gln Gly Thr Thr
   100 105 110
   Leu Thr Val Ser Glu Ser Gln Ser Phe Pro Asn Val Phe Pro Leu Val
   115 120 125
   Ser Cys Glu Ser Pro Leu Ser Asp Lys Asn Leu Val Ala Met Gly Cys
   130 135 140
   Leu Ala Arg Asp Phe Leu Pro Ser Thr Ile Ser Phe Thr Trp Asn Tyr
   145 150 155 160
   Gln Asn Asn Thr Glu Val Ile Gln Gly Ile Arg Thr Phe Pro Thr Leu
   165 170 175
   Arg Thr Gly Gly Lys Tyr Leu Ala Thr Ser Gln Val Leu Leu Ser Pro
   180 185 190
   Lys Ser Ile Leu Glu Gly Ser Asp Glu Tyr Leu Val Cys Lys Ile His
   195 200 205
   Tyr Gly Gly Lys Asn Arg Asp Leu His Val Pro Ile Pro Ala Val Ala
   210 215 220
   Glu Met Asn Pro Asn Val Asn Val Phe Val Pro Pro Arg Asp Gly Phe
   225 230 235 240
   Ser Gly Pro Ala Pro Arg Lys Ser Lys Leu Ile Cys Glu Ala Thr Asn
   245 250 255
   Phe Thr Pro Lys Pro Ile Thr Val Ser Trp Leu Lys Asp Gly Lys Leu
   260 265 270
   Val Glu Ser Gly Phe Thr Thr Asp Pro Val Thr Ile Glu Asn Lys Gly
   275 280 285
   Ser Thr Pro Gln Thr Tyr Lys Val Ile Ser Thr Leu Thr Ile Ser Glu
   290 295 300
   Ile Asp Trp Leu Asn Leu Asn Val Tyr Thr Cys Arg Val Asp His Arg
   305 310 315 320
   Gly Leu Thr Phe Leu Lys Asn Val Ser Ser Thr Cys Ala Ala Ser Pro
   325 330 335
   Ser Thr Asp Ile Leu Thr Phe Thr Ile Pro Pro Ser Phe Ala Asp Ile
   340 345 350
   Phe Leu Ser Lys Ser Ala Asn Leu Thr Cys Leu Val Ser Asn Leu Ala
   355 360 365
   Thr Tyr Glu Thr Leu Asn Ile Ser Trp Ala Ser Gln Ser Gly Glu Pro
   370 375 380
   Leu Glu Thr Lys Ile Lys Ile Met Glu Ser His Pro Asn Gly Thr Phe
   385 390 395 400
   Ser Ala Lys Gly Val Ala Ser Val Cys Val Glu Asp Trp Asn Asn Arg
   405 410 415
   Lys Glu Phe Val Cys Thr Val Thr His Arg Asp Leu Pro Ser Pro Gln
   420 425 430
   Lys Lys Phe Ile Ser Lys Pro Asn Glu Val His Lys His Pro Pro Ala
   435 440 445
   Val Tyr Leu Leu Pro Pro Ala Arg Glu Gln Leu Asn Leu Arg Glu Ser
   450 455 460
   Ala Thr Val Thr Cys Leu Val Lys Gly Phe Ser Pro Ala Asp Ile Ser
   465 470 475 480
   Val Gln Trp Leu Gln Arg Gly Gln Leu Leu Pro Gln Glu Lys Tyr Val
   485 490 495
   Thr Ser Ala Pro Met Pro Glu Pro Gly Ala Pro Gly Phe Tyr Phe Thr
   500 505 510
   His Ser Ile Leu Thr Val Thr Glu Glu Glu Trp Asn Ser Gly Glu Thr
   515 520 525
   Tyr Thr Cys Val Val Gly His Glu Ala Leu Pro His Leu Val Thr Glu
   530 535 540
   Arg Thr Val Asp Lys Ser Thr Gly Lys Pro Thr Leu Tyr Asn Val Ser
   545 550 555 560
   Leu Ile Met Ser Asp Thr Gly Gly Thr Cys Tyr
   565 570
<210> 110
   <211> 573
   <212> PRT
   <213> Künstliche Sequenz
<220>
   <223> Beschreibung der künstlichen Sequenz: muriner Antikörper
<400> 110
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Glu Ser Gln Ser Phe Pro Asn Val Phe Pro
   115 120 125
   Leu Val Ser Cys Glu Ser Pro Leu Ser Asp Lys Asn Leu Val Ala Met
   130 135 140
   Gly Cys Leu Ala Arg Asp Phe Leu Pro Ser Thr Ile Ser Phe Thr Trp
   145 150 155 160
   Asn Tyr Gln Asn Asn Thr Glu Val Ile Gln Gly Ile Arg Thr Phe Pro
   165 170 175
   Thr Leu Arg Thr Gly Gly Lys Tyr Leu Ala Thr Ser Gln Val Leu Leu
   180 185 190
   Ser Pro Lys Ser Ile Leu Glu Gly Ser Asp Glu Tyr Leu Val Cys Lys
   195 200 205
   Ile His Tyr Gly Gly Lys Asn Arg Asp Leu His Val Pro Ile Pro Ala
   210 215 220
   Val Ala Glu Met Asn Pro Asn Val Asn Val Phe Val Pro Pro Arg Asp
   225 230 235 240
   Gly Phe Ser Gly Pro Ala Pro Arg Lys Ser Lys Leu Ile Cys Glu Ala
   245 250 255
   Thr Asn Phe Thr Pro Lys Pro Ile Thr Val Ser Trp Leu Lys Asp Gly
   260 265 270
   Lys Leu Val Glu Ser Gly Phe Thr Thr Asp Pro Val Thr Ile Glu Asn
   275 280 285
   Lys Gly Ser Thr Pro Gln Thr Tyr Lys Val Ile Ser Thr Leu Thr Ile
   290 295 300
   Ser Glu Ile Asp Trp Leu Asn Leu Asn Val Tyr Thr Cys Arg Val Asp
   305 310 315 320
   His Arg Gly Leu Thr Phe Leu Lys Asn Val Ser Ser Thr Cys Ala Ala
   325 330 335
   Ser Pro Ser Thr Asp Ile Leu Thr Phe Thr Ile Pro Pro Ser Phe Ala
   340 345 350
   Asp Ile Phe Leu Ser Lys Ser Ala Asn Leu Thr Cys Leu Val Ser Asn
   355 360 365
   Leu Ala Thr Tyr Glu Thr Leu Asn Ile Ser Trp Ala Ser Gln Ser Gly
   370 375 380
   Glu Pro Leu Glu Thr Lys Ile Lys Ile Met Glu Ser His Pro Asn Gly
   385 390 395 400
   Thr Phe Ser Ala Lys Gly Val Ala Ser Val Cys Val Glu Asp Trp Asn
   405 410 415
   Asn Arg Lys Glu Phe Val Cys Thr Val Thr His Arg Asp Leu Pro Ser
   420 425 430
   Pro Gln Lys Lys Phe Ile Ser Lys Pro Asn Glu Val His Lys His Pro
   435 440 445
   Pro Ala Val Tyr Leu Leu Pro Pro Ala Arg Glu Gln Leu Asn Leu Arg
   450 455 460
   Glu Ser Ala Thr Val Thr Cys Leu Val Lys Gly Phe Ser Pro Ala Asp
   465 470 475 480
   Ile Ser Val Gln Trp Leu Gln Arg Gly Gln Leu Leu Pro Gln Glu Lys
   485 490 495
   Tyr Val Thr Ser Ala Pro Met Pro Glu Pro Gly Ala Pro Gly Phe Tyr
   500 505 510
   Phe Thr His Ser Ile Leu Thr Val Thr Glu Glu Glu Trp Asn Ser Gly
   515 520 525
   Glu Thr Tyr Thr Cys Val Val Gly His Glu Ala Leu Pro His Leu Val
   530 535 540
   Thr Glu Arg Thr Val Asp Lys Ser Thr Gly Lys Pro Thr Leu Tyr Asn
   545 550 555 560
   Val Ser Leu Ile Met Ser Asp Thr Gly Gly Thr Cys Tyr
   565 570
<210> 111
   <211> 448
   <212> PRT
   <213> Künstliche Sequenz
<220>
   <223> Beschreibung der künstlichen Sequenz: chimärer Antikörper Maus/Mensch
<400> 111
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Gly Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Asp Glu Leu Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 112
   <211> 571
   <212> PRT
   <213> Künstliche Sequenz
<220>
   <223> Beschreibung der künstlichen Sequenz: chimärer Antikörper Maus/Mensch
<400> 112
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Trp Phe Ala Tyr Trp Gly Gln Gly
   100 105 110
   Thr Thr Val Thr Val Ser Gly Ser Ala Ser Ala Pro Thr Leu Phe Pro
   115 120 125
   Leu Val Ser Cys Glu Asn Ser Pro Ser Asp Thr Ser Ser Val Ala Val
   130 135 140
   Gly Cys Leu Ala Gln Asp Phe Leu Pro Asp Ser Ile Thr Leu Ser Trp
   145 150 155 160
   Lys Tyr Lys Asn Asn Ser Asp Ile Ser Ser Thr Arg Gly Phe Pro Ser
   165 170 175
   Val Leu Arg Gly Gly Lys Tyr Ala Ala Thr Ser Gln Val Leu Leu Pro
   180 185 190
   Ser Lys Asp Val Met Gln Gly Thr Asp Glu His Val Val Cys Lys Val
   195 200 205
   Gln His Pro Asn Gly Asn Lys Glu Lys Asn Val Pro Leu Pro Val Ile
   210 215 220
   Ala Glu Leu Pro Pro Lys Val Ser Val Phe Val Pro Pro Arg Asp Gly
   225 230 235 240
   Phe Phe Gly Asn Pro Arg Lys Ser Lys Leu Ile Cys Gln Ala Thr Gly
   245 250 255
   Phe Ser Pro Arg Gln Ile Gln Val Ser Trp Leu Arg Glu Gly Lys Gln
   260 265 270
   Val Gly Ser Gly Val Thr Thr Asp Gln Val Gln Ala Glu Ala Lys Glu
   275 280 285
   Ser Gly Pro Thr Thr Tyr Lys Val Thr Ser Thr Leu Thr Ile Lys Glu
   290 295 300
   Ser Asp Trp Leu Gly Gln Ser Met Phe Thr Cys Arg Val Asp His Arg
   305 310 315 320
   Gly Leu Thr Phe Gln Gln Asn Ala Ser Ser Met Cys Val Pro Asp Gln
   325 330 335
   Asp Thr Ala Ile Arg Val Phe Ala Ile Pro Pro Ser Phe Ala Ser Ile
   340 345 350
   Phe Leu Thr Lys Ser Thr Lys Leu Thr Cys Leu Val Thr Asp Leu Thr
   355 360 365
   Thr Tyr Asp Ser Val Thr Ile Ser Trp Thr Arg Gln Asn Gly Glu Ala
   370 375 380
   Val Lys Thr His Thr Asn Ile Ser Glu Ser His Pro Asn Ala Thr Phe
   385 390 395 400
   Ser Ala Val Gly Glu Ala Ser Ile Cys Glu Asp Asp Trp Asn Ser Gly
   405 410 415
   Glu Arg Phe Thr Cys Thr Val Thr His Thr Asp Leu Pro Ser Pro Leu
   420 425 430
   Lys Gln Thr Ile Ser Arg Pro Lys Gly Val Ala Leu His Arg Pro Asp
   435 440 445
   Val Tyr Leu Leu Pro Pro Ala Arg Glu Gln Leu Asn Leu Arg Glu Ser
   450 455 460
   Ala Thr Ile Thr Cys Leu Val Thr Gly Phe Ser Pro Ala Asp Val Phe
   465 470 475 480
   Val Gln Trp Met Gln Arg Gly Gln Pro Leu Ser Pro Glu Lys Tyr Val
   485 490 495
   Thr Ser Ala Pro Met Pro Glu Pro Gln Ala Pro Gly Arg Tyr Phe Ala
   500 505 510
   His Ser Ile Leu Thr Val Ser Glu Glu Glu Trp Asn Thr Gly Glu Thr
   515 520 525
   Tyr Thr Cys Val Val Ala His Glu Ala Leu Pro Asn Arg Val Thr Glu
   530 535 540
   Arg Thr Val Asp Lys Ser Thr Gly Lys Pro Thr Leu Tyr Asn Val Ser
   545 550 555 560
   Leu Val Met Ser Asp Thr Ala Gly Thr Cys Tyr
   565 570
<210> 113
   <211> 219
   <212> PRT
   <213> Künstliche Sequenz
<220>
   <223> Beschreibung der künstlichen Sequenz: chimärer Antikörper Maus/Mensch
<400> 113
   Asp Ile Gln Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215

## Beschreibung

Die Erfindung betrifft Erkennungsmoleküle, die gegen Tumore gerichtet sind und zur Diagnose und Therapie von Tumorerkrankungen verwendet werden können.

Tumor- oder Krebserkrankungen sind Geschwulsterkrankungen, die eine örtlich umschriebene Zunahme des Gewebevolumens beschreiben. Im weiteren Sinne ist jede lokalisierte Anschwellung durch Ödeme, akute und/oder chronische Entzündungen, eine aneurysmatische Erweiterung oder auch eine entzündlich bedingte Organschwellung ein Tumor. Im engeren Sinne werden vor allem gewebliche Neubildungen wie Gewächse, Blastome und/oder Neoplasien in Form eines spontanen, verschiedenartig enthemmten, autonomen und irreversiblen Überschußwachstums von körpereigenem Gewebe, das in der Regel mit unterschiedlich ausgeprägtem Verlust spezifischer Zellen und Gewebefunktionen verbunden ist, als Tumorerkrankungen verstanden. Es ist möglich, Tumore nach ihrem biologischen Verhalten, aber auch nach einer histogenetischen Systematik bzw. nach klinischen oder pathologischen Befunden zu systematisieren.

Insbesondere im klinischen Bereich kann es erforderlich sein, Tumore möglichst frühzeitig und auch selektiv zu erkennen, da eine frühzeitige Erkennung und die dann folgende Behandlung bzw. Entfernung sicherstellt, daß die Geschwulst erfolgreich behandelt werden kann, ohne daß die befallenen Organstrukturen bzw. Genabschnitte deformiert werden, wobei weiterhin verhindert werden kann, daß sich Metastasen ausbilden. Auch bei den Folgeuntersuchungen nach einer Krebsbehandlung müssen kleinste Metastasen frühzeitig detektiert werden, um die weitere Nachbehandlung zu optimieren. Für weite Bereiche der Arbeitsmedizin ist es außerdem notwendig, zu bestimmen, ob ein Gewebe oder ein Organ eine potentielle Krebsanfälligkeit zeigt, ohne daß das Organ bzw. das Gewebe bereits selbst entartet bzw. transformiert ist.

Die älteste und zugleich einfachste und zum Teil auch noch heute mit Erfolg angewendete Methode, einen Tumor zu erkennen, ist das Tasten und Schauen. So ist z.B. das Mammakarzinom bzw. das Prostatakarzinom als Knoten ertastbar. Hinweise auf Hautkrebs sind durch auffällige Muttermale durch den Arzt oder den Patienten selbst optisch zu detektieren. Andere optische Verfahren sind beispielsweise die bildgebenden Methoden. Hier werden mit Hilfe von Apparaten Bilder vom Körper aufgenommen, auf denen ein Tumor erkennbar ist. Zu diesen Methoden zählen zum Beispiel Röntgenbestrahlung wie auch die Computer-Tomographie (CT). Bei diesen Verfahren wird der Körper mit energiereicher Strahlung durchleuchtet, wobei die entarteten Gewebestrukturen aufgrund der veränderten Durchlässigkeit für diese Strahlung im Vergleich zum gesunden Gewebe erkennbar sind. Häufig werden bei diesen Methoden Kontrastmittel verwendet, die in entsprechende Regionen gespritzt werden und die Absorption erhöhen. Außerdem ist die Krebsdiagnose mittels Ultraschall sowie durch die Verwendung radioaktiv markierter Antikörper möglich, wobei die tumortypischen Antigene an die zu untersuchenden Organe binden und so die Tumore innerhalb des bildgebenden Verfahrens erkennbar machen. Neben den bildgebenden Methoden sind Laboruntersuchungen ein weiteres wichtiges Mittel zur Krebsfrüherkennung. Dabei werden Proben von Urin, Blut oder auch Gewebeproben auf Abnormitäten untersucht. Dies kann beispielsweise eine veränderte Zusammensetzung dieser Proben sein, aber auch das Auftreten von Substanzen, die normalerweise nicht oder nur in geringen Mengen vorkommen. Diese Substanzen werden allgemein als Tumormarker bezeichnet. Sie werden entweder vom Tumorgewebe selbst produziert oder als Reaktion des Körpers auf den Tumor gebildet. Als Tumormarker werden neben Substanzen auch zelluläre Veränderungen bezeichnet, deren qualitative oder quantitative Analyse eine Aussage über das Vorliegen, den Verlauf oder eine Prognose von bösartigen Erkrankungen ermöglicht. Tumormarker sind meist physiologisch vorkommende bzw. modifizierte Substanzen, die gegenüber physiologischen Bedingungen oder der normalen genotypischen/phänotypischen Ausprägung im Urin, Serum oder anderen Körperflüssigkeiten in erhöhter oder erniedrigter Konzentration oder in oder auf Tumorzellen nachweisbar sind, wobei diese Substanzen vom Tumorgewebe synthetisiert und/oder sezerniert und folgend durch Tumorzerfall freigesetzt oder als Reaktion des Organismus auf einen Tumor gebildet werden. Es ist eine Vielzahl von Tumormarkern beschrieben worden, deren Einsatz insbesondere beim Dickdarmkrebs, Brustkrebs, Eierstockkrebs, Prostata- und Hodenkrebs und beim kleinzelligen Lungenkarzinom als sinnvoll gilt. Zu diesen Krebsmarkern gehört beispielsweise das CEA, CA 15-3, CA 125, Alpha-Fetoprotein, HCG, das prostataspezifische Antigen, die neuronspezifische Enolase, CA 19-9 und SCC.

Die genannten Marker zeigen durch eine Erhöhung im Serum oder in Geweben oder durch ihr Vorhandensein als modifizierte Proteine, Lipide und/oder Kohlenhydrate zum einen beispielsweise (i) entzündliche Erkrankungen, Darmpolypen, Virusentzündungen aber insbesondere auch (ii) Zirrhosen, Entartungen, Tumore und Metastasen an. Ein Großteil dieser Marker besteht aus Molekülen, die Protein- als auch Kohlenhydratstrukturen, ggf. Lipide umfassen. Je geringer der Proteinanteil und demgemäß je größer der Kohlenhydrat- oder Lipidanteil dieser Marker ist, um so schwieriger können diese beispielsweise mit Erkennungsmolekülen, wie z.B. Antikörpern, detektiert werden. Bisher wurden durch Immunisierung von Mäusen mit Hilfe der Hybridom-Technologie verschiedene Antikörper gegen Kohlenhydratstrukturen hergestellt.

Die Krebsdiagnostik mit Erkennungsmolekülen weist mehrere Nachteile auf. So können bestimmte Tumormarker auch bei nichtkanzerogenen Krankheiten auftreten, wodurch die eingesetzten Erkennungsmoleküle eine positive Reaktion anzeigen; weiterhin bedeutet eine Nichtinteraktion der Erkennungsmoleküle nicht, daß keine Tumorkrankheit vorhanden ist. Ein weiterer Nachteil ist, daß die bekannten Erkennungssubstanzen in der Regel unspezifisch sind. Das bedeutet, daß ein positiver Nachweis nur in seltenen Fällen auf eine bestimmte Art der Tumorerkrankung weist. Ein weiterer, ganz entscheidender Nachteil der bekannten Erkennungsmoleküle ist außerdem, daß sie nur bedingt zur Verlaufskontrolle der Entwicklung von Tumoren, beispielsweise nach einer Operation, verwendet werden können. Das heißt, die bekannten Tumormarker können in der Regel nicht zur Früherkennung oder zur Nachbehandlung, insbesondere nicht zur Prophylaxe eingesetzt werden.

Neben diesen allgemeinen Nachteilen treten bei Erkennungsmolekülen, die gegen Kohlenhydratstrukturen gerichtet sind, spezielle Nachteile auf. Die Immunisierung mit Kohlenhydratantigenen führt meist nur zu einer primären IgM-Antwort bzw. die Immunantwort bleibt vollständig aus, da viele Kohlenhydratstrukturen auch Autoantigene sind. Weil Kohlenhydrate T-Cell-unabhängige Antigene sind, die nicht in der Lage sind, einen Klassenswitch und die damit verbundene Reifung durch somatische Mutationen hervorzurufen, bleibt die Antikörperantwort meist auf die IgM-Klasse beschränkt. Aufgrund der generell schwachen Wechselwirkung und der notwendigen Multivalenz ist es deshalb schwierig, Antikörper mit hoher Affinität herzustellen. Ein Problem bei Antikörpern gegen Kohlenhydratstrukturen sind nicht nur die geringe Affinität, sondern auch die Spezifität. Besonders gegen kurze nichtgeladene Kohlenhydratstrukturen ist es äußerst schwierig, spezifische Antikörper herzustellen, wobei eine gewisse Spezifität in vielen Fällen auch nur dann erreicht wird, wenn die Kohlenhydratstruktur auf einem bestimmten Träger lokalisiert ist. So beschreiben Karsten et al. (1995) Hybridoma 14(1), 37-44, die Bindungs- und Epitop-Spezifität eines monoklonalen Antikörpers, der spezifisch an das Thomsen-Friedenreich-Antigen (Epitop Galβ1-3GalNAc) bindet und u.a. für die Immunohistochemie geeignet ist. Dagegen erkennt beispielsweise der Antikörper JAA/F11, der gegen Galβ1→3GalNAc gerichtet ist, nicht nur dieses Antigen selbst, sondern auch GIcNAcβ1→6Galβ1→3-(GlcNAcβ1→6)GalNAc sowie, wenn auch mit geringerer Avidität, Galβ1-3GlcNAc. Auch die neueren Möglichkeiten der Gewinnung von Erkennungsmolekülen durch verschiedene Formen der kombinatorischen Techniken, wie beispielsweise der Phagendisplay-Technologie, lösen die genannten Nachteile nicht. Auch hier bleibt das Problem der schwachen Erkennungsmolekül-Kohlenhydrat-Interaktion. Zu berücksichtigen ist hierbei insbesondere, daß die durch Immunisierung meistgewonnenen primären IgM-Antikörper für den therapeutischen Einsatz zu groß sind. Ein weiterer Nachteil der bekannten Erkennungsmoleküle gegen Tumormarker ist der, dass sie den Tumor erst erkennbar machen, wenn dieser eine kritische Größe bereits erreicht hat. Das heißt, frühe Stadien des Tumorwachstums können mit den bekannten Erkennungsmolekülen, die gegen Tumormarker gerichtet sind, nicht bestimmt werden.

Ein weiterer Nachteil der bekannten Erkennungssubstanzen ist, daß sie nicht 'funktional' eingesetzt werden können. Funktional bedeutet, daß die Erkennungsmoleküle nicht nur so an die Tumormarker binden, daß diese detektiert werden, sondern daß sie somit über Marker mit der Tumorzelle wechselwirken, daß die Tumorzelle in ihrem Wachstum beeinträchtigt wird. Es ist möglich, daß die Erkennungsmoleküle mit bestimmten Tumormarkern, die beispielsweise auf Tumorzelloberflächen immobilisiert sind, so spezifisch interagieren, daß der durch die Tumormarker charakterisierte Tumor therapeutisch behandelt wird. Diese funktional aktiven Erkennungsmoleküle sind zum einen in der Lage, tumorzellenassoziierten Tumormarker zu detektieren und gleichzeitig durch ihre Bindung an diese tumorspezifische Struktur die Tumorzelle an einem weiteren Wachstum oder an einer Ausbildung von Metastasen zu hindern. Die bekannten Erkennungsmoleküle sind nachteilhafterweise in nur wenigen Fällen in der Lage, das Tumorwachstum zu beeinflussen. In der Regel müssen daher zusätzlich Substanzen, die das Tumorwachstum einschränken bzw. inhibieren, an den Antikörper gekoppelt werden, so daß dieser die "Fähre" dieser Substanz ist, aber nicht das Agens der Behandlung.

Aufgabe der Erfindung ist es daher, Erkennungsmoleküle bereitzustellen, mit denen zum einen Tumore einfach, sicher und effizient detektiert werden können, und die weiterhin in der Prophylaxe, Therapie und/oder Nachbehandlung von Tumoren eingesetzt werden können.

Die Erfindung löst dieses technische Problem durch die Bereitstellung von Erkennungsmolekülen umfassend eine Aminosäuresequenz, die die Aminosäuresequenz SEQ ID Nr. 1 und die Aminosäuresequenz SEQ ID Nr. 2 oder 3 und die Aminosäuresequenz SEQ ID Nr. 4, 5 oder 6 enthält, wobei die Erkennungsmoleküle das Antigen Core-1 spezifisch binden.

Die Definitionen der Begriffe, die im folgenden gemacht werden, treffen mutatis mutandis auf zuvor gemachte, diese und die nachfolgenden Ausführungen zu.

Unter dem Begriff Erkennungsmolekül versteht man erfindungsgemäß ein Molekül, das, insbesondere unter stringenten Bedingungen, die Kohlenhydratstruktur Core-1 spezifisch bindet.

Unter Core-1 versteht man erfindungsgemäß die Kohlenhydratstruktur Galβ1-3GalNAc, die als α-Anomer (Galβ1-3GalNAcα) oder β-Anomer (Galβ1-3GalNAcβ) vorliegen kann. Bevorzugt ist hier die α-anomere Variante. Die erfindungsgemäßen Erkennungsmoleküle können aber auch nur das alpha-Anomer Galβ1-3GalNAcα oder beide Anomere Galβ1-3GalNAcα und Galβ1-3GalNAcβ in gleicher Weise binden.

Unter einer spezifischen Bindung gegen Core-1 versteht man erfindungsgemäß eine Bindung, die nur Core-1, bevorzugt das α-Anomer, erkennt oder die Core-1 und Core-2 (Galβ1-3(GlcNAcβ1-6)GalNAcα) erkennt. Die Erkennungsmoleküle zeigen dabei keine Kreuzreaktivität mit anderen Derivaten und Anomeren dieser Kohlenhydratstrukturen wie sie in Beispiel 7 aufgeführt sind. Die erfindungsgemäßen Erkennungsmoleküle interagieren nicht mit Gala1-3GalNAca, Ga1α1-3GalNAcβ, GalNAcα, Neu5Acα2-3Galβ1-3GalNAcα, Galβ1-3(Neu5Acα2-6)GalNAcα, GlcNAcβ1-2Galβ1-3GalNAcα, GlcNAca1-3Ga1β1-3GalNAcα, GalNAcα1-3Galβ und 3'-O-Su-Ga1β1-3GalNAcα unter den in Beispiel 7 beschriebenen Bedingungen. Die Bestimmung erfolgt dabei insbesondere über Spezifitätstests mit definierten synthetischen Kohlenhydratstrukturen.

In einer bevorzugten Ausführungsform umfasst ein erfindungsgemäßes Erkennungsmolekül, das das Antigen Core-1 spezifisch bindet:
a) eine erste Aminosäuresequenz, die die Aminosäuresequenz SEQ ID Nr. 1 und die Aminosäuresequenz SEQ ID Nr. 2 oder 3 und die Aminosäuresequenz SEQ ID Nr. 4 oder 5 oder 6 enthält; und
b) eine zweite Aminosäuresequenz, die die Aminosäuresequenz SEQ ID Nr. 7 oder 8 oder 9 und die Aminosäuresequenz SEQ ID Nr. 10 oder 11 und die Aminosäuresequenz SEQ ID Nr. 12 oder 13 enthält.

Die erste und zweite Aminosäuresequenz können dabei auf einem oder mehreren, dort bevorzugt zwei, Polypeptiden vorkommen.

Die erfindungsgemäßen Core-1 bindenden Erkennungsmoleküle sind dadurch charakterisiert, dass sie ein definiertes Set von einzelnen Aminosäuresequenzen beinhalten. Die Aminosäuresequenz dieser Erkennungsmoleküle enthält ein oder zwei Tripletts definierter Sequenzen. Diese Sequenzen stellen die Bindungsdomänen dar und definieren die Spezifität des Erkennungsmoleküls. Das 1-Triplett-Erkennungsmolekül enthält die Aminosäuresequenz SEQ ID NO. 1, die Aminosäuresequenz SEQ ID NO. 2 oder 3 und die Aminosäuresequenz SEQ ID NO. 4 oder 5 oder 6. Core-1-spezifische Erkennungsmoleküle, die durch zwei Tripletts definiert sind, enthalten für das erste Triplett die Aminosäuresequenz SEQ ID NO. 1, die Aminosäuresequenz SEQ ID NO. 2 oder 3 und die Aminosäuresequenz SEQ ID NO. 4 oder 5 oder 6 und für das zweite Triplett die Aminosäuresequenz SEQ ID NO. 7 oder 8 oder 9, die Aminosäuresequenz SEQ ID NO. 10 oder 11 und die Aminosäuresequenz SEQ ID NO. 12 oder 13. Dabei können das erste und zweite Triplett entweder auf einer oder mehreren Polypeptidketten vorkommen, die im letzteren Fall gemeinsam das bindende Erkennungsmolekül bilden. Im Weiteren werden diese Tripletts im Sinne der Erfindung als Triplettsequenz 1 für die erste umfasste Aminosäuresequenz und als Triplettsequenz 2 für die zweite umfasste Aminosäuresequenz, siehe Definition a) und b) der obigen Beschreibung, bezeichnet. Das Erkennungsmolekül kann erfindungsgemäß ein Antikörper sein, insbesondere ein muriner, chimärer oder humaner IgG oder IgM, eine scFv-Struktur oder eine andere.

Eine weitere Ausführungsform der Erfindung betrifft Erkennungsmoleküle, bei denen mindestens eine Aminosäuresequenz der SEQ ID NO. 1 bis 13 durch Mutation, Deletion und/oder Insertion verändert ist, wobei jedoch die Eigenschaft der Bindungsspezifität gegen Core-1 weiter besteht. Dies dient vorteilhafterweise der Verbesserung der Erkennungsmoleküle, beispielsweise in Bezug auf Affinität, Löslichkeit und/oder Produzierbarkeit.

In einer bevorzugten Ausführungsform erfolgt die Modifikation eines Erkennungsmoleküls durch eine oder mehrere Mutationen in einer oder mehreren Aminosäuresequenzen ausgewählt aus SEQ ID NO. 1 bis 13, wobei einzelne Aminosäuren durch Aminosäuren mit analogen physikochemischen Eigenschaften ersetzt werden, die die 3-dimensionale Struktur der Bindungsdomäne des Erkennungsmoleküls mit Vorteil nicht grundlegend verändern, so dass die Core-1 Spezifität des Erkennungsmoleküls erhalten bleibt. Aminosäuren mit analogen physikochemischen Eigenschaften im Sinne der Erfindung können in 6 verschiedene Gruppen zusammengefaßt werden und sind in Tabelle 1 dargestellt.

**Tabelle 1: Aminosäuren mit analogen physikochemischen Eigenschaften unberücksichtigt der molekularen Größe. Eigenschaft oder Aminosäure funktionelle Gruppe**

| | |
|---|---|
| aliphatisch | Glycin |
| | Alanin |
| | Valin |
| | Leucin |
| | Isoleucin |
| Hydroxy-Gruppe | Serin |
| | Threonin |
| Carboky-Gruppe | Asparaginsäure |
| | Glutaminsäure |
| Amid-Gruppe | Asparagin |
| | Glutamin |
| Amino-Gruppe | Lysin |
| | Arginin |
| aromatisch | Phenylalanin |
| | Tyrosin |
| | Tryptophan |

In einer weiter bevorzugten Ausführungsform der erfindungsgemäßen Erkennungsmoleküle, die Core-1 spezifisch binden, ist mindestens eine Aminosäuresequenz der Aminosäuresequenzen SEQ ID NO. 1, 2, 3, 7, 8 und/oder 9 durch kanonische Strukturvarianten bzw. äquivalente Strukturen mit den Aminosäuresequenzen SEQ ID NO. 14 bis 45 ersetzt, wobei die SEQ ID NO. 1 durch eine Sequenz der Sequenzen SEQ ID NO. 14 bis 17 (CDRH1), die SEQ ID NO. 2 oder 3 durch eine Sequenz der Sequenzen SEQ ID NO. 18 bis 27 (CDRH2) und die SEQ ID NO. 7 oder 8 oder 9 durch eine Sequenz der Sequenzen SEQ ID NO. 28 bis 45 (CDRL1) ersetzt ist.

Der generelle Zusammenhang zwischen einer Aminosäuresequenz und der Tertiärstruktur der von diesen Sequenzen gebildeten Loops ist dem Fachmann bekannt und wurde ausführlich untersucht [Rooman et al., 1989; Martin, Thornton, 1996]. Ein spezielles Beispiel stellen die Immunglobuline dar. Durch Analyse der Loop-Konformationen der hypervariablen Regionen (complementarity determining regions, CDRs) in der leichten und der schweren Kette von Antikörpermolekülen wurden sogenannte kanonische Klassen definiert [Chothia, Lesk, 1987; Chothia et al., 1986, 1989, 1992; Wu, Cygler, 1993]. Auf dieser Grundlage wurden die kanonischen Strukturvarianten SEQ ID NO. 14 bis 45 der SEQ ID NO 1, 2, 3, 7, 8 und 9 abgeleitet.

Die Aminosäuresequenzen SEQ ID NO. 1 bis 13 oder deren Modifikationen in einem Core-1 spezifischen Erkennungsmolekül im Sinne der Erfindung bilden räumliche Strukturen aus, beispielsweise so genannte Loops, die dadurch gekennzeichnet sind, dass sie eine definierbare Tertiärstruktur und/oder Quartärstruktur besitzen. Die Bindungsregion eines Erkennungsmoleküls mit dem Core-1 Antigen wird von Aminosäureresten gebildet, die von bis zu sechs variablen Loops an der Oberfläche des Moleküls bereitgestellt werden, und die spezifisch mit Core-1 interagieren.

In einer weiteren Ausführungsform der Erfindung werden Erkennungsmoleküle, die Core-1 spezifisch binden, bereitgestellt, bei denen mindestens eine Sequenz der Sequenzen des Tripletts weggelassen wird, die nicht unmittelbar an der Interaktion mit dem Core-1 Antigen beteiligt ist.

In einer weiteren Ausführungsform umfassen die Erkennungsmoleküle mindestens eine der Aminosäuresequenzen der SEQ ID NO. 1 bis 13 oder deren oben beschriebene Varianten doppelt oder mehrfach, wobei diese Dopplungen auch als Varianten der gleichen Aminosäuresequenz vorkommen können. Alle die in diesem Abschnitt beschriebenen Erkennungsmoleküle erkennen vorteilhafterweise das Antigen Core-1 spezifisch. Im folgenden werden auch diese Erkennungsmoleküle, die streng genommen aufgrund des Weglassens oder Vervielfältigen von Sequenzen keine Triplettsequenzen tragen, trotzdem als Triplettsequenz 1 oder Triplettsequenz 2 bezeichnet, um die Anschaulichkeit zu vereinfachen.

In einer weiteren Ausführungsform, umfassen die erfindungsgemäßen Erkennungsmoleküle, die das Core-1 Antigen spezifisch binden, Aminosäuresequenzen, die eine Homologie von mindestens 60%, vorzugsweise 70%, bevorzugt 80%, ganz besonders bevorzugt 90% gegenüber den Sequenzen SEQ ID NO. 1 bis 13 aufweisen.

Die Erkennungsmoleküle im Sinne der Erfindung können weiterhin Gerüstsequenzen umfassen, die die umfassenden Aminosäuresequenzen Aminosäuresequenz SEQ ID NO. 1 und die Aminosäuresequenz SEQ ID NO. 2 oder 3 und die Aminosäuresequenz SEQ ID Nr. 4 oder 5 oder 6, oder deren oben beschriebene Varianten, voneinander trennen, und Gerüstsequenzen, die die Aminosäuresequenzen SEQ ID Nr. 7 oder 8 oder 9 und die Aminosäuresequenz SEQ ID Nr. 10 oder 11 und die Aminosäuresequenz SEQ ID Nr. 12 oder 13 , oder deren oben beschriebene Varianten, voneinander trennen. Die erste und zweite Aminosäuresequenz können dabei auf einem oder mehreren, bevorzugt zwei, Polypeptidketten vorkommen. Diese Gerüstsequenzen werden im Sinne der Erfindung als Spacer oder Frameworksequenzen bezeichnet und können unterschiedliche Längen und Sequenzen haben. Dabei sind ebenfalls solche Erkennungsmoleküle ausdrücklich mit eingeschlossen, bei denen nicht alle Aminosäuresequenzen der SEQ ID Nr. 1 bis 13 oder deren oben beschriebenen Varianten durch Spacer getrennt werden. Darüber hinaus haben die Erkennungsmoleküle vorzugsweise weitere flankierende Aminosäuresequenzen, die im Sinne der Erfindung auch als Gerüstsequenzen bezeichnet werden.

Die Gerüstsequenzen haben insbesondere die Aufgabe, die beschriebenen Aminosäuresequenzen, die für die Core-1 spezifische Bindung der Erkennungsmoleküle verantwortlich beziehungsweise beteiligt sind, in eine geeignete Anordnung und räumliche Struktur zu bringen, damit die Bindung an das Core-1 erfolgen kann. Es kann vorgesehen sein, dass die Aminosäuresequenzen SEQ ID NO. 1 bis NO. 13 ohne mindestens eine zusätzliche Aminosäuresequenz als Gerüstsequenz das Core-1 Antigen im Sinne der Erfindung nicht spezifisch binden können. Darüber hinaus können die Gerüstsequenzen den Erkennungsmolekülen z.B. die notwendige biologische und chemische Stabilität geben, damit die räumliche Struktur effektiv aufgebaut und für die Funktion und Anwendung in einer geeigneten funktionellen Form, die die Core-1 Bindung beinhaltet, erhalten werden kann.

In einer bevorzugten Ausführungsform werden die Triplettsequenzen in bestehende Proteine durch Austausch von Aminosäuresequenzen und/oder durch Hinzufügung eingefügt, wobei die bestehenden Proteinsequenzen als Gerüstsequenzen im Sinne der Erfindung dienen, beziehungsweise Gerüstsequenzen aus geeigneten Proteinen entnommen sind. Dabei können diese Gerüstsequenzen beispielsweise durch Mutationen, Deletionen oder Insertionen verändert werden. Hierbei bedient man sich dem Fachmann an sich bekannter Methoden der Molekularbiologie, Biochemie und Protein-Engineering. Bevorzugte Proteine hierfür sind Proteine der Immunglobulin-Superfamilie, Protease-Inhibitoren, Lektine, Helix-Bündel-Proteine und Lipocaline, wie sie z.B. offenbart sind in: Nygren und Uhlen, 1997; Nuttall SD et al., 1999 und Skerra, 2000.

In einer weiter bevorzugten Ausführungsform sind die Gerüstsequenzen Antikörpergerüstsequenzen aus einer oder verschiedenen Spezies oder Aminosäuresequenzen, die die Consensussequenz der Gerüstsequenzen muriner, humaner und/oder Antikörper anderer Säuger nachahmen. Eine Consensussequenz ist eine idealisierte Sequenz, in der repräsentativ an jeder Position die am meisten vorkommende Aminosäure steht, wenn viele existierende Sequenzen, beispielsweise aus Antikörper-Datenbanken, miteinander verglichen werden. Dabei sind die hier bevorzugten Erkennungsmoleküle dadurch gekennzeichnet, dass die Gerüstsequenzen für die erste Triplettsequenz 1 umfassend die Aminosäuresequenz SEQ ID NO. 1, die Aminosäuresequenz SEQ ID NO. 2 oder 3 und die Aminosäuresequenz SEQ ID NO. 4 oder 5 oder 6, oder deren oben beschriebene Varianten, Antikörpergerüstsequenzen der variablen schweren Kette VH, die in der Literatur auch als Framework-Sequenzen bezeichnet werden, sind und die Gerüstsequenzen für die Triplettsequenz 2 umfassend die Aminosäuresequenz SEQ ID NO. 7 oder 8 oder 9, die Aminosäuresequenz SEQ ID NO. 10 oder 11 und die Aminosäuresequenz SEQ ID NO. 12 oder 13 , oder deren oben beschrieben Varianten, Antikörpergerüstsequenzen der variablen leichten Kette VL sind.

Bevorzugt sind weiterhin Antikörpergerüstsequenzen von Antikörpern aus Säugetieren, besonders bevorzugt sind Antikörpergerüstsequenzen humanen und/oder murinen Ursprungs. Die Gerüstsequenzen können dabei aus Antikörpergerüstsequenzen verschiedener Spezies kombiniert werden. Diese Antikörpergerüstsequenzen sind dem Fachmann bekannt und in verschiedenen Datenbanken zugänglich wie der Kabat-Datenbank (immuno.bme.nwu.edu) oder der Datenbank des National Center for Biotechnology Information (www.ncbi.nlm.nih.gov). Ebenfalls können diese Antikörpergerüststrukturen durch weitere Aminosäuren verlängert, und/oder durch eine oder mehrere Mutationen, z.B. Deletionen und/oder Insertionen, verändert werden, wobei die spezifische Bindung an Core-1 erhalten bleibt.

Werden in einer bevorzugten Variante der Erfindung die Triplettsequenzen mit Antikörpergerüstsequenzen kombiniert, stellt das Erkennungsmolekül eine variable Kette eines Antikörpers oder eine davon abgeleitete Struktur dar.

Besonders bevorzugte Antikörpergerüstsequenzen als Gerüstsequenzen im Sinne der Erfindung sind für die variable schwere Kette die Aminosäuresequenzen entsprechend FRH1, FRH2, FRH3 und FHR4 in Tabelle 2 und für die variable leichte Kette die Aminosäuresequenzen entsprechend FRL1, FRL2, FRL3 und FRL4 in Tabelle 2, wobei die Aminosäuresequenzen der Triplettsequenzen 1 und 2 mit den SEQ ID NO. 1 bis 13 den entsprechenden CDR-Regionen der Antikörper entsprechen. Dabei setzen sich die variable schwere (VH) bzw. leichte (VL) Antikörperkette wie folgt zusammen: die VH: FRH1-CDRH1-FRH2-CDRH2-FRH3-CDRH3-FRH4 und die VL: FRL1-CDRL1-FRL2-CDRL2-FRL3-CDRL3-FRL4. Tabelle 2 erläutert die Positionen im Detail. Die Positionen der einzelnen Aminosäuren bzw. Aminosäuresequenzen entsprechen der Nummerierung von Aminosäuren in Antikörpermolekülen nach Kabat.

**Tabelle 2:**

| **Name** | **Positionsbereich** | **Pos.** | **Aminosäure bzw. Aminosäuresequenz** |
|---|---|---|---|
| FRH1 | 1 bis 30 | 1 | Q oder E |
| | | 2 | V |
| | | 3 | Q, K oder T |
| | | 4 | L |
| | | 5 | K oder V |
| | | 6 | E oder Q |
| | | 7 | S |
| | | 8 | G |
| | | 9 | A |
| | | 10 | E |
| | | 11 | L oder V |
| | | 12 | V oder K |
| | | 13 | R oder K |
| | | 14 | P |
| | | 15 | G |
| | | 16 | T oder A |
| | | 17 | S |
| | | 18 | V |
| | | 19 | K |
| | | 20 | I oder V |
| | | 21 | S oder P |
| | | 22 | C |
| | | 23 | K |
| | | 24 | A, V, S oder T |
| | | 25 | S |
| | | 26 | G |
| | | 27 | Y, F, S oder D |
| | | 28 | T |
| | | 29 | F, L oder I |
| | | 30 | T |
| CDRH1 | 31 bis 35 | | SEQ ID NO. 1 und Varianten |
| FRH2 | 36 bis 49 | 36 | W |
| | | 37 | V |
| | | 38 | K oder R |
| | | 39 | Q |
| | | 40 | R oder A |
| | | 41 | P |
| | | 42 | G |
| | | 43 | H oder Q |
| | | 44 | G |
| | | 45 | L |
| | | 46 | E |
| | | 47 | W oder R |
| | | 48 | I oder M |
| | | 49 | G |
| CDRH2 | 50 bis 65, wobei zusätzlich die Pos. 52a eingeführt ist | | SEQ ID NO. 2 oder 3 und Varianten |
| FRH3 | 66 bis 94 | 66 | K oder R |
| | | 67 | A oder V |
| | | 68 | T |
| | | 69 | L oder M |
| | | 70 | T |
| | | 71 | A, L oder T |
| | | 72 | D |
| | | 73 | T |
| | | 74 | S |
| | | 75 | S oder T |
| | | 76 | S |
| | | 77 | T |
| | | 78 | A |
| | | 79 | Y |
| | | 80 | M |
| | | 81 | Q oder E |
| | | 82 | L |
| | | 82a | S |
| | | 82b | S oder R |
| | | 82c | L |
| | | 83 | T oder R |
| | | 84 | S |
| | | 85 | E |
| | | 86 | D |
| | | 87 | S oder T |
| | | 88 | A |
| | | 89 | V |
| | | 90 | Y |
| | | 91 | F oder Y |
| | | 92 | C |
| | | 93 | A |
| | | 94 | Y, K oder R |
| CDRH3 | 95 bis 102, wobei zusätzlich die Pos. 100a und 100b eingeführt sind | | SEQ ID NO. 4, 5 oder 6 und Varianten |
| FRH4 | 103 bis 113 | 103 | W |
| | | 104 | G |
| | | 105 | Q |
| | | 106 | G |
| | | 107 | T |
| | | 108 | T, S oder L |
| | | 109 | V oder L |
| | | 110 | T |
| | | 111 | V |
| | | 112 | S |
| | | 113 | S oder A |
| FRL1 | 1 bis 23 | 1 | D |
| | | 2 | I, V oder L |
| | | 3 | Q oder L |
| | | 4 | M |
| | | 5 | T |
| | | 6 | Q |
| | | 7 | T oder S |
| | | 8 | P |
| | | 9 | L |
| | | 10 | S |
| | | 11 | L |
| | | 12 | P |
| | | 13 | V |
| | | 14 | S oder T |
| | | 15 | L oder P |
| | | 16 | G |
| | | 17 | D oder E |
| | | 18 | Q oder P |
| | | 19 | A |
| | | 20 | S |
| | | 21 | I |
| | | 22 | S |
| | | 23 | C |
| CDRL1 | 22 bis 34, wobei zusätzlich die Pos. 27a, 27b, 27c, 27d und 27e eingeführt sind | | SEQ ID NO. 7, 8 oder 9 und Varianten |
| FRL2 | 35 bis 49 | 35 | W |
| | | 36 | Y |
| | | 37 | L |
| | | 38 | Q |
| | | 39 | K |
| | | 40 | P |
| | | 41 | G |
| | | 42 | Q |
| | | 43 | S |
| | | 44 | P |
| | | 45 | K oder Q |
| | | 46 | L |
| | | 47 | L |
| | | 48 | I oder V |
| | | 49 | Y |
| CDRL2 | 50 bis 56 | | SEQ ID NO. 10 oder 11 und Varianten |
| FRL3 | 57 bis 88 | 57 | G |
| | | 58 | V |
| | | 59 | P |
| | | 60 | D |
| | | 61 | R |
| | | 62 | F |
| | | 63 | S |
| | | 64 | G |
| | | 65 | S |
| | | 66 | G |
| | | 67 | S |
| | | 68 | G |
| | | 69 | T |
| | | 70 | D |
| | | 71 | F |
| | | 72 | T |
| | | 73 | L |
| | | 74 | K |
| | | 75 | I |
| | | 76 | S |
| | | 77 | R |
| | | 78 | V |
| | | 79 | E |
| | | 80 | A |
| | | 81 | E |
| | | 82 | D |
| | | 83 | L oder V |
| | | 84 | G |
| | | 85 | V |
| | | 86 | Y |
| | | 87 | Y |
| | | 88 | C |
| CDRL3 | 89 bis 97 | | SEQ ID NO. 12 oder 13 und Varianten |
| FRL4 | 98 bis 108 | 98 | F |
| | | 99 | G |
| | | 100 | G oder Q |
| | | 101 | G |
| | | 102 | T |
| | | 103 | K |
| | | 104 | L |
| | | 105 | E |
| | | 106 | I oder L |
| | | 106a | K |
| | | 107 | R |
| | | 108 | A |

Die Aminosäuresequenzen SEQ ID NO. 46 bis 79 entsprechen Aminosäuresequenzen mit bevorzugten Gerüstsequenzen für die variable schwere Kette. Die Aminosäuresequenzen SEQ ID NO. 80 bis 94 entsprechen Aminosäuresequenzen mit bevorzugten Gerüstsequenzen für die variable leichte Kette.

Die zu verwendenden Techniken und Methoden zur Herstellung dieser Sequenzen sind dem Fachmann bekannt, ebenso ist der Fachmann in der Lage, geeignete Gerüstsequenzen und/oder Mutationen auszuwählen.

Im Sinne der Erfindung können die Core-1 spezifischen Erkennungsmoleküle in verschiedenen Formaten vorliegen. Die grundlegende Struktur des Erkennungsmoleküls sind eine oder mehrere Polypeptidketten, die oben beschriebenen erfindungsgemäßen Triplettsequenz 1 oder Triplettsequenzen 1 und 2 und Gerüstsequenzen umfassen. Beispielsweise sind die Aminosäuresequenz der variablen schweren Kette mit den Gerüstsequenzen und den Triplettsequenzen 1 und die Aminosäuresequenz der variablen leichten Kette mit den Gerüstsequenzen und den Triplettsequenzen 2 nicht-kovalent oder kovalent miteinander verknüpft, und können auf einer oder mehreren Polypeptidketten liegen. Mehrere Polypeptidketten können kovalent, beispielsweise durch Disulfidbrücken, oder nicht-kovalent verbunden als Erkennungsmolekül vorliegen.

Zu den unterschiedlichen erfindungsgemäßen Formaten der Erkennungsmoleküle gehören insbesondere die Verknüpfung der Triplettsequenzen mit Aminosäuresequenzen, die über die oben beschriebenen Gerüstsequenzen hinausgehen. In einer bevorzugten Variante umfassen daher erfindungsgemäße Erkennungsmoleküle neben den Triplettsequenzen und den Gerüstsequenzen weitere Zusatzsequenzen. Zusatzsequenzen sind insbesondere Aminosäuresequenzen, die primär nicht der räumlichen Anordnung der Triplettsequenzen wie in Form der Gerüstsequenzen dienen, diese jedoch vorteilhaft durch sekundäre oder tertiäre Wechselwirkungen beeinflussen können. Beispielsweise stabilisieren Zusatzsequenzen in Form von konstanten Domänen eines Antikörpers den Antikörper und bewirken eine Dimerisierung, wodurch es zu einer verbesserten Bindung des Antikörpers kommt, oder beispielsweise bewirkt eine Fusion eines scFv mit einer Domäne eines Bakteriophagenhüllproteins eine Aktivitätssteigerung der scFv-Bindung, wie sie z.B. in Jensen KB et al., 2002 offenbart ist.

In einer bevorzugten Ausführungsform umfassen die Erkennungsmoleküle Aminosäuresequenzen mit Gerüstsequenzen auf Antikörperbasis und neben den Triplettsequenzen weitere Zusatzsequenzen. Die Zusatzsequenzen haben insbesondere mindestens eine der folgenden Aufgaben:
a) Verknüpfung einer Triplettsequenz mit ihren entsprechend geeigneten Gerüstsequenzen mit mindestens einer weiteren Triplettsequenz mit ihren entsprechend geeigneten Gerüstsequenzen, um beispielsweise eine Bindungsfähigkeit zu erzeugen oder zu verbessern;
b) der Stabilisierung der Domänen, beispielsweise durch einen Linker zwischen zwei Proteindomänen oder Aminosäuresequenzen, die mit anderen der gleichen oder einer zweiten Kette in Wechselwirkung treten;
c) Effektorfunktionen für immunologische Aufgaben, beispielsweise durch Fusion mit Fc-Teil von Antikörpern, Chemokinen, Cytokinen, Wachstumsfaktoren oder Teilen davon, oder Antikörpern mit einer anderen Spezifität oder Fragmenten davon, zur Rekrutierung von Zellen des Immunsystems, beispielsweise Makrophagen, oder Teilen des Komplementsystems;
d) Fusion mit Tags, beispielsweise Multimerisierungssequenzen - zum Beispiel µ-tail-Sequenz aus IgM oder Assoziationsdomäne aus p53 oder MBL - zur Multimerisierung der Core-1 bindenden Anteile für eine multivalente Bindung oder zur Aufreinigung der Erkennungsmoleküle, beispielsweise His-Tag oder zum Nachweis, beispielsweise myc-Tag oder zur Markierung oder Chelatisierung von Erkennungsmolekülen, beispielsweise durch Lysin-reiche Sequenzen.

Geeignete Strukturen sind dem Fachmann bekannt oder durch logische Schlussfolgerung aus dem Stand der Technik abzuleiten.

Dabei weiter bevorzugte Ausführungsformen sind erfindungsgemäße Erkennungsmoleküle, die folgende Formate umfassen: single chain Antikörperfragment (scFv), Fv-Fragment, Fab-Fragment, F(ab)₂-Fragment, Multibody (Dia-, Tria-, Tetrabody), Immunglobulin der Isotypen IgG, IgM, IgA, IgE, IgD oder deren Subklassen, beispielsweise IgG1, oder von Immunglobulinen abgeleitete Erkennungsmoleküle, die mindestens eine konstante Domäne umfassen.

In einer bevorzugten Ausführungsform sind die erfindungsgemäßen Erkennungsmoleküle aus einer schweren und einer leichten Polypeptidkette zusammengesetzt, wobei die Aminosäuresequenzen der schweren und leichten Kette jeweils eine der oben beschriebenen Triplettstrukturen umfassen, die die CDR Regionen des Antikörpers darstellen, die entsprechenden Antikörpergerüstsequenzen, die die Frameworksequenzen der Antikörper darstellen, und Zusatzsequenzen, die mindestens eine der konstanten Domänen des Antikörperisotyps umfassen. Die beiden Ketten können miteinander kovalente Bindungen eingehen. Die konstanten Regionen und variablen Regionen können dabei Sequenzen von Antikörpern aus einer oder verschiedenen Spezies enthalten. Es können Teile von konstanten Domänen oder ganze konstante Domänen deletiert oder mutiert sein, beispielsweise um die Effektorfunktion der Zusatzsequenzen zu verändern, beispielsweise die Bindung an Fc-Rezeptoren zu verhindern oder zu verbessern. In einer bevorzugten Ausführungsform ist das Erkennungsmolekül ein muriner, chimärisierter, humanisierter, partiell humaner oder humaner Antikörper oder Antikörperfragment. Die Chimärisierung erfolgt beispielsweise durch Verknüpfung der variablen Antikörperdomänen mit konstanten Antikörperdomänen oder Fragmenten der konstanten Domäne von Antikörpern verschiedener Spezies. Bevorzugt sind Sequenzen der konstanten Domänen humaner Antikörper.

Die Antikörpergerüstsequenzen können so gewählt werden, dass die Sequenzen weitestgehend homolog zu humanen Antikörpersequenzen sind. Die Wahl für den Speziesursprung der Gerüstsequenzen hängt auch von der Anwendung ab. So werden für eine therapeutische Anwendung in bestimmten Bereichen möglichst große Anteile an humanen Gerüstsequenzen bevorzugt, vor allem dann, wenn eine human anti-Maus Antikörperantwort (HAMA) vermieden werden soll. In anderen therapeutischen Bereichen ist ein Xenoanteil vorteilhaft, da er das Immunsystem in einer zusätzlichen Weise stimuliert. Eine Kombination beider ist in einigen Fällen besonders geeignet, vor allem dann, wenn in einer Erstimmunisierung ein Xenoanteil vorteilhaft und bei späteren Anwendungen ein spezieskonformer und damit humaner Anteil vorteilhaft ist.

Bevorzugt ist eine Homologie zu humanen Consensussequenzen, wobei für die variable schwere Kette die HuHI und für die variable leichte Kette die HuKII bevorzugt wird. Besonders bevorzugt ist eine Homologie zu humanen Keimbahnsequenzen, die dem Fachmann bekannt sind und zum Beispiel über die V BASE Datenbank (www.mrc-cpe.cam.ac.uk) zugänglich sind.

Die zu verwendenden Techniken und Methoden zur Herstellung dieser Sequenzen sind dem Fachmann bekannt, ebenso ist der Fachmann in der Lage, geeignete humane Sequenzen auszuwählen und/oder möglicherweise notwendige Mutationen der Sequenzen durchzuführen.

In einer weiteren Ausführungsform sind zusätzlich die Triplettsequenzen, die im allgemeinen den Bindungs-Loops (CDR-Regionen) entsprechen und die bevorzugt starke Homologien zu den entsprechenden Sequenzbereichen in der humanen Keimbahnsequenz haben, diesen schrittweise durch einfache Mutationen angeglichen, ohne die spezifische Bindung an Core-1 zu beeinträchtigen. Erkennungsmoleküle mit diesen Sequenzen werden hier als partiell humane Antikörper oder Antikörperfragmente bezeichnet. Bevorzugte humanisierte Sequenzen stellen z.B. die Sequenzen SEQ ID NO. 56 bis 79 bzw. SEQ ID NO. 85 bis 94 dar.

In einer weiteren bevorzugten Ausführungsform werden bestimmte Aminosäuren der Antikörpergerüstsequenzen einer Spezies durch andere ausgetauscht, um normalerweise weniger immunogene Regionen zu generieren. Dies beinhaltet dem Fachmann an sich bekannte Technologien, beispielsweise Technologien der Humanisierung, beispielsweise CDR-Grafting, Resurfacing, Chain-Shuffling mit Mutationen und Deimmunisierung durch Mutation oder Deletion von humanen MHC Epitopen.

In einer bevorzugten Ausführungsform handelt es sich um ein vom IgM abgeleitetes Erkennungsmolekül mit den entsprechenden konstanten Domänen eines IgM, bevorzugt humanen Sequenzen. Im Sinne der Erfindnung setzten sich Immunglobuline aus der schweren Kette und der leichten Kette eines Antikörpers zusammen, wobei bevorzugt 2 leichte und 2 schwere Ketten eine Einheit darstellen. Immunglobuline des IgM Typs bestehen meist aus 5 solchen Einheiten, die zusätzlich zu Disulfidbrücken durch die J-Kette miteinander verknüpft sind.

In einer besonders bevorzugten Ausführungsform ist die J-Kette nicht vorhanden, wobei es ebenfalls zur Multimerisierung der Untereinheiten kommt, wobei hier hexa- und pentamere Strukturen vorliegen können.

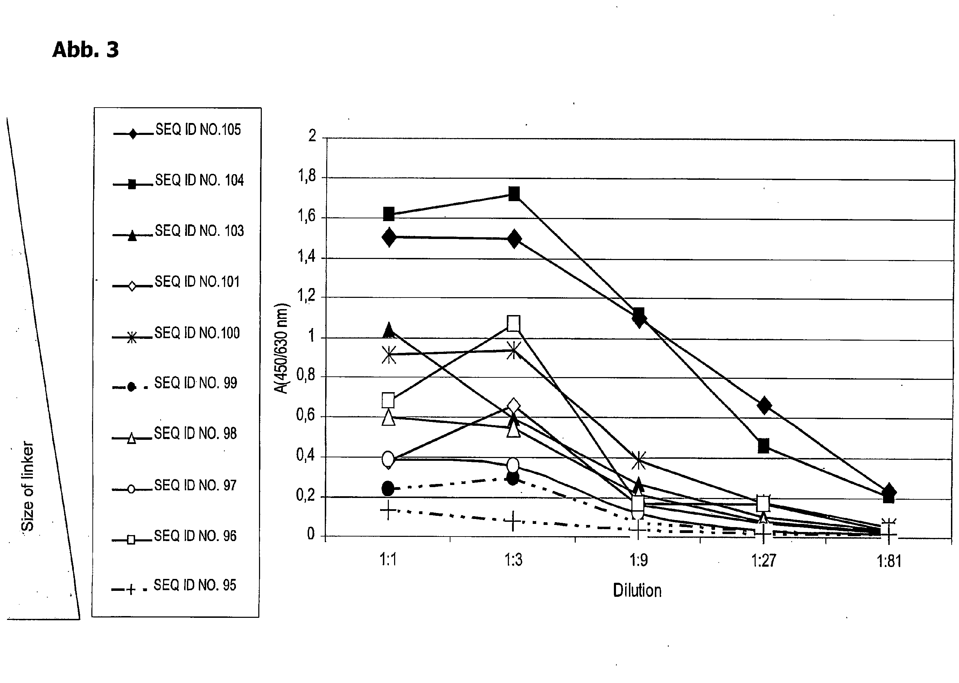

In einer bevorzugten Ausführungsform der Erkennungsmoleküle handelt es sich um Single chain Antikörperfragmente umfassend eine Triplettstruktur 1 mit entsprechenden oben beschriebenen Antikörpergerüstsequenzen, die die CDR Regionen des Antikörpers und Frameworksequenzen der variablen Domäne der schweren Kette von Antikörpern darstellen, und eine Triplettstruktur 2 mit den entsprechenden oben beschriebenen Antikörpergerüstsequenzen, die die CDR Regionen des Antikörpers und Frameworksequenzen der variablen Domäne der leichten Kette von Antikörpern darstellen, die kovalent miteinander in Form eines Fusionsproteins verknüpft sind. Hierbei sind die Sequenzen direkt oder durch einen Linker miteinander verknüpft. Bevorzugt sind hier scFv Formate ohne Linker oder mit einem Linker von 1 bis 9 Aminosäuren Länge. Diese scFv Antikörper bilden multimere Strukturen (beispielsweise Dia-, Tria-, Tetrabodies), die im Sinne der Erfindung auch als Multibodies bezeichnet werden und zeigen aufgrund der Multivalenz höhere Avidität zum Core-1 Antigen. Es wurden Core-1 spezifische Erkennungsmoleküle im scFv Format mit verschiedenen Linkerlängen konstruiert (SEQ ID NO. 95 bis 106) und ihre Bindungscharakteristik im ELISA untersucht. Eine schrittweise Linkerverkürzung führte zu einer Erhöhung der Bindung an Asialoglykophorin, einem Core-1 tragenden Glykoprotein, wie in Abbildung 3 dargestellt. Die besten Bindungseigenschaften zeigten hierbei die Varianten mit der SEQ ID NO. 104 und 105. Diese multivalenten Konstrukte im Dia/Triabody Format sind besonders bevorzugte Ausführungsformen der Erfindung und sind aufgrund verbesserter pharmakokinetischer Eigenschaften für die Tumortherapie von Vorteil.

In einer weiter bevorzugten Ausführungsform sind die Erkennungsmoleküle fusioniert, chemisch gekoppelt, kovalent oder nicht-kovalent assoziiert mit (i) Immunglobulindomänen verschiedener Spezies, (ii) Enzymmolekülen, (iii) Interaktionsdomänen, (iv) Signalsequenzen, (v) Fluoreszenzfarbstoffen, (vi) Toxinen, (vii) katalytischen Antikörpern, (viii) einem oder mehreren Antikörpern oder Antikörperfragmenten mit anderer Spezifität, (ix) zytolytischen Komponenten, (x) Immunmodulatoren, (xi) Immuneffektoren, (xii) MHC-Klasse I oder Klasse II Antigenen, (xiii) Chelatoren zur radioaktiven Markierung, (xiv) Radioisotopen, (xv) Liposomen, (xvi) Transmembrandomänen, (xvii) Viren und/oder Zellen. Außerdem können die Erkennungsmoleküle insbesondere mit einem Tag fusioniert sein, die die Detektion des Erkennungsmoleküls und deren Aufreinigung ermöglichen, wie zum Beispiel ein Myc-Tag oder ein His-Tag. Technologien zur Herstellung dieser Konstrukte sind dem Fachmann bekannt, ebenso ist der Fachmann in der Lage, geeignete Sequenzen und Komponenten auszuwählen und mit den erfindungsgemäßen Erkennungsmolekülen in geeigneter Weise zu verbinden.

In einer weiteren bevorzugten Ausführungsform sind die beschriebenen Erkennungsmoleküle auf Antikörper- oder Antikörperfragment-Basis mit Peptiden oder Proteinen, die nicht von Immunglobulinen abgeleitet sind, fusioniert. Beispielsweise wird die Multimerisierungsdomäne eines Nicht-Immunglobulinmoleküls mit einem scFv fusioniert, insbesondere das C-terminale Ende der alpha-Kette des C4 Bindungsproteins, wie es bei Tonye Libyh M. et al., 1997 beschrieben ist, und somit ein multivalentes Erkennungsmolekül konstruiert.

In einer weiteren Ausführungsform wird ein scFv mit einer Transmembrandomäne eines Nicht-Immunglobulinmoleküls fusioniert, beispielsweise mit der Transmembrandomäne des c-erb B2, des h-PDGFR, des humanen Transferrinrezeptors oder des humanen Asialoglykoprotein-Rezeptors (Liäo et al., 2000), und somit die Expression von Bindungsmolekülen auf der Oberfläche von Zellen ermöglicht.

Eine weitere bevorzugte Ausführungsform der Erfindung umfasst erfindungsgemäße Erkennungsmoleküle, die weiterhin Aminosäuresequenzen umfassen, die spezifisch an Makrophagen oder andere Immuneffektorzellen binden. Beispielsweise umfassen die erfindungsgemäßen Erkennungsmoleküle weiterhin eine Antikörperbindungsstelle gegen CD64, wodurch es in Form eines bispezifischen Antikörpers beziehungsweise Antikörperfragments (Diabodies) zur Bindung von Makrophagen an Core-1 positive Tumorzellen kommt, was zu deren Bekämpfung und/oder Zerstörung führt.

Eine bevorzugte Ausführungsform der Erfindung betrifft radioaktiv markierte Core-1 spezifische Erkennungsmoleküle. Eine bevorzugte Form sind Erkennungsmoleküle auf der Basis von Antikörpern oder Antikörperfragmenten. Eine weitere bevorzugte Ausführungsform sind radioaktiv markierte erfindungsgemäße Erkennungsmoleküle im single chain Format (einschließlich als Dia-, Tria-, Tetrabodies). Weitere bevorzugte Formen sind radioaktiv markierte single chain Antikörperfragmente und ganze Immunglobuline, beispielsweise erfindungsgemäße chimäre oder humanisierte IgG oder IgM Antikörper oder humanisierte Antikörperfragmente. Die Erfindung ist selbstverständlich nicht auf diese Antikörper, die radioaktive Markierung und diese Formate der Antikörper beschränkt.

Antikörperfragmente wie die bevorzugten multivalenten scFv Fragmente insbesondere ohne oder mit sehr kurzem Linker bieten gegenüber intakten monoklonalen Antikörpern einen Vorteil für das Targeting von soliden Tumoren. Bei intakten Antikörpern, die in Biodistributionsstudien eine spezifische Anreicherung im Tumorareal zeigen, fällt bei genauer Untersuchung des Tumors eine inhomogene Antikörperverteilung mit vornehmlicher Anreicherung im Randbereich auf. Zentral gelegene Tumoranteile werden aufgrund von Tumornekrosen, inhomogener Antigenverteilung sowie einem erhöhten interstitiellen Gewebedruck mit diesen Antikörperkonstrukten nicht erreicht. Kleinere. Antikörperfragmente zeigen dagegen eine schnelle Tumormarkierung, dringen tiefer in den Tumor ein und werden gleichzeitig relativ schnell aus der Blutbahn entfernt. Die Dissoziationskonstante von monovalenten Antikörperfragmenten wie Fabs oder scFv ist allerdings oftmals zu niedrig, was in einer kurzen Verweildauer an den Tumorzellen resultiert. Deshalb bieten multivalente Antikörperkonstrukte wie Multibodies (Diabodies, Tria/Tetrabodies), F(ab')₂ und andere Minibodies (multivalente Antikörperkonstrukte bestehend aus der Bindungsdomäne und einer Multimerisierungssequenz, beispielsweise scFv und CH3 Domäne eines IgG) in der Tumortherapie viele Vorteile. Multivalenten Konstrukte im Dia/Triabody Format sind bevorzugte Ausführungsformen der Erfindung und sind aufgrund verbesserter pharmakokinetischer Eigenschaften für die Tumortherapie von Vorteil und wurden zur Verwendung in der Tumortherapie weiter entwickelt. Sie können als Vehikel für die spezifische Anreicherung von zum Beispiel zytotoxischen Substanzen wie Chemotherapeutika oder Radionuklide im Tumor verwendet werden. Durch geeignete Radionuklidwahl können Tumorzellen über eine Distanz von mehreren Zelldurchmessern abgetötet werden, wodurch auch Antigen-negative Tumorzellen in einem Tumorareal erfasst und die schlechte Penetration der Antikörper in solide Tumoren zumindest teilweise ausgeglichen werden können.

Eine besonders bevorzugte Ausführungsform der Erfindung sind radioaktiv-markierten Multibodies - insbesondere wie unter Beispiel 9 näher ausgeführt -, die besonders vorteilhafte pharmakokinetischen Eigenschaften vereinen mit einer in der Kombination gegenüber ganzen Immunglobulinen und scFv verbesserten Tumorretention, Tumorpenetration, Serumhalbwertszeit und Serum zu Tumor-Verteilungsverhältnis. Weitere Vorteile sind die hohe Avidität und die bakterielle Expression, die es erlaubt, kostengünstig diese Erkennungsmoleküle herzustellen. Damit eignet sich dieses besondere Format der erfindungsgemäßen Erkennungsmoleküle vorteilhafterweise bevorzugt für die Behandlung von kleinen Primärtumoren, Metastasen und minimal residual Erkrankungen.

Eine bevorzugte Ausführungsform der Erfindung sind nicht radioaktiv markierte Erkennungsmoleküle. Eine bevorzugte Form hierbei sind Erkennungsmoleküle auf der Basis von Antikörpern oder Antikörperfragmenten.

Eine besonders bevorzugte Ausführungsform sind chimäre und humanisierte Immunglobuline auf der Basis von IgM Molekülen zur Inhibition der Lebermetastasierung und zur Bekämpfung residualer Tumorzellen.

Weitere bevorzugte Ausführungsformen sind toxin- oder Zytostatika-gekoppelte erfindungsgemäße chimärisierte oder humanisierte IgG und IgM basierte Erkennungsmoleküle und im Besonderen Multibodies (Dia- Tria-, Tetrabodies) mit besonders vorteilhaften pharmakokinetischen Eigenschaften wie oben ausgeführt.

Des weiteren können Liposomen, die beispielsweise mit Toxinen oder Zytostatika beladen sind, auf ihrer Oberfläche erfindungsgemäße Erkennungsmoleküle tragen.

Der Fachmann ist in der Lage, geeignete Radioisotope, Toxine und Zytostatika auszuwählen. Geeignete Techniken, Verfahren, Dosierungen und Formulierungen sind dem Fachmann bekannt.

Weiterhin können Effektorzellen des Immunsystems, auf deren Oberfläche erfindungsgemäße Erkennungsmoleküle gebunden sind, durch diese Moleküle zu Core-1 tragenden Tumorzellen dirigiert/adressiert werden und dadurch deren Bekämpfung und/oder Zerstören vermitteln. Bevorzugte Effektorzellen sind Makrophagen, dendritische Zellen und NK-Zellen, die aus dem Patienten gewonnen werden und ex vivo mit den Erkennungsmolekülen gekoppelt werden. Weiter bevorzugt sind Zelllinien dieser Zelltypen. Die Kopplung erfolgt beispielsweise durch bispezifische Erkennungsmoleküle, die neben den Core-1 spezifischen Anteilen weiterhin Aminosäuren umfassen, die eine Bindung an die Effektorzellen vermitteln. Beispielsweise sind dies bispezifische Antikörper, Komplementanteile oder konstante Domänen von Antikörpern.

Bevorzugt sind hierbei Makrophagen aus dem Patienten, die nach Gewinnung mit einem bispezifischen Antikörper beispielsweise in Form ganzer Antikörper, bevorzugt chemisch gekoppelter Fab-Fragmente oder weiter bevorzugt Diabodies, die zum einen CD64 erkennen und zum anderen erfindungsgemäß Core-1 spezifisch sind. Diese Makrophagen, die über die CD64 Spezifität die bispezifischen Erkennungsmoleküle tragen, werden dem Patienten in einer geeigneten Formulierung wieder zugeführt, um den Core-1 positiven Tumor zu bekämpfen. Die hierzu verwendeten Techniken und die geeigneten Verfahren, Dosierungen und Formulierungen sind dem Fachmann bekannt. Eine weiter bevorzugte Variante sind Makrophagen aus dem Patienten, die nach Gewinnung mit einem erfindungsgemäßen Core-1 spezifischen Antikörper oder Antiköperfragment, die den konstanten Teil eines Antikörpers umfassen, der an Makrophagen über die an sich bekannten Fc-Rezeptoren bindet. Dabei können die Erkennungsmoleküle entweder als ganze Antikörper, bevorzugt chimäre oder humanisierte IgG oder IgM, oder als Antikörperfragment, beispielsweise scFv, Fab oder Multibodies in Form eines Fusionsproteins oder chemisch gekoppelt mit dem dem Fachmann bekannten Teil der konstanten Domäne von Antikörpern, an die Makrophagen binden. Diese die Erkennungsmoleküle tragenden Makrophagen werden dem Patienten in einer geeigneten Formulierung wieder zugeführt, um den Core-1 positiven Tumor zu bekämpfen. Die hierzu verwendeten Techniken und die geeigneten Verfahren, Dosierungen und Formulierungen sind dem Fachmann bekannt.

Zelllinien oder Zellen aus dem Körper wie die oben beschriebenen Effektorzellen, die mit Molekülen transfiziert werden, können die erfindungsgemäßen Core-1 spezifischen Erkennungsmolekülen und weiterhin Elemente umfassen, die eine Expression und eine Verankerung in der Membran bewirken, beispielsweise transmembrane Domäne, und die Aktivierung der Effektorzellen bei Kontakt mit einer Core-1 tragenden Tumorzelle vermitteln. Die entsprechenden Elemente sind dem Fachmann bekannt. Beispielsweise wird eine dendritische Zelllinie mit einem Vektor transfiziert, der ein Erkennungsmolekül umfasst, das ein erfindungsgemäßes scFv oder Multibody und eine Transmembrandomäne und eine aktivierende Domäne umfasst. In einem anderen Beispiel werden dazu Makrophagen viral transfiziert. Diese die Erkennungsmoleküle tragenden Effektorzellen werden einem Patienten in einer geeigneten Formulierung zugeführt um den Core-1 positiven Tumor zu bekämpfen. Die hierzu verwendeten Techniken und die geeigneten Verfahren, Dosierungen und Formulierungen sind dem Fachmann bekannt.

Nukleinsäuremoleküle, die ein oder mehrere genetische Sequenzen umfassen, kodieren mindestens eines der oben beschriebenen erfindungsgemäßen Erkennungsmoleküle und/oder Konstrukte. Aufgrund des degenerierten genetischen Codes können diese Nukleinsäuremoleküle sehr unterschiedliche Sequenzen haben. Die Wahl der Codons ist ebenfalls von der Zelle abhängig, die für die Herstellung des Erkennungsmoleküls verwendet wird, da in unterschiedlichen Zellen aus unterschiedlichen Organismen häufig unterschiedliche Codons bevorzugt werden und die Expressionsrate stark beeinflusst werden kann, beispielsweise sind die in eukaryontischen Genen bevorzugt verwendeten Codons AGA und AGG für Arginin in Bakterien nur selten vertreten. Hier treten die Codons CGC und CGU deutlich häufiger auf. Das Nukleinsäuremolekül ist bevorzugteine genomische DNA, eine cDNA und/oder eine RNA. Die Kriterien zur Wahl geeigneter Codons und die Herstellung eines geeigneten Nukleinsäuremoleküls sind dem Fachmann bekannt.

Zur Expression der Erkennungsmoleküle insbesondere in Zellen sind Vektoren geeignet. Unter einem Vektor versteht man im Sinne der Erfindung ein Nukleinsäuremolekül, das zur Expression des Erkennungsmoleküls dient und das eine Nukleinsäuresequenz, die ein oder mehrere genetische Sequenzen umfasst, die mindestens eines der oben beschriebenen Erkennungsmoleküle kodieren, und insbesondere mindestens einen Promotor umfasst, der die Expression des Erkennungsmoleküls bewirkt. Vektoren können dabei selbstverständlich weitere Elemente umfassen, die dem Fachmann bekannt sind und die beispielsweise der Vermehrung von Vektoren zur Herstellung in geeigneten Zellen und zur Klonierung dienen. Die Nukleinsäuresequenzen können auf einem oder mehreren Vektoren vorliegen, beispielsweise wird in einer bevorzugten Ausführungsform die schwere Kette eines erfindungsgemäßen Immunglobulins durch einen und die leichte Kette durch einen anderen Vektor kodiert. Bevorzugt sind die variable Domäne der leichten Kette und die variable Domäne der schweren Kette auf dem gleichen Vektor unter einem Promotor als Fusionsprotein kodiert. Außerdem können Nukleinsäuresequenzen, die Teile eines Erkennungsmoleküls kodieren, durch unterschiedliche dem Fachmann bekannte Promotoren exprimiert werden. Des weiteren können die unterschiedlichen Nukleinsäuresequenzen auf einem gemeinsamen Vektor liegen. Dabei kann jede Sequenz durch einen eigenen, gleichen oder unterschiedlichen, Promotor exprimiert werden oder die Sequenzen können in einem bicistronischen Vektor unter einem Promotor vorliegen. Bevorzugt werden durch die unterschiedlichen Promotoren unterschiedliche Expressionsraten der Teile der Erkennungsmoleküle erreicht, die eine Bildung des gesamten Erkennungsmoleküls gegenüber einer gleichen Expressionsrate der verschiedenen Teile verbessert. Weiterhin bevorzugt werden Promotoren verwendet, die induzierbar sind, um eine Expression des Erkennungsmoleküls zu verbessern. Besonders bevorzugt umfassen die Vektoren weiterhin andere dem Fachmann bekannte regulatorische Elemente, beispielsweise Enhancer, die die Expression des Erkennungsmoleküls oder Teile davon verstärken, beispielsweise der CMV Enhancer oder Immunglobulin-Enhancer-Sequenzen. Bevorzugt umfassen die Nukleinsäuremoleküle und Vektoren zusätzlich Nukleinsäuresequenzen, die als Signalsequenzen zur Sekretion des Erkennungsmoleküls oder Teilen davon dienen, die dem Fachmann an sich bekannt sind, beispielsweise PelB, OmpA oder MalE für prokaryontische Zellsysteme bzw. das Signalpeptid des T-Zellrezeptors, der Immunglobulinketten, des t-PA oder EPO für eukaryontische Zellsysteme [Boel et al., 2000; Herrera et al., 2000]. Dies erleichtert vorteilhafterweise die Reinigung und/oder verbessert die Ausbeute der Erkennungsmoleküle. Die Verfahren zur Herstellung der oben beschriebenen Nukleinsäuren und Vektoren, geeigneter Promotoren, Enhancer und Vektorkonstrukte sowie die Kriterien zu deren Wahl sind dem Fachmann bekannt und werden in den Beispielen näher erläutert.

Insbesondere umfasst der Vektor weiterhin Nukleinsäuresequenzen, die für virale Proteine kodieren. Als eine besondere Form eines Vektors wird dabei der Virus selbst bezeichnet, dessen genetisches Material eine Nukleinsäuresequenz umfasst, die für ein erfindungsgemäßes Erkennungsmolekül kodiert. In einer bevorzugten Form ist das Erkennungsmolekül ein Fusionsprotein mit einem Virushüllprotein oder Teilen davon, das es ermöglicht, dass nicht nur das genetische Material die Nukleinsäuresequenz des Erkennungsmoleküls umfasst, sondern auch das Erkennungsmolekül selbst auf der Oberfläche des Virus bindungsaktiv vorliegt, beispielsweise ein erfindungsgemäßes scFv Erkennungsmolekül als Fusionsprotein mit einem Hüllprotein von für gentherapeutische Anwendungen geeigneten Adenoviren, Poxviren oder Vacciniaviren. Dies vermittelt die Adressierung des Virus zu einer Core-1 exprimierenden Tumorzelle, wodurch es zur Expression des Erkennungsmoleküls in der Tumorzelle kommt. Dies kann zur Expression des Erkennungsmoleküls in vivo im Organismus oder in vitro in der Zellkultur verwendet werden. Bevorzugt werden dabei bekannte Systeme verwendet, die einen Helfervirus zur Replikation verwenden, um beispielsweise die Sicherheit eines diesen Vektor umfassenden gentherapeutischen Verfahrens zu gewährleisten. Die Verfahren zur Herstellung der beschriebenen viralen Vektoren, zur Infektion und Expression der Erkennungsmoleküle sind dem Fachmann bekannt.

Der Vektor kann ein Fusionsprotein aus einem erfindungsgemäßen Erkennungsmolekül und einem Protein oder Peptid, das spezifisch an ein Virus bindet, umfassen. Die gewonnenen Erkennungsmoleküle können so mit Vorteil zur Adressierung des Virus an eine Core-1 exprimierende Zelle verwendet werden. So kann z.B. der Transfer des genetischen Materials über Infektionen vermittelt werden, wodurch es ermöglicht wird, spezifische Moleküle, die durch das genetische Material des Virus kodiert werden, in den Zellen in vivo im Organismus in Form einer Gentherapie oder in vitro in der Zellkultur zu exprimieren.

Weiterhin betrifft die Erfindung ein Verfahren zur Gewinnung der Erkennungsmoleküle umfassend das Einbringen von ein oder mehreren Vektoren, die ein oder mehrere Nukleinsäuremoleküle enthalten, in eine geeignete Wirtszelle, die Kultivierung dieser Wirtszelle unter geeigneten Bedingungen und die Bereitstellung von ein oder mehreren Erkennungsmolekülen aus den Zellen oder dem Kulturmedium. Unter dem Begriff "Einbringen von Vektoren" versteht man im Sinne der Erfindung dem Fachmann an sich bekannte Technologien mit denen der Vektor in eine Wirtszelle gebracht wird, beispielsweise Elektroporation, Transfektion unter Verwendung kationischer Lipide oder Infektion und dort transient oder stabil verbleibt. Unter dem Begriff "Bereitstellung von einem oder mehreren Erkennungsmolekülen" versteht man im Sinne der Erfindung dem Fachmann an sich bekannte Technologien mit denen die während des Kultivierungsprozesses exprimierten Erkennungsmoleküle aus dem Kulturüberstand und/oder Zellen gewonnen werden, beispielsweise unterschiedliche proteinchemische Reinigungsschritte beispielsweise Fraktionierung, Konzentrierung, Fällungen, und/oder Chromatographie. Die in dem Verfahren zu verwendenden Techniken und Methoden sind dem Fachmann bekannt, ebenso ist der Fachmann in der Lage, geeignete Wirtszellen und Kultivierungsbedingungen sowie Methoden zur Bereitstellung aus den Zellen und/oder dem Kulturüberstand auszuwählen. Hierbei wählt der Fachmann beispielsweise, wie bereits oben ausgeführt, Nukleinsäuresequenzen mit geeigneten Codons und Promotorsequenzen abgestimmt auf die Wirtszelle, um eine möglichst starke Expression von aktiven Erkennungsmolekülen zu gewinnen. In einer bevorzugten Ausführungsform verwendet der Fachmann bespielsweise affinitätschromatographische Schritte, beispielsweise Chromatographie an Protein A oder Protein G oder Protein L oder beipielsweise Metallionen-Affinitätschromatographie über einen zusätzlich eingefügten His-Tag. In den Beispielen ist dies beispielhaft näher ausgeführt.

Der Begriff "Gewinnung" umfasst neben den zuvor explizit genannten Schritten auch zusätzliche Schritte, wie etwa Vorbehandlungen des Ausgangsstoffes oder Weiterbehandlungen des Endproduktes. Vorbehandlungsverfahren sind an sich dem Fachmann bekannt. Weiterbehandlungsverfahren umfassen neben den oben beschrieben Bereitstellungsverfahren beispielsweise auch die endgültige Zusammensetzungen und/oder Formulierung des mit dem Herstellungsverfahren gewonnenen Erkennungsmoleküls in geeigneten Verwendungs- und/oder Darreichungsformen. Die Art der Verwendungs- oder Darreichungsform, z.B. Lösung, Lyophilisat oder Tablette, hängt hierbei von der beabsichtigten Verwendung ab. Dem Fachmann ist hierbei bekannt, welche Darreichungsform sich für welchen Verwendungszweck eignet. Je nach Darreichungsform kann das durch das erfindungsgemäße Verfahren hergestellte Erkennungsmolekül zusammen mit Hilfs-, Träger- oder weiteren Wirkstoffen vorliegen. Hilfsstoffe sind hierbei vorzugsweise Adjuvantien, weitere Wirkstoffe, vorzugsweise immunstimulatorische Moleküle, wie Interleukine. Das mit dem erfindungsgemäßen Verfahren hergestellte Erkennungsmolekül kann auch in Weiterbehandlungsschritten chemisch modifiziert werden. Vorzugsweise wird das Erkennungsmolekül hierbei mit einem oder mehreren weiteren Molekülen in geeigneter Weise, d.h. durch chemische oder physikalische Interaktion, verbunden. Als weitere Moleküle im Sinne der Erfindung dienen bevorzugt anderen Proteine oder Peptide, die mit dem durch das erfindungsgemäße Verfahren hergestellten Erkennungsmolekül kovalent oder nicht-kovalent verknüpft werden, beispielsweise um bispezifische Erkennungsmoleküle herzustellen, indem ein erfindungsgemäßes Erkennungsmolekül, das spezifisch das Core-1 Antigen erkennt, mit einem zweiten Molekül verknüpft wird, das beispielsweise eine Immuneffektorzelle (beispielsweise Makrophage, NK-Zellen, Dendritische Zellen) spezifisch bindet oder beispielsweise eine Verknüpfung mit Interleukinen (beispielsweise IL-2, IL-7, IL-12, IL-15), Chemokinen oder Wachstumsfaktoren, wodurch über die Wirkung dieser Moleküle über die Bindung des erfindungsgemäßen Erkennungsmoleküls Immuneffektoren an die Core-1 positiven Tumorzellen dirigiert werden und diese beispielsweise bekämpfen und/oder zerstören. Diese weiteren Moleküle oder Teile davon können wie bereits weiter oben beschrieben auch Teil des Erkennungsmoleküls selbst sein und werden in diesem Fall nicht über die hier beschriebenen chemischen oder physikalischen Methoden nach der Expression des Erkennungsmoleküls verknüpft. Unter "Immuneffektoren" versteht man im Sinne der Erfindung solche Komponenten der Erfindung die direkt oder indirekt eine Bekämpfung und/oder Zerstörung von Core-1 positiven Tumorzellen bewirken können, beispielsweise Immuneffektorzellen, wie beispielsweise Makrophagen, NK-Zellen, Dendritische Zellen, oder Effektormoleküle, wie beispielsweise Proteine oder Peptide des Komplementsystems. Als weitere Moleküle im Rahmen des erfindungsgemäßen Verfahrens sind besonders Substanzen geeignet, die eine therapeutische oder diagnostische Wirkung entfalten, beispielsweise Radioisotope oder Toxine. Diese Substanzen werden über an sich bekannte Verfahren mit den Erkennungsmolekülen verknüpft, beispielsweise werden Radioisotope entweder direkt eingelagert (beispielsweise Iod) oder über einen kovalent gekoppelten Chelator (beispielsweise Yttrium, Indium, Bismut) gebunden. Die Schritte des Weiterbehandlunqsverfahrens sind dem Fachmann bekannt.

Die zur Expression der Erkennungsmoleküle erfindungsgemäß verwendeten Zellen können prokaryontische oder eukaryontische Zellen sein, beispielsweise Bakterien-, Hefe- (bevorzugt S.cerevisiae oder P.pastoris), Insekten- (D.melanogaster), Pflanzen-, Säugerzellen (bevorzugt Hamster-, Maus- oder humane Zelllinien) oder Organismen wie transgene Tiere und Pflanzen. Vorzugsweise werden zur Expression der erfindungsgemäßen Erkennungsmoleküle in einem prokaryontischen System E.coli und zur Expression in einem eukaryontischen System die Säugerzelllinien NS0 SP2/0, CHO-K1, CHOdhfr-, COS-1, COS-7, HEK293, K562, Namalwa oder Percy 6 verwendet.

Mit Hilfe von Wirtszellen, die durch das oben beschriebene Verfahren hergestellt wurden, können erfindungsgemäße Erkennungsmoleküle hergestellt werden. Selbstverständlich können die Wirtszellen Teil eines Klons sein oder ihn selber darstellen. Organismen wiederum umfassen diese Wirtszellen. Die zu verwendenden Techniken und Methoden zur Herstellung dieser Organismen sind dem Fachmann bekannt.

Weiterhin sind Zusammensetzungen für therapeutische, prophylaktische oder diagnostische Zwecke geeignet, umfassend mindestens ein erfindungsgemäßes Erkennungsmolekül in einer geeigneten, insbesondere einer pharmazeutisch geeigneten Form oder Zusammensetzung. Die pharmazeutische Zusammensetzung umfaßt insbesondere zusätzliche Stoffe und Substanzen, beispielsweise medizinische und/oder pharmazeutisch-technische Hilfsstoffe. Im Sinne der Erfindung gelten als Arzneimittel sowohl solche pharmazeutischen Zusammensetzungen, die für therapeutische und prophylaktische Zwecke verwendet werden, als auch solche pharmazeutischen Zusammensetzungen, die in vivo als Diagnostikum eingesetzt werden. Bevorzugt handelt es sich um Zusammensetzungen für die ex vivo Diagnostik die zusätzliche Stoffe und Substanzen enthalten können. Diese Ausführungsform ist unter der Beschreibung für die Diagnostika näher ausgeführt.

"Arzneimittel oder pharmazeutische Zusammensetzungen", die vorliegend synonym verwendet werden, sind erfindungsgemäß Stoffe und Zubereitungen aus Stoffen, die dazu bestimmt sind, durch Anwendung am oder im menschlichen Körper Krankheiten, Leiden, Körperschäden oder krankhafte Beschwerden zu heilen, zu lindern oder zu verhüten. Medizinische Hilfsstoffe sind erfindungsgemäß solche Stoffe, die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Beispiele für pharmazeutisch verträgliche Träger sind nachstehend aufgeführt. Die Arzneimittelformulierung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen z.B. Phosphat-gepufferte Kochsalzlösungen, Wasser, Emulsionen wie z.B. Öl/Wasser-Emulsionen, verschiedene Arten von Detergenzien, sterile Lösungen, etc. Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, z.B. in einem Bereich von 1µg bis 10 g an Erkennungsmolekülen pro Tag und Patient Bevorzugt werden dabei Dosen von 1 mg bis 1 g. Die Verabreichung kann auf verschiedenen Wegen erfolgen, beispielsweise intravenös, intraperitoneal, intrarektal, intragastrointestinal, intranodal, intramuskulär, lokal, beispielsweise in den Tumor, aber auch subkutan, intradermal oder auf der Haut oder über die Schleimhäute. Die Verabreichung von Nukleinsäuren kann auch in Form von Gen-Therapie geschehen, beispielsweise über weiter oben beschriebene virale Vektoren. Die Art der Dosierung und des Verabreichungsweges kann vom behandelnden Arzt entsprechend den klinischen Faktoren bestimmt werden. Es ist dem Fachmann bekannt, dass die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Die pharmazeutischen Zusammensetzungen oder das Arzneimittel umfasst insbesondere eine pharmakologische Substanz, die ein oder mehrere erfindungsgemäße Erkennungsmoleküle oder/und diese kodierende Nukleinsäuremoleküle in einer geeigneten Lösung oder Verabreichungsform enthält. Diese können entweder alleine mit den entsprechenden unter Arzneimitteln oder pharmazeutischen Zusammensetzungen beschriebenen Hilfsstoffen oder in Kombination mit einem oder mehreren Adjuvantien, beispielsweise QS-21, GPI-0100 oder andere Saponine, Wasser-Öl Emulsionen wie beispielsweise Montanide Adjuvantien, Polylysin, Polyargininverbindungen, DNA-Verbindungen wie beispielsweise CpG, Detox, bakterielle Vakzine wie beispielsweise Thyphusvakzine oder BCG-Vakzine, und/oder einem anderen geeigneten Stoff zur Wirkungsverstärkung verabreicht werden; vorzugsweise immunstimulatorische Moleküle, wie Interleukine, beispielsweise IL-2, IL-12, IL-4 und/oder Wachstumsfaktoren, beispielsweise GM-CSF. Diese werden in bekannten Methoden mit den erfindungsgemäßen Erkennungsmolekülen gemischt und in einer geeigneten Formulierung und Dosierung verabreicht. Formulierungen, Dosierungen und geeignete Komponenten sind dem Fachmann bekannt.

Die pharmazeutische Zusammensetzung oder das Arzneimittel kann selbstverständlich auch eine Kombination von 2 oder mehreren der erfindungsgemäßen pharmazeutischen Zusammensetzungen oder Arzneimittel sein, sowie eine Kombination mit anderen Arzneimitteln, Tumorvakzinen oder Tumorbehandlungen, wie beispielsweise Antikörpertherapien, Chemotherapien oder Radiotherapien, die auf eine geeignete Weise zeitlich gemeinsam oder getrennt verabreicht beziehungsweise angewandt werden. Herstellung der Arzneimittel oder pharmazeutischen Zusammensetzungen erfolgt nach an sich bekannten Methoden.

Die Arzneimittel oder pharmazeutischen Zusammensetzungen können insbesondere zur Behandlung von Core-1 positiven Tumorerkrankungen eingesetzt werden, wie beispielsweise Mammakarzinome, Zervikalkarzinome, Ovarialkarzinome, Colonkarzinome, Gastrointestinalkarzinome, Pankreaskarzinome, Lungenkarzinome, Prostatakarzinome. Zu diesen Tumorerkrankungen können auch Core-1 und/oder Core-2 positive Tumorerkrankungen gehören. Die Behandlung geht beispielsweise gegen Primärtumoren, minimal residuale Tumorerkrankungen, Relapses und/oder Metastasen. Die Behandlung der Tumoren kann auch als adjunvante Behandlung erfolgen. Die Verwendung der Arzneimittel kann auch zur Prophylaxe von Core-1 positiven Tumorerkrankungen erfolgen. Die prophylaktische Anwendung zielt beispielsweise auf eine Prophylaxe des Tumors sowie von Metastasen. Die Tumormittel werden in einer geeigneten Form nach bekannten Methoden verabreicht. Eine bevorzugte Variante ist die Injektion beziehungsweise Verabreichung der Arzneimittel intravenös, lokal in Körperkavitäten, beispielsweise intraperitoneal, intrarektal, intragastrointestinal, lokal beispielsweise direkt in den Tumor, Organe oder Lymphgefäße (intranodal), aber auch subkutan, intradermal oder auf der Haut, intramuskulär. Verabreichungsarten können bevorzugterweise auch kombiniert werden, wobei sie an verschiedenen Behandlungstagen oder an einem Behandlungstag verabreicht werden können. Dabei können erfindungsgemäß auch 2 oder mehrere der erfindungsgemäßen Arzneimittel oder pharmazeutischen Zusammensetzungen kombiniert werden oder eine oder mehrere erfindungsgemäße Arzneimittel mit ein oder mehreren Arzneimitteln oder Tumorbehandlungen, wie beispielsweise Antikörpertherapien, Chemotherapien oder Radiotherapien, die zeitlich gemeinsam oder getrennt verabreicht beziehungsweise angewandt werden.

Ein Arzneimittel oder eine pharmazeutische Zusammensetzung werden in einem Verfahren hergestellt, umfassend die Schritte der Herstellung von Erkennungsmolekülen und weiterhin umfassend den Schritt der Formulierung der erfindungsgemäßen Erkennungsmoleküle in pharmazeutisch verträglicher Form. Die hierfür bevorzugten erfindungsgemäßen Erkennungsmoleküle sind weiter oben als Ausführungsformen zur Behandlung von Tumorerkrankungen und Prophylaxe, sowie weiter unten unter in vivo Diagnostika näher beschrieben.

Die erfindungsgemäßen Erkennungsmoleküle und durch das Verfahren hergestellten Stoffe und Zusammensetzungen können demgemäß bevorzugt zur Prophylaxe, Diagnose, Verlaufskontrolle und/oder Behandlung von Tumorerkrankungen verwendet werden. Die Verwendung der Erkennungsmoleküle, der Vektoren und/oder des Arzneimittels oder der pharmazeutischen Zusammensetzung zur Prophylaxe und/oder Behandlung von Krebserkrankungen, einschließlich Tumoren und Metastasen ist weiterhin bevorzugt.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (z.B. Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nichtkleinzelliges Bronchialkarzinom, Haferzellen-Karzinom, papilläres Karzinom, szirrhöses Karzinom, bronchioloalveoläres Karzinom, Bronchiai-Karzinom, Plattenepithelkarzinom und Transitionalzell-Karzinom); histiocytische Funktionsstörung; Leukämie (z.B. in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-ZellTumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (z.B. Ewing-Sarkom, experimentell, Kaposi-Sarkom und Niastzell-Sarkom); Neoplasmen (z.B. Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe von Krebserkrankungen oder Tumorerkrankungen, die Zellen umfassen, die das Core-1 in der erfindungsgemäßen Definition umfassen, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantationsbezogene Malignitäten, metastasierte Tumoren umfassend Gehirnmetastasen, Lungenmetastasen, Lebernetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

Die erfindungsgemäßen Erkennungsmoleküle können bei der Behandlung oder Prophylaxe von Tumorerkrankungen direkt eingesetzt werden oder mit zusätzlichen Effektorstrukturen gekoppelt werden. Unter "Effektorstrukturen" versteht man erfindungsgemäß solche chemischen oder biochemischen Verbindnungen, Moleküle oder Atome, die direkt oder indirekt eine Abtötung oder Schädigung, einschließlich beispielsweise Wachstumsverlangsamung oder Wachstumsinhibition von Tumorzellen bewirken. Hierzu gehören beispielsweise Radioisotope, Toxine, Cytostatika und andere Effektormoleküle wie beispielsweise Cytokine und Chemokine oder andere Strukturen, die selbst Effektoren darstellen oder an die Effektormoleküle gekoppelt werden, beispielsweise mit Toxinen oder Cytostatika beladene Liposomen, die erfindungsgemäße Erkennungsmoleküle tragen. Beim letzteren Beispiel der Liposomen sind insbesondere auch solche Effektorstrukturen gemeint, die neben dem Erkennungsmolekül für die Tumorspezifität auch solche Moleküle tragen, die für eine Aufnahme der Effektorstrukturen oder Teile davon in die Zellen verantwortlich sind, wie beispielsweise Antikörper gegen Rezeptoren, die eine Rezeptor-vermittelte Endozytose bewirken. Vorzugsweise umfassen die Erkennungsmoleküle in diesen Fällen eine Transmembrandomäne, die ihnen eine Insertion in die Liposomenmembran erlaubt oder in einer anderen bevorzugten Ausführungsform werden die Erkennungsmoleküle chemisch auf die Liposomenoberfläche gekoppelt. Die hierfür verwendeten Techniken sind dem Fachmann bekannt, einschließlich der Herstellung der Liposomen. Auch die Verbindung der Erkennungsmoleküle mit den anderen Effektorstrukturen erfolgt nach an sich bekannten Methoden. Die Kopplungen können dabei wie bereits oben ausgeführt beispielsweise direkt durch kovalente oder nicht-kovalente Beladung erfolgen, durch chemische Kopplung, wobei ein zusätzliches chemisches oder biologisches Molekül notwendig sein kann, beispielsweise ein Chelator oder ein Linker, oder in Form von Fusionsproteinen oder -peptiden durch Fusion. Eingesetzt werden die Erkennungsmoleküle bei der Behandlung von Tumorerkrankungen mit Core-1 tragenden Tumoren, und/oder für eine Untergruppe an erfindungsgemäßen Erkennungsmolekülen, die weiter oben über ihre Spezifität für Core-1 und Core-2 beschrieben sind, Core-2 und/oder Core-1 tragenden Tumorzellen oder zur Prophylaxe, die beispielsweise die Ausbildung von primären Tumoren oder Metastasen verhindert. Bevorzugtes Ziel ist dabei die Behandlung der minimalen residualen Erkrankung und von Metastasen. Eine weitere bevorzugte Anwendung ist dabei die Inhibition der Lebermetastasierung von Core-1 und/oder Core-2 positiven Tumorzellen. Die erfindungsgemäßen Erkennungsmoleküle werden dabei in einer geeigneten Formulierung einmalig oder wiederholt in geeigneten zeitlichen Abständen und Dosen verabreicht.

Im Folgenden und davor versteht man im Sinne der Erfindung unter dem Core-1 Antigen auch Core-1 und/oder Core-2 und unter Core-1 positiven Zellen oder Tumorzellen und/oder -geweben auch Core-1 und/oder Core-2 positive Zellen oder Tumorzellen und/oder -gewebe.

In einer bevorzugten Ausführungsform werden die erfindungsgemäßen oben beschriebenen radioaktiven Erkennungsmoleküle mit einer Applikation von nicht markierten erfindungsgemäßen Core-1 spezifischen Erkennungsmolekülen kombiniert. Dies dient der Verbesserung des Hintergrundes und damit einer spezifischeren Bindung an den Tumor, indem potentielle Core-1 tragende Moleküle im Blut abgesättigt werden. Bevorzugt werden dabei IgM abgleitete Erkennungsmoleküle verwendet, beispielsweise der in den Beispielen beschriebene cIgM oder die humanisierte Form davon, da diese vor allem an Core-1 Antigen im Blut binden und damit den Hintergrund und die Serumbelastung mit Radioaktivität erniedrigen und das relative Tumortargeting erhöhen, während aufgrund der Größe der Moleküle ein Eindringen in Gewebe und Tumoren limitiert ist. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

Virale Vektoren können zur gentherapeutischen Anwendung kommen, bei der insbesondere die Oberfläche der Viren erfindungsgemäße Erkennungsmoleküle tragen.

Unter Verwendung der erfindungsgemäßen Erkennungsmoleküle ist es möglich, aus einem großen Pool unterschiedlicher Moleküle Core-1 tragende Moleküle zu identifizieren und/oder zu gewinnen, die für eine Anwendung in der Tumorbehandlung, Tumorprophylaxe und Tumordiagnose vorteilhaft verwendet werden können. Unter Core-1 tragenden Molekülen versteht man erfindungsgemäß Moleküle, die Core-1 und/oder Core-2 Strukturen tragen und spezifisch von den erfindungsgemäßen Erkennungsmolekülen gebunden werden. Erfindungsgemäß sind Core-1 tragende Moleküle Glykoproteine, Glykopeptide und/oder Glykolipide sowie auch Zellen oder andere Trägersubstanzen, wie beispielsweise Viren, Bakterien, Teile von Zellen, wie beispielsweise Exosomen oder Zelllysate, oder Liposomen, die ein oder mehrere Core-1 Strukturen enthalten. Die Core-1 tragenden Moleküle können aus Zellen oder Zelllinien, aus Kulturüberständen, aus Tumorgewebe, Tumorzellen oder Körperflüssigkeiten, wie Blut, Blutserum, Lymphe, Urin, Spinalflüssigkeit oder Sperma angereichert oder isoliert werden.

Die zuvor eingeführten Definitionen der Begriffe sind für die Begriffe in den nachfolgend beschriebenen Verfahren mutatis mutandis anwendbar.

Core-1 tragende Moleküle werden in einem Verfahren durch Bindung an die oben beschriebenen erfindungsgemäßen Core-1 spezifischen Erkennunasmoleküle identifiziert und/ oder isoliert und gewonnen. Nach dem Verfahren können die oben beschriebenen Core-1 tragenden Moleküle aus Körperflüssigkeiten oder aus Überständen von Zellkulturen durch eine Affinitätschromatographie gewonnen werden. Dabei können weitere Reinigungs- und/oder Konzentrierungsschritte nach an sich bekannten Methoden mit einem oder mehreren Affinitätschromatographieschritten kombiniert werden. Ebenso können tumorassoziierte Core-1 tragende Moleküle aus Tumorzellen, Tumorgeweben oder Tumorzelllinien gewonnen werden, indem ein geeigneter Schritt nach an sich bekannten Methoden vorgeschaltet wird, der es erlaubt, die zellassoziierten Core-1 tragenden Moleküle für die Affinitätsreinigung zugänglich zu machen, beispielsweise durch Solubilisierung mit geeigneten Detergenzien oder durch Abspaltung durch Proteolyse oder durch Zelllyse.

In einem weiteren Verfahren werden Core-1 tragende Moleküle oder Zellen aus Geweben gewonnen. Hierzu wird das Gewebe nach an sich bekannten Methoden aufgeschlossen, um die Core-1 tragenden Moleküle oder Zellen zugänglich zu machen, beispielsweise durch proteolytische oder mechanische Methoden. Dem Fachmann sind diese Verfahren bekannt.

Wie oben ausgeführt werden auch Core-1 positive Zellen oder Zelllinien unter Verwendung der Core-1 spezifischen Erkennungsmoleküle isoliert oder angereichert und von solchen Zellen, die keine oder geringe Mengen an Core-1 Strukturen tragen, getrennt. Unter dem Begriff "Isolierung oder Anreicherung der Zellen" sind alle Maßnahmen zur Separierung von Zellen zu verstehen, die durch das Tragen von Core-1 Strukturen einen Komplex mit den erfindungsgemäßen Erkennungsmolekülen gebildet haben. Dem Fachmann sind diese Verfahren bekannt. Vorzugsweise wird hierfür die FACS oder die MACS Methode eingesetzt. Beispielsweise erfolgt die Anreicherung durch Bindung von erfindungsgemäßen Erkennungsmolekülen an die Core-1 Struktur auf der Zelloberfläche und anschließender Selektion der so markierten Zellen durch Bindung an Trägermaterialien, die spezifisch mit dem Erkennungsmolekül interagieren, beispielsweise anti-Maus IgM Antikörper gekoppelt an Magnetbeads (MACS-Sortierung). Außerdem können die Core-1 spezifischen Erkennungsmoleküle selbst kovalent an einen Träger gekoppelt sein. Ein weiteres Beispiel ist die Gewinnung mit Hilfe eines FACS-Sorters, der Zellen, die die Erkennungsmoleküle tragen, die fluoreszenzmarkiert wurden, sortiert. Beide Methoden sind dem Fachmann bekannt. Diese so angereicherten Core-1 positiven Zellen können für die Herstellung von Vakzinen verwendet werden, beispielsweise zur Beladung von Dendritischen Zellen oder direkt als Tumorzelllysat in einer Vakzinen Zusammensetzung. Die vorhergehende Anreicherung von Core-1 positiven Zellen soll zu einer höheren Tumorspezifität der Vakzinierung führen. Dem Fachmann sind diese Verfahren bekannt.

Die vorliegende Erfindung betrifft weiterhin Verfahren zur Herstellung eines Diagnostikums umfassend die Schritte des o.g. Verfahrens zur Herstellung der erfindungsgemäßen Core-1 spezifischen Erkennungsmoleküle und weiterhin umfassend den Schritt der Formulierung der Erkennungsmoleküle in einer diagnostisch verwendbaren Form

Unter dem Begriff "Diagnostikum" sind erfindungsgemäß Stoffe und Zubereitungen aus Stoffen definiert, die dazu bestimmt sind, durch Anwendung am oder im menschlichen Körper oder Teilen davon Krankheiten, Leiden, Körperschäden oder krankhafte Beschwerden zu erkennen. Als Teile des menschlichen Körpers sind vorzugsweise Körperflüssigkeiten, wie Blut, Blutserum, Lymphe, Urin, Spinalflüssigkeit oder Sperma, oder Gewebebiopsien oder -proben zu verstehen.

Die Formulierung des Diagnostikums umfasst vorzugsweise die Modifikation der hergestellten Erkennungsmoleküle mit Substanzen, die einen Nachweis des Antigens Core-1, in bestimmten Ausführungsformen, die von der Feinspezifität des erfindungsgemäßen Erkennungsmoleküls abhängen, definitionsgemäß auch das Antigen Core-2, erlauben. Geeignete Substanzen sind im Stand der Technik bekannt. Ausgehend von der Wahl der Substanz ist der Fachmann in der Lage, geeignete Maßnahmen zur Formulierung des Diagnostikums einzusetzen.

Für die Diagnostik können erfindungsgemäß auch Substanzen nach an sich bekannten Methoden an die Erkennungsmoleküle gekoppelt werden, die einen Nachweis der Core-1 Antigene und/oder deren Trägermoleküle und/oder -zellen erleichtern, beispielsweise durch Biotinylierung, Fluoreszenzmarkierung, radioaktive Markierung oder Enzymkopplung der Erkennungsmoleküle.

In einem weiteren Verfahren zur Tumordiagnostik und Prognose werden erfindungsgemäße Erkennungsmoleküle verwendet, die Core-1 Antigene und/oder deren Trägermoleküle im Serum von Menschen erkennen. Die Bestimmung erfolgt bevorzugt qualitativ, quantitativ und/oder in zeitlich relativen Quantitäten nach an sich bekannten Methoden. Die gleichen Verfahren werden auch zur Verlaufskontrolle von Tumorerkrankungen und zur Kontrolle von Behandlungsverläufen einschließlich des Monitorings von Immunantworten und zur Kontrolle und Dosierung von Tumorbehandlungen eingesetzt. Die in den Verfahren verwendeten Methoden sind an sich bekannt, beispielsweise ELISA, Westerblot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), Immuncytochemie und Immunhistochemie.

In den bevorzugten Verfahren zur Tumordiagnostik und Prognose werden erfindungsgemäße Core-1 spezifische Erkennungsmoleküle in an sich bekannten Verfahren eingesetzt, um das Antigen Core-1 im Serum oder in Gewebspräparaten nachzuweisen. Dabei wird das Antigen Core-1 auf Trägermolekülen, in Immunkomplexen auf Trägermolekülen vorliegendes Core-1 und/oder auf Zellen gebundenes Core-1 nachgewiesen und das Vorhandensein des Core-1 Antigens und/oder der Core-1 tragenden Moleküle qualitativ, quantitativ und/oder in relativen Quantitäten nach an sich bekannten Methoden bestimmt. Die gleichen Verfahren werden auch zur Verlaufskontrolle von Tumorerkrankungen und zur Kontrolle von Behandlungsverläufen eingesetzt. Die in den Verfahren verwendeten Methoden sind an sich bekannt, beispielsweise ELISA, Western-Blot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), Immuncytochemie und Immunhistochemie.

Bevorzugt ist ein Gewebsschnelltest, bei dem in einem immunhistologischen Verfahren die Gewebsproben mit fluoreszenzmarkierten erfindungsgemäßen Erkennungsmolekülen gefärbt werden. In einem weiter bevorzugten Verfahren wird das erfindungsgemäße Erkennungsmolekül, bevorzugt ein Antikörper des Isotyps IgM, mit einem weiteren Antikörper, der spezifisch das Antigen MUC1 erkennt, bevorzugt Isotyp IgG1, kombiniert. Der Vorteil dabei ist, dass beispielsweise für die Diagnostik von gastrointestinalen Karzinomen (z.B. Colorektale Karzinome und Magenkarzinome) diese in einem frühen Stadium erkannt und gleichzeitig eine Prognose bezüglich des Krankheitsverlaufes und/oder der Lebermetastasierungsgefahr gegeben werden kann, wobei ein höheres Niveau an Core-1 Antigen eine schlechtere Verlaufsprognose und eine um das mehrfach höhere Wahrscheinlichkeit der Lebermetastasierung bedeutet. Weiterhin können die Antikörper und Erkennungsmoleküle bevorzugt direkt mit unterschiedlichen Fluoreszenzfarbstoffen, beispielsweise Cy3 und Cy5 oder Cy3 und FITC, markiert sein. Wenn eine Signalverstärkung vorteilhaft ist, werden die Antikörper und/oder Erkennungsmoleküle durch markierte sekundäre Antikörper oder das Biotin-Streptavidin verstärkt. Dabei ist es vorteilhaft, unterschiedliche Isotypen und/oder Speziessequenzen im konstanten Teil der Antikörper zu verwenden. Die hierbei verwendeten Technologien und Methoden, beispielsweise der Markierung und der Immunhistologie, sowohl die Wahl der geeigneten Formate der Erkennungsmoleküle sind dem Fachmann bekannt. Das beschriebene diagnostische Verfahren ist nicht auf gastrointestinale Tumoren beschränkt, sondern anwendbar für alle das Antigen Core-1 tragende Tumorerkrankungen. wird. Dieser besteht aus einem Fängerantikörper, der Trägermoleküle des Core-1 Antigens aus dem Serum an eine feste Phase bindet, und einem Nachweisantikörper, hierunter fallen auch andere erfindungsgemäße Erkennungsmoleküle, die das Core-1 Antigen erkennen. Damit kann unterschieden werden, welches Trägermolekül das Core-1 trägt. In einer bevorzugten Form kann damit auf den Ursprung des Primärtumors Rückschlüsse gezogen werden. Als Fängerantikörper können verschiedene Antikörper dienen, die Glykoproteine erkennen, die O-Glykosylierungen tragen. Bevorzugt verwendet werden Antikörper gegen das eoitheliale Muzin MUC1 als Fängerantikörper, das häufig ein Träger des Core-1 im Tumorfall ist. Alternativ werden alle Antigene im Blut bestimmt, die das Core-1 Antigen tragen. Dies ist dadurch möglich, dass das Core-1 Antigen in der Regel in mehreren Kopien pro Trägermolekül vorkommt. Hierbei wird ein erfindungsgemäßes Core-1 spezifisches Erkennungsmolekül als Fängerantikörper verwendet und ein markiertes erfindungsgemäßes Core-1 spezifisches Erkennungsmolekül als Nachweisantikörper, wobei die Erkennungsmoleküle keine Antikörper sein müssen. Bevorzugt wird ein IgM als Erkennungsmolekül mindestens als Fänger- oder Nachweisantikörper verwendet. Weiter bevorzugt wird der Nachweisantikörper mit Biotin markiert und das System über Streptavidin in Kombination mit einem geeigneten Nachweisverfahren nachgewiesen. Ein geeignetes Nachweisverfahren sind beispielsweise POD Markierungen oder Fluoreszenzmarkierungen des Streptavidins.

Bevorzugt werden für einen serologischen Tumortest die Bestimmung des Core-1 Antigens, wie zuvor beschrieben, mit der Bestimmung anderer serologischer Tumormarker kombiniert, beispielsweise PSA, CEA oder AFP. Eine hierbei bevorzugte Ausführungsform ist die Bestimmung des MUC1 und des Core-1 Antigens. Bevorzugt wird hierbei das MUC1 mit Hilfe eines MUC1 spezifischen Antikörpers aus dem Serum an eine feste Phase immobilisiert und mit einem zweiten anti-MUCl spezifischen Antikörper, bevorzugt solche, die die DTR-Region in einer glykosylierten Form verbessert erkennen, als Nachweisantikörper nachgewiesen und das Core-1 Antigen auf dem mit Hilfe eines anti-MUC1 Fängerantikörpers immobilisierten MUC1 mit einem erfindungsgemäßen Erkennungsmolekül nachgewiesen. Dieser diagnostische Test verbindet eine Früherkennung mit einer prognostischen Aussage über den Krankheitsverlauf und/oder der Lebermetastasierungswahrscheinlichkeit. Die hierbei verwendeten Technologien, beispielsweise der Markierung und der Serologie, einschließlich der Nachweismethoden, sind dem Fachmann bekannt. Die beschriebenen diagnostischen Verfahren sind nicht auf gastrointestinale Tumoren beschränkt, sondern anwendbar für alle das Antigen Core-1 tragende Tumoren. Die beschriebenen serologischen Tests dienen der Diagnose, des Monitorings des Verlaufs der Tumorerkrankung und der Prognose von Core-1 Antigen-positiven Tumoren.

In einem weiteren Verfahren werden die erfindungsgemäßen Core-1-spezifischen Erkennungsmoleküle zu einer in vivo Diagnostik verwendet. Hierfür werden die Erkennungsmoleküle mit geeigneten an sich bekannten Verfahren markiert und somit für an sich bekannte bildgebende Verfahren am Menschen zugänglich gemacht, beispielsweise Radioimmundiagnostik, PET-Scan Verfahren oder Immunofluoreszenzendoskopie, beispielweise durch Kopplung und/oder Beladung mit entsprechenden Molekülen, beispielsweise radioaktiven Isotope, beispielsweise das Indium, oder Fluoreszenzfarbstoffe, beispielsweise dem Cy3, Cy2, Cy5 oder FITC. In einer bevorzugten Ausführungsform werden erfindungsgemäße Multibodies mit einem geeigneten Chelator (beispielsweise DOTA oder DTPA) kovalent gekoppelt und mit Indium-111 beladen und zur in vivo Diagnostik eingesetzt. Diese werden bevorzugt intravenös in einer für das Individuum geeigneten Dosis verabreicht und die Lokalisation des Core-1 Antigens und eines potentiellen Tumors nach an sich bekannten Verfahren gemessen. Die hierfür verwendeten Verfahren und Technologien, einschließlich der bildgebenden Verfahren, sind dem Fachmann bekannt, ebenfalls kann der Fachmann eine geeignete Dosis und Formulierungen erstellen.

Weiterhin werden bevorzugt Immunglobuline, bevorzugt IgM und IgG, wie oben beschrieben und in den Beispielen näher ausgeführt, radioaktiv-markiert, beispielsweise mit Indium-111, und lokal in den Tumor oder in den Tumor versorgende oder entsorgende Blutgefäße gegeben. Dies dient zur Bestimmung der Größe des Tumors oder der Bestimmung befallener Lymphknoten. Die hierfür verwendeten Verfahren und Technologien sind dem Fachmann bekannt, ebenfalls kann der Fachmann eine geeignete Dosis und Formulierungen erstellen.

Die erfindungsgemäßen radioaktiv-markierten Erkennungsmoleküle können auch über andere Applikationsrouten verabreicht werden. Dabei bevorzugte Routen sind intraperitoneal, intranodal oder intrarectal bzw. intragastrointestinal. Intraperitoneal ist dabei besonders vorteilhaft zur Bestimmung von Tumoren, die über das Peritoneum zugänglich sind und/oder in dieses metastasieren, beispielsweise Ovarialkarzimome und bestimmte gastrointestinale Karzinome. Intrarectale bzw. intragastrointestinale Verabreichung ist vorteilhaft für bestimmte gastrointestinale Tumoren und deren Lokalisation und Größenbestimmung. Intranodal kann in bestimmten Fällen dafür verwendet werden einzelne Lymphknoten direkt zu infiltrieren.

In einer bevorzugten Ausführungsform werden die erfindungsgemäßen oben beschriebenen radioaktiven Erkennungsmoleküle für in vivo Diagnostika mit einer Applikation von nicht markierten erfindungsgemäßen Core-1 spezifischen Erkennungsmolekülen kombiniert. Dies dient der Verbesserung des Hintergrundes. Bevorzugt werden dabei IgM abgeleitete Erkennungsmoleküle verwendet, da diese vor allem an Core-1 Antigen im Blut binden und damit den Hintergrund deutlich erniedrigen, während aufgrund der Größe der Moleküle ein Eindringen in Gewebe und Tumoren limitiert ist. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

Des weiteren bevorzugt werden erfindungsgemäße Erkennungsmoleküle, bevorzugt Immunglobuline, Multibodies oder Antikörperfragmente, weiter bevorzugt IgM, IgG und Multibodies, mit einem Fluoreszenzfarbstoff markiert und in vivo verabreicht. Bevorzugte Applikationsrouten sind hierbei intrarectal, intragastrointestinal, intraperitoneal, intravenös und in zuführende oder abführende Blutgefäße. Besonders bevorzugt ist die Lokalisation gastrointestinaler Karzinome, die durch eine Fluoreszenzendoskopie nach Applikation der fluoreszenzmarkierten Erkennungsmoleküle durchgeführt wird. Weiter bevorzugt wird ein erfindungsgemäßes Erkennungsmolekül mit mindestens einem Antikörper gegen ein weiteres Tumorantigen kombiniert, bevorzugt anti-MUCl Antikörper. Bevorzugt werden dabei unterschiedliche Fluoreszenzfarbstoffe verwendet, die eine Unterscheidung der Erkennungsmoleküle und Antikörper erlauben, womit eine prognostische Aussage mit einer Früherkennung und einer größeren Anzahl an Fällen kombiniert wird. Bevorzugte Fluoreszenzfarbstoffe sind solche mit geringer Hintergrundfluoreszenz, die dem Fachmann bekannt sind. Die hierfür verwendeten Verfahren und Technologien, einschließlich der bildgebenden Verfahren, beispielsweise der Fluoreszenz-Endoskopie, sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

Die Erfindung weist mehrere Vorteile auf: Die erfindungsgemäßen Core-1 spezifischen Erkennungsmoleküle erkennen Karzinomarten spezifisch, wodurch sie mit Vorteil bei vielen Tumorpatienten verschiedener Indikation zu einer Diagnose und/oder Therapie verwendet werden können. Darüber hinaus binden die Erkennungsmoleküle vorteilhafterweise praktisch nicht auf normalen Geweben. Dies ist gegenüber den bekannten Tumormarkern ein besonderer Vorteil und eine herausragende Eigenschaft der erfindungsgemäßen Erkennungsmoleküle. Vorteilhaft ist weiterhin, daß die Erkennungsmoleküle das Core-1 Antigen Träger-unabhängig erkennen. Ein besonderer Vorteil der erfindungsgemäßen Erkennungsmoleküle ist die hohe Spezifität für das Tumorgewebe. Dies ist insbesondere in der hohen Spezifität für definite Kohlenhydrat-Antigene begründet. Bei einer unspezifischen Erkennung anderer Kohlenhydratstrukturen würde sich nämlich die Gefahr der unspezifischen Erkennung von Nicht-Tumorgewebe erhöhen. Weiterhin weisen die erfindungsgemäßen Erkennungsmoleküle eine hohe Affinität auf. Hierdurch ist insbesondere die Möglichkeit gegeben, geringervalente Fragmente zu konstruieren, wie IgG und Multibodies. Die Möglichkeit dieser verschiedenen Formate ist vorteilhaft für die Entwicklung von Therapeutika. Die Core-1 und/oder Core-2 Struktur an der Zelloberfläche erhöht die Wahrscheinlichkeit der Ausbildung von Metastasen, beispielsweise von Lebermetastasen; durch die Blockierung der Core-1 und/oder Core-2 Struktur mit Erkennungsmolekülen wird die Metastasenbildung reduziert bzw. inhibiert.

Im folgenden soll die Erfindung anhand von Beispielen näher erläutert werden, ohne auf diese Beispiele beschränkt zu sein.

### Beispiele

### 1. Herstellung von Core-1 spezifischen Multibodies mit kurzen Linkern

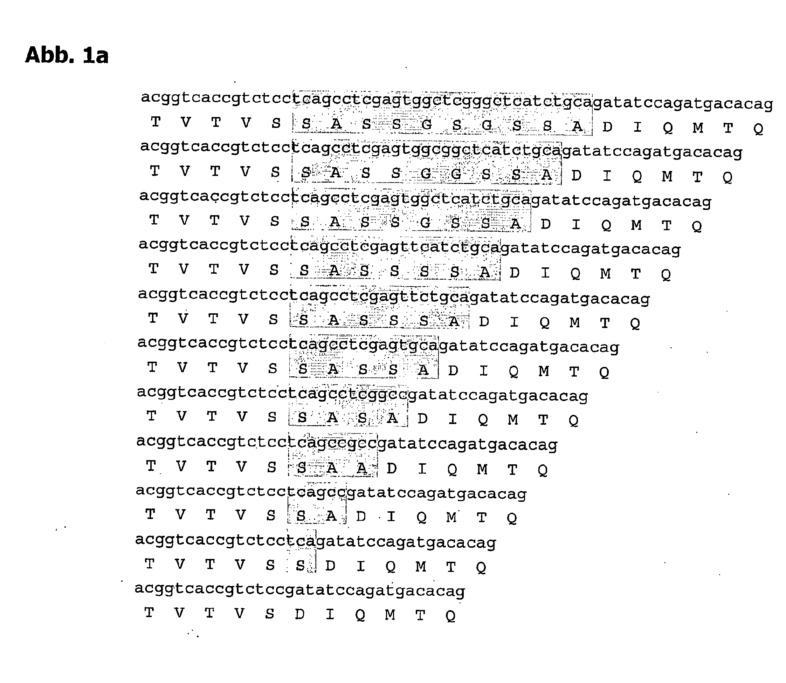

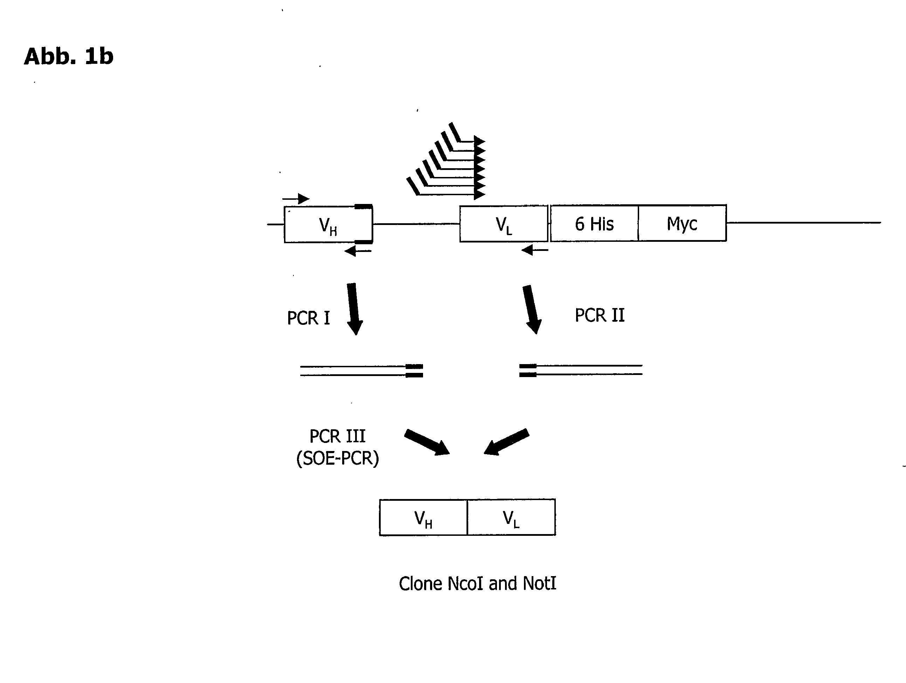

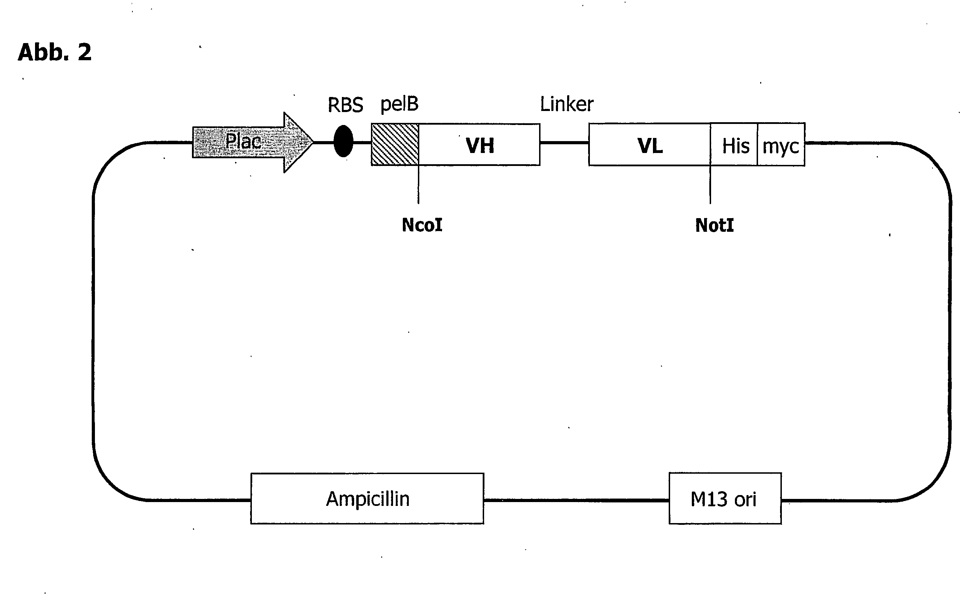

Multibodies mit den Sequenzen SEQ ID NO. 96 bis 106 wurden durch Verkürzung oder Deletion des Linkers zwischen der V_{H} und der V_{L} des single chain Antikörpers mit der Sequenz SEQ ID NO. 95 gebildet (Abb. 1a). Hierfür wurden die V_{H} und die V_{L} mit spezifischen Primern so amplifiziert, daß 22 Nukleotide am 3'-Ende der V_{H} und am 5'-Ende der V_{L} einen komplementären Bereich ausbilden (Abb. 1b, PCR I und PCR II), und anschließend die beiden PCR-Fragmente nach Aufreinigung in einer SOE-PCR miteinander verknüpft (Abb. 1b, PCR III). Zum Schluß wurde das PCR-Fragment über NcoI/NotI in einen prokaryontischen Expressionsvektor kloniert. Dieser Vektor enthält den lacZ Promotor, eine Ribosomenbindungsstelle (RBS), das M13 origin, die pelB Signalsequenz zur Sekretion ins Periplasma, ein Ampicillin-Resistenzgen und eine Klonierungskassette, um an das C-terminale Ende des scFv mit ein Hexa-Histidin-Tag zur effizienten Aufreinigung und ein c-myc-Tag zu koppeln (Abb. 2).

### 2. Bakterielle Expression und Aufreinigung der Core-1 spezifischen Multibodies

Die Antikörperfragmente aus Beispiel 1 wurden in *Escherichia coli* exprimiert und aufgereinigt. Hierfür wurde das entsprechende Plasmid durch Elektroporation in elektrokompetente *E.coli* transformiert und über Nacht in 2xTY Medium (10 g Hefeextrakt, 16 g Trypton, 5 g NaCl per L) mit 100 µg/mL Ampicillin kultiviert. Diese Kultur wurde 1:100 mit 2xTY Medium, dem 100 µg/ml Ampicillin und 0,5% Glukose zugesetzt wurde, verdünnt und bei 37°C inkubiert, bis eine OD_{600 nm} von ca. 0,6 erreicht war. Dann wurde der Kultur zur Induktion 1 mM IPTG zugesetzt und diese bei 25°C weitere 5 h inkubiert. Die Bakterien wurden durch Zentrifugation bei 4000xg für 20 min geerntet, das Zellpellet in TES Puffer (30 mM Tris-HC1, pH 8.0, 20% Saccharose, 1 mM EDTA) resuspendiert und 20 min auf Eis inkubiert. Anschließend wurden 5 mM MgSO₄ zugefügt und die Suspension für weitere 20 min auf Eis inkubiert. Durch Zentrifugation bei 4000 x g für 60 min wurde dann die Periplasmafraktion gewonnen und über Nacht bei 4°C gegen Bindungspuffer (50 mM Phosphatpuffer, pH 8.0, 300 mM NaCl, 10 mM Imidazol) dialysiert. Die in der Periplasmafraktion enthaltenen Antikörperfragmente wurden unter Verwendung des C-terminalen His-Tags durch Metallionen-Affinitäts-chromatographie (HiTrap Chelating HP, Amersham Pharmacia Biotech) aufgereinigt. Dafür wurde die dialysierte Fraktion auf die vorher mit Bindungspuffer äquilibrierte Säule gegeben und mit Waschpuffer (50 mM Phosphatpuffer, pH 8.0, 300 mM NaCl, 30 mM Imidazol) die nicht bindenden Proteine von der Säule gewaschen. Anschließend wurden die Antikörperfragmente mit Elutionspuffer (50 mM Phosphatpuffer, pH 8.0, 300 mM NaCl, 300 mM Imidazol) eluiert. Dieses Aufreinigungsprotokoll wurde für alle Core-1 spezifischen Antikörperfragmente mit Hexa-Histidin-Tag verwendet, beispielsweise den humanisierten single chain Antikörpern aus Beispiel 6.

### 3. Analyse der Core-1 spezifischen Multibodies im scFv Format mit unterschiedlicher Linkerlänge im ELISA

Multibodies mit den Aminosäuresequenzen SEQ ID NO. 95, 96, 97, 98, 99, 100, 101, 103, 104 und 105 wurden wie oben beschrieben in E.coli exprimiert und die Periplasmafraktionen gewonnen. Als Antigen für den ELISA Test wurde Asialoglykophorin (Sigma), ein Core-1 tragendes Glykoprotein, eingesetzt. Aus der Stammlösungen (1mg in 1ml Bi-dest. H₂O), die portioniert bei -20°C aufbewahrt werden, wurde eine Verdünnung von 5 µg/ml in PBS hergestellt. Davon wurden 50µl/well in eine Mikrotiterplatte (NUNCLON-TC Microwell 96 F) pipettiert und die Testplatte über Nacht bei 4°C inkubiert. Am nächsten Tag wurde die Testplatte mit PBS/0,2% Tween 3x gewaschen. Anschließend wurden mit 2% BSA in PBS unspezifische Bindungsstellen blockiert und 50 µl der jeweiligen mit PBS/1% BSA in verschiedenen Verdünnungsschritten verdünnten Fraktionen aufgetragen und 2 h bei 37°C inkubiert. Nach drei Waschschritten mit PBS/0,2% Tween wurden zum Nachweis der spezifisch gebundenen Antikörperkonstrukte als Zweit-Antikörper Peroxidase-gekoppelter anti-His Tag-Antikörper eingesetzt. Zum Nachweis des gebundenen sekundären Antikörpers erfolgte eine Farbreaktion mit TMB (3,3',5,5'-Tetramethylbenzidine). Nach 15 Minuten wurde die Reaktion durch Zugabe von 2,5N H₂SO₄ abgestoppt. Die Messung erfolgte mit einem Mikrotiterplattenphotometer mit Filter 450nm im Dual-mode gegen einen Referenzfilter 630nm. Das Ergebnis ist in Abbildung 3 dargestellt. Eine schrittweise Linkerverkürzung führt zu einer Erhöhung der Bindung an Asialoglykophorin. Die besten Bindungseigenschaften zeigen die Varianten mit der SEQ ID NO. 104 und 105. Diese multivalenten Konstrukte im Dia/Triabody Format sind bevorzugte Ausführungsformen der Erfindung und sind aufgrund verbesserter pharmakokinetischer Eigenschaften für die Tumortherapie von Vorteil.

### 4. Klonierung der Vektoren zur Expression chimärer Core-1 spezifischer IgG und IgM Antikörper

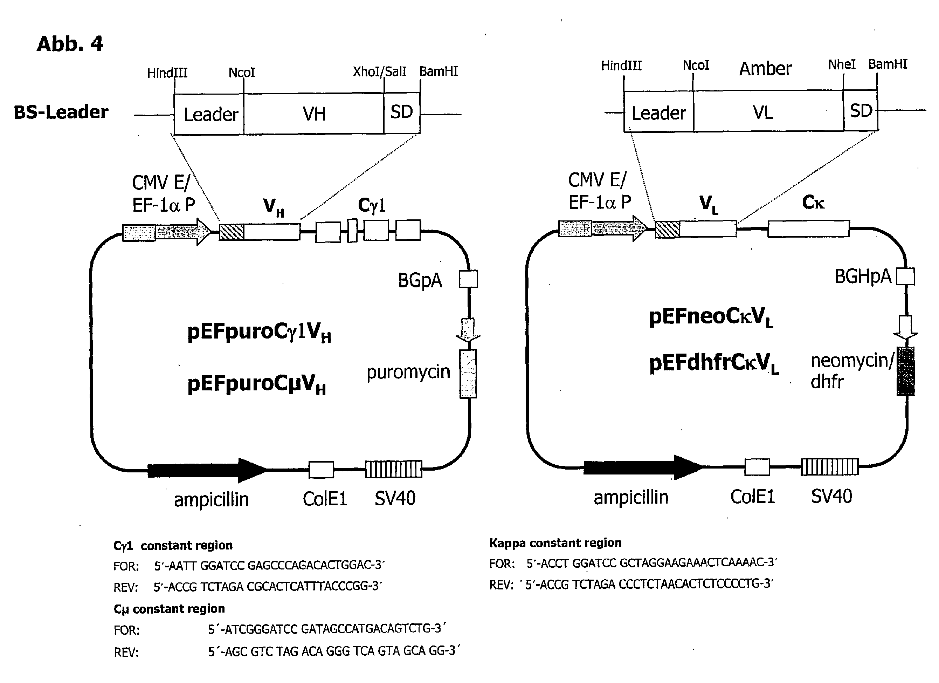

Das NcoI/XhoI DNA Fragment aus dem scFv Vektor, das für die V_{H} kodiert (Abb. 4), wurde in den NcoI/SalI geschnittenen BS-Leader Vektor kloniert. Der BS-Leader Vektor enthält eine Klonierungskassette zur Einführung der T-Zellrezeptor Signalpeptidsequenz an das 5'-Ende sowie einer Splice-Donor-Sequenz an das 3'-Ende der Sequenzen der variablen Domänen (Abb. 4). Die V_{L}-Sequenz des entsprechenden Antikörpers wurde mit spezifischen Primern zur Einführung der NcoI-Schnittstelle am 5'-Ende und der NheI-Schnittstelle am 3'-Ende in der PCR unter Verwendung der scFv Sequenz als Templat amplifiziert und nach NcoI/NheI Verdau in den gleich verdauten BS-Leader Vektor kloniert. Danach wurde jeweils das HindIII/BamHI Fragment aus dem BS-Leader Vektor in den entsprechenden eukaryontischen Expressionsvektor kloniert. Diese Vektoren (pEFpuroCγ1V_{H}, pEFpuroCµV_{H} und pEFneoCκV_{L}) enthalten den EF-1α-Promotor und den HCMV-Enhancer, das SV40-origin, das BGH-Polyadenylierungssignal, das Puromycin-Resistenzgen im Vektor für die schwere Kette und das Neomycin-Resistenzgen oder das Dehydrofolatreduktase-Gen im Vektor für die leichte Kette sowie die genomischen Sequenzen der humanen konstanten γ1 Region oder µ Region für die schwere Kette bzw. der humanen konstanten κ Region für die leichte Kette (Primer zur Amplifizierung aus genomischer humaner DNA und Vektorkarte siehe Abb. 4).

### 5. Eukaryontische Expression Core-1 spezifischer chimärer IgG und IgM Antikörper in CHO Zellen und deren Aufreinigung

Zur Expression der chimären Antikörper cIgG-Karo4 bestehend aus den Sequenzen SEQ ID NO. 111 und 113 und cIgM-Karo4 bestehend aus den Sequenzen SEQ ID NO. 112 und 113 wurden CHOdhfr- Zellen (ATCC-Nr. CRL-9096) mit einem Gemisch der Vektoren für die schwere und die leichte Kette (1:3) durch Elektroporation (10⁶ Zellen/ml, 500 V, 50 µs) cotransfiziert und in Selektionsmedium (CHO-S-SFM II Medium (Life Technologies), HT Supplement (Biochrom), 400 µg/ml G418, 5 µg/ml Puromycin) 2 Wochen kultiviert. Nach Einzelzellklonierung in einer 96-Loch-Platte wurden die Überstände im ELISA (Asialoglykophorin als Antigen, anti human Fcyl- POD gekoppelt bzw. anti human Fc5µ- POD gekoppelt (Dianova) als Sekundärantikörper) getestet und der Klon mit der höchsten Antikörperproduktionsrate selektioniert (ca. 0,5 µg/10⁶ Zellen/24 h).
Zur Antikörperproduktion wurden die stabil transfizierten, den chimären IgG bzw. IgM sekretierenden CHO Zellen in Spinnerflaschen in CHO-S-SFM II Medium, ergänzt durch HT Supplement, kultiviert, bis eine Zelldichte von ca. 1 x 10⁶ Zellen/ml erreicht war. Nach Abtrennung der Zellen vom Zellkulturüberstand durch Zentrifugation (400xg, 15 min) wurde der chimäre Antikörper unter Verwendung einer Protein A - Säule (HiTrap rProtein A FF, Amersham Pharmacia Biotech) für den chimären IgG bzw. einer anti human Fc5µ- Antikörper Affinitätssäule aufgereinigt. Die durch pH-Sprung eluierte gereinigte Antikörperfraktion wurde unter Verwendung von Centriprep-Zentrifugenröhrchen (cut off 50 kDa, Millipore) in PBS umgepuffert und aufkonzentriert.

### 6. Angleichung der Sequenz der Core-1 spezifischen Antikörpersequenzen an humane Keimbahnsequenzen

Für die Angleichung der Core-1 bindenden Antikörpersequenzen an humane Sequenzen wurde in der Datenbank humaner Keimbahnsequenzen nach homologen Sequenzen gesucht und unter Verwendung der humanen Consensus-Sequenzen und den Erkenntnissen der kanonischen Struktur humaner Antikörper wurden humanisierte Core-1-bindende Sequenzen entwickelt. Für die variable schwere Kette diente die humane Keimbahnsequenz VH1-46 als Vorlage, für die variable leichte Kette die Sequenz A18.

Die humanisierten V_{H} bzw. V_{L} Sequenzen SEQ ID NO. 56 bis 79 bzw. 85 bis 94 wurden mit Hilfe der gene assembly PCR (singleoverlap extension PCR) hergestellt. Die PCR Reaktion erfolgte nach folgendem Schema: erste Denaturierung 94°C für 2min, dann 30 Zyklen Denaturierung bei 94°C für 45 sec, Annealing bei 55°C für 45 sec und Elongation bei 73°C für 1,5 min und am Ende einen Elongationsschritt bei 73°C für 7 min.

Die so hergestellten V_{H} und V_{L} Ketten wurden mit den Enzymen NcoI und XhoI bzw. NotI und XhoI geschnitten und zur Seqeunzierung in einen Klonierungsvektor (pLitmus 28 bzw. pBluescript KS) kloniert. Die richtigen V_{H} und V_{L} Ketten wurden anschließend erneut amplifiziert, um am 3'-Ende der V_{H} und am 5'-Ende der V_{L} eine BbsI Schnittstelle einzufügen, um darüber die V_{H} und die V_{L} mit nur einem Alanin als Linker zu verknüpfen. Nach der Ligation wurden die kompletten scFv (die Ligationsprodukte) unter Verwendung der flankierenden Primer amplifiziert und in einen bakteriellen Expressionsvektor kloniert.

### 7. Spezifitätsanalyse der Core-1 spezifischen Erkennungsmoleküle im ELISA

Als Antigene wurden verschiedene Kohlenhydrat-PAA-Konjugate (Synthesome) und Glykoproteine verwendet: Asialoglykophorin (AGP), Glykophorin (GP) und Asialofetuine (Sigma); die PAA (Poly[N-(2-hydroxyethyl) Acrylamid)-Konjugate: Galß1-3GalNAcα1-OC₃H₆NH-PAA und Galβ1-3GalNAcα1-*p*-OC₆H₄NH-PAA als Core-1 (alpha-Anomer) Konjugate mit unterschiedlichen Linkerlängen, Galβ1-3GalNAcβ1-OC₃H₆NH-PAA als beta-Anomer des Core-1, Galα1-3GalNAcα1-OC₃H₆NH-PAA und Galα1-3GalNAcβ1-OC₃H₆NH-PAA als weitere Stereoanomere des Core-1, die Core-2 Struktur Galβ1-3(GlcNAcβ1-6)GalNAcα1-OC₃H₆NH-PAA und die Derivate GalNAcal-OC₃H₆NH-PAA, Neu5Acα2-3Galβ1-3GalNAcα1-OC₃H₆NH-PAA, Galβ1-3(Neu5Acα2-6)GalNAcα1-OC₃H₆NH-PAA, GlcNAcβl-2Galβ1-3GalNAcα1-OC₃H₆NH-PAA, GlcNAca1-3Galβ1-3GalNAcα1-OC₃H₆NH-PAA, GalNAcα1-3Galβ1-OC₃H₆NH-PAA und 3'-O-Su-Galβ1-3GalNAcα1-OC₃H₆NH-PAA.

Aus den jeweiligen Stammlösungen (1mg in 1ml Bi-dest.H₂O), die portioniert bei -20°C aufbewahrt werden, wurde eine Verdünnung von 5 µg/ml in PBS hergestellt. Davon wurden 50µl/well in eine Mikrotiterplatte (NUNCLON-TC Microwell 96 F) pipettiert und die Testplatte 1 h bei 37°C und über Nacht bei 4°C inkubiert. Am nächsten Tag wurde die Testplatte mit PBS/0,2% Tween 3x gewaschen. Anschließend wurden mit 2% BSA in PBS unspezifische Bindungsstellen blockiert und 50 µl des ersten Antikörpers aufgetragen (chimärer IgG bzw. IgM: gereinigt 0,1 µg/ml in PBS/0,1% BSA oder unverdünnter Kulturüberstand produzierender CHOdhfr- Zellen; Multibodies: 10 µg/ml in PBS/0,1% BSA). Nach drei Waschschritten mit PBS/0,2% Tween wurden zum Nachweis der spezifisch gebundenen Antikörperkonstrukte die entsprechenden Zweit-Antikörper, Peroxidase-gekoppelt, eingesetzt (ein anti-Maus bzw. anti-human Fcγ1 bzw. µ -Antikörper für die ganzen Antikörper, ein anti-His Tag-Antikörper für Multibodies). Zum Nachweis des gebundenen sekundären Antikörpers erfolgte eine Farbreaktion mit TMB (3,3',5,5'- Tetramethylbenzidine). Nach 15 Minuten wurde die Reaktion durch Zugabe von 2,5N H₂SO₄ abgestoppt. Die Messung erfolgte mit einem Mikrotiterplattenphotometer mit Filter 450nm im Dual-mode gegen einen Referenzfilter 630nm.

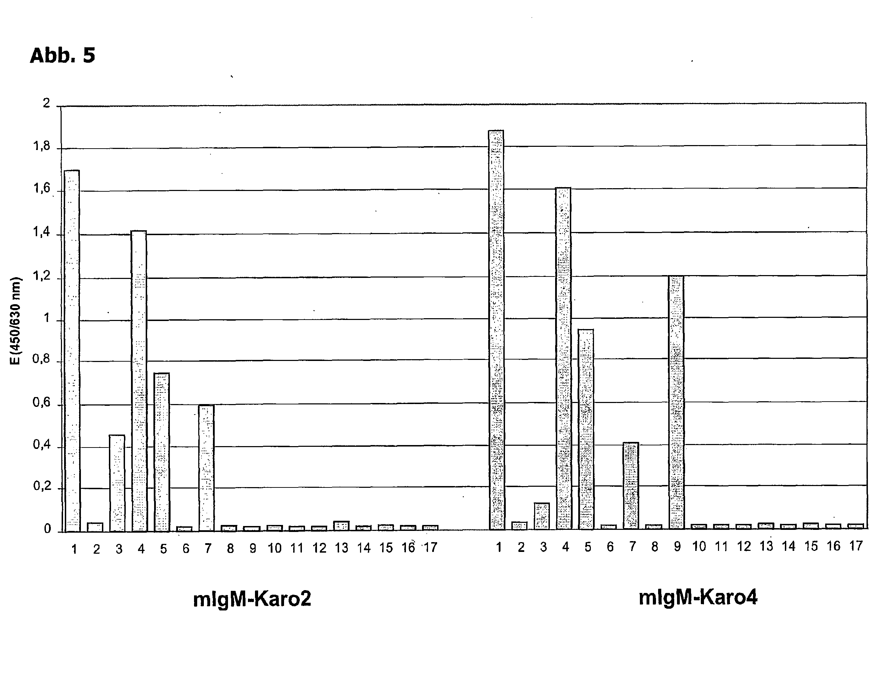

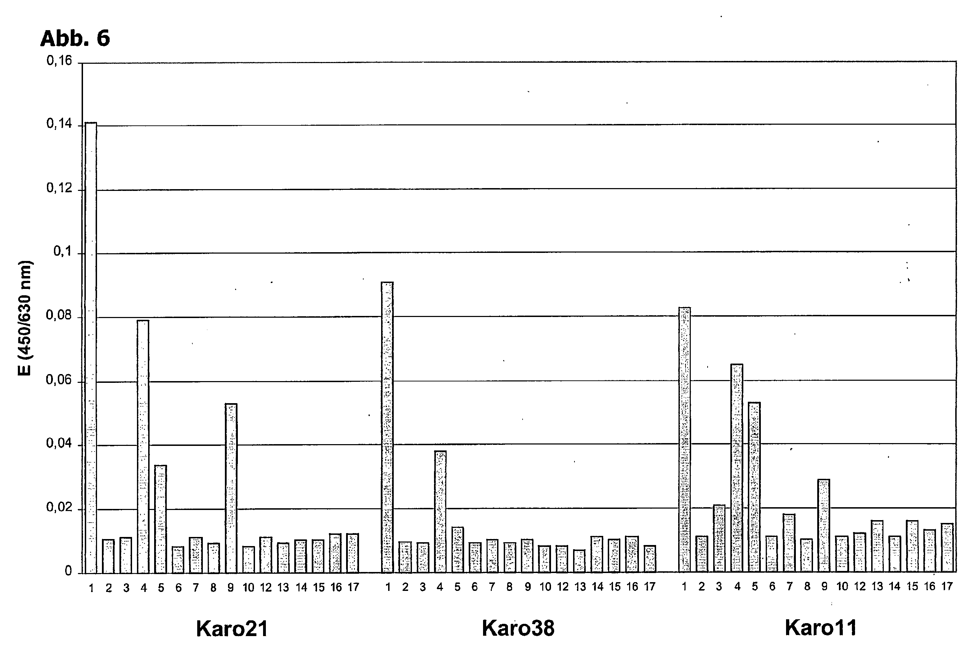

Repräsentative Ergebnisse sind in den Abbildungen 5 und 6 dargestellt. In Abbildung 5 sind zwei Erkennungsmoleküle mit variierenden Loop-Sequenzen im IgM-Format verglichen. Die Antikörperkonstrukte mIgM-Karo2 (SEQ ID NO. 107 und SEQ ID NO. 109) und mIgM-Karo4 (SEQ ID NO. 108 und SEQ ID NO. 110) binden hochspezifisch an das Antigen Core-1, bevorzugt an das alpha-Anomer Galβ1-3GalNAcα und schwächer an das beta-Anomer Galβ1-3GalNAcβ. Die erfindungsgemäßen Erkennungsmoleküle können auch nur das alpha-Anomer Galβ1-3GalNAcα oder beide Anomere Galβ1-3GalNAcα und Galβ1-3GalNAcβ in gleicher Weise binden. Zusätzlich bindet mIgM-Karo4 die Core-2 Struktur Galβ1-3(GlcNAcβ1-6)GalNAcα. Alle anderen getesteten Kohlenhydratstrukturen, auch strukturell stark verwandte Strukturen werden durch die hier beanspruchten Bindungsproteine nicht erkannt. Als Core-1 tragendes Glykoprotein zeigt AGP ein starkes Signal mit beiden Varianten, wobei das ebenfalls Core-1 tragende Glykoprotein Asialofetuin deutlich stärker mit der Karo2-Variante reagiert, was sehr wahrscheinlich mit der unterschiedlichen Core-1 Dichte in beiden Proteinen zusammenhängt. Abbildung 6 zeigt das Spezifitätsmuster der beispielhaft ausgewählten humanisierten Erkennungsmoleküle Karo11 (SEQ ID NO. 56 und SEQ ID NO. 90), Karo21 (SEQ ID NO. 59 und SEQ ID NO. 90) und Karo38 (SEQ ID NO. 69 und SEQ ID NO. 90) mit variierenden Gerüstsequenzen im scFv-Format mit einer Aminosäure als Linker. Auch hier zeigt sich das gleiche Spezifitätsmuster, wie in der Definition der Core-1 spezifischen Bindung im Sinne der Erfindung beschrieben (siehe oben).

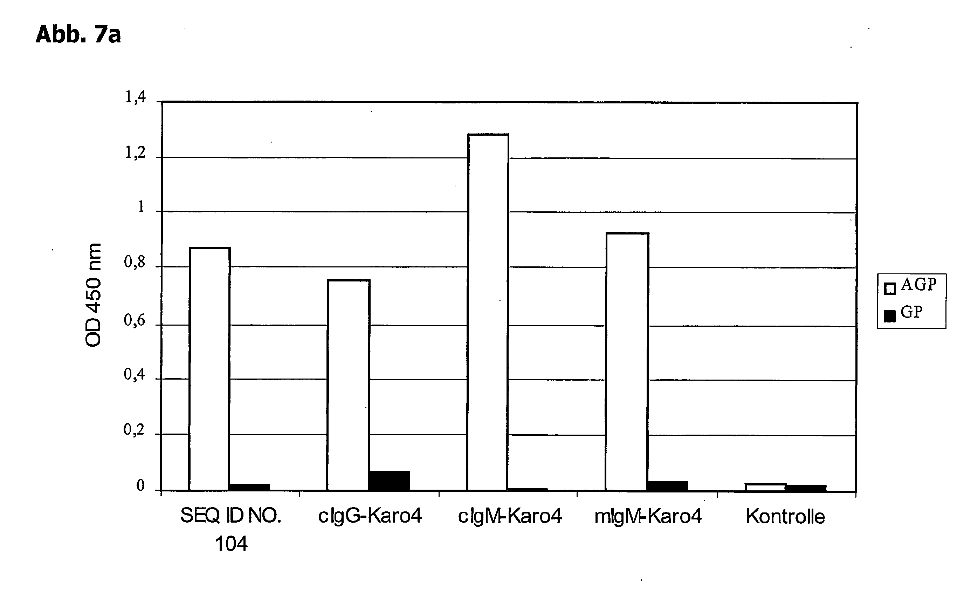

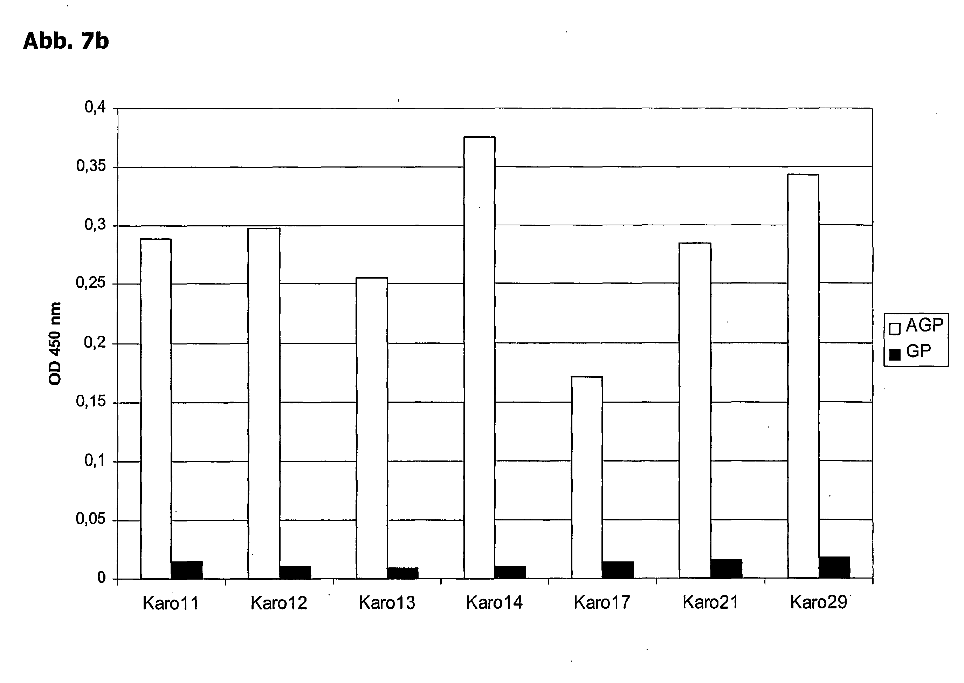

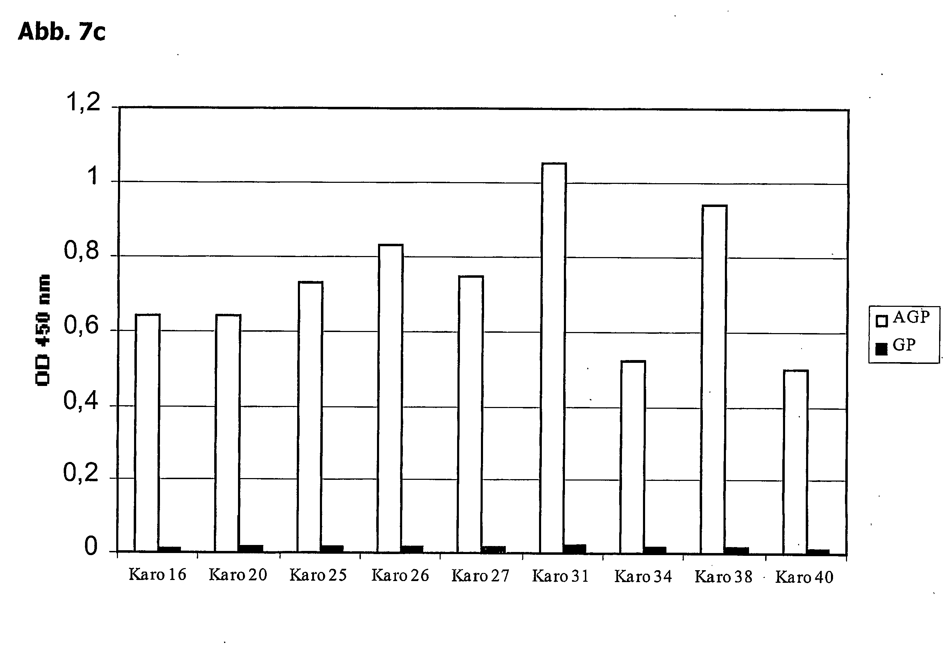

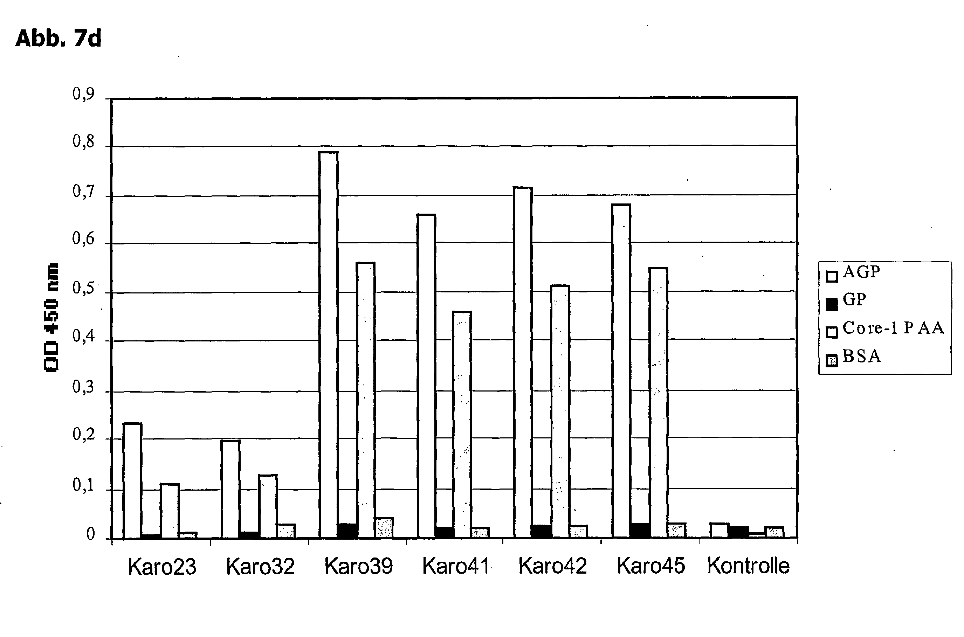

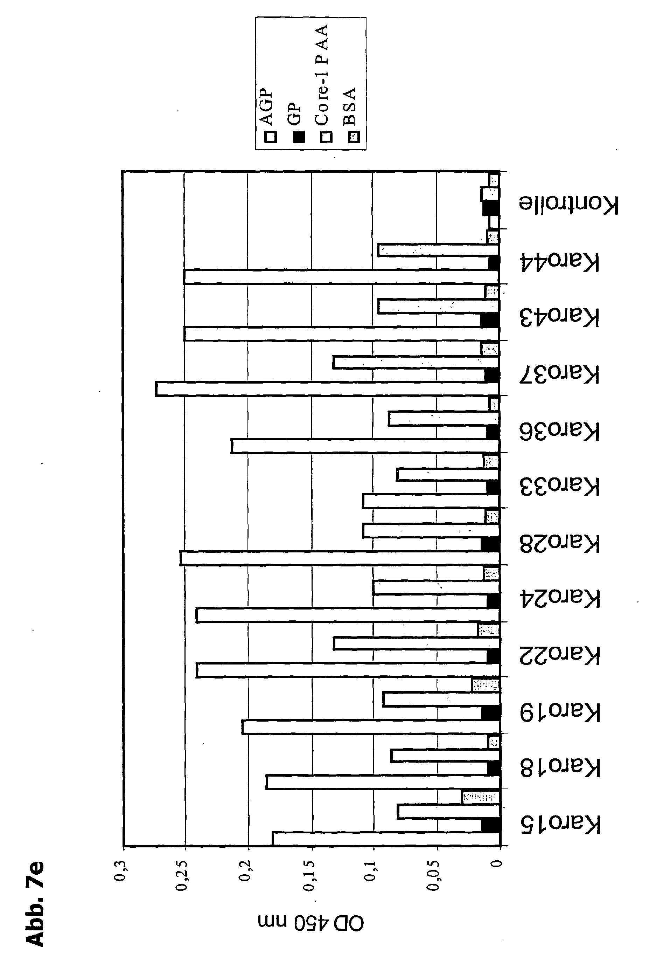

Die spezifische Bindung der verschiedenen bevorzugten Formate und Kombinationen im ELISA, beispielhaft an AGP, GP und/oder Galβ1-3GalNAcα1-OC₃H₆NH-PAA, ist in den Abbildungen 7 a bis e dargestellt.

### 8. Immunhistologische und immunzytologische Färbungen

Für die immunhistologischen Färbungen wurden Gefrierschnitte entsprechender Gewebeproben luftgetrocknet und mit 10% Formaldehyd in PBS 15 min fixiert. Zur Reduktion der endogenen Peroxidase-Aktivität wurden die Schnitte mit 3% Wasserstoffperoxid in PBS behandelt und nach Blockierung unspezifischer Bindungsstellen mit präabsorbiertem Kaninchenserum an Neuraminidase-behandelten Erythrozyten mit einem Core-1 spezifischen Primärantikörper inkubiert. Anschließend wurden die Präparate mit einem entsprechenden Sekundärantikörper (anti-Maus bzw. anti-human IgG oder IgM, POD gekoppelt) inkubiert. Die Farbreaktion erfolgte unter Verwendung des Peroxidase-Substrats Diaminobenzidin und die Gegenfärbung mit Hämatoxylin.

Das beispielhafte erfindungsgemäße Erkennungsmolekül mIgM-Karo4 reagiert nur mit sehr wenigen Strukturen im Normalgewebe. Diese befinden sich aber in für einen Antikörper nicht zugänglichen Bereichen (Tabelle 3).

**Tabelle 3: Reaktion von humanem Normalgewebe mit dem Core-1 spezifischen Antikörper mIgM-Karo4**

| **Gewebetyp** | **Reaktivität** |
|---|---|
| Epidermis - Basalmembran | negativ |
| Magen | |
| Foveola Epithelium | negativ |
| Fundusdrüsen | negativ |
| Korpusdrüsen | negativ |
| Colon Mucosa | negativ |
| Milz | |
| Trabeculae lienis | negativ |
| Retikularzellen | negativ |
| Lymphozyten | negativ |
| Endothelium | negativ |
| Prostata | negativ |
| Leber | |
| Hepatozyten | negativ |
| Kupffer-Zellen | negativ |
| Gallenwege | negativ |
| Lymphknoten | |
| Lymphozyten | negativ |
| Retikularzellen | negativ |
| Gallenblase | negativ |
| Nebenniere | |
| Nebennierenrinde | negativ |
| Nebennierenmark | negativ |
| Blase | negativ |
| Herz | negativ |
| Bauchspeicheldrüse | |
| Drüsengänge | positiv |
| Azini | negativ |
| Langerhans'sche Inseln | negativ |

Die beanspruchten Erkennungsmoleküle reagieren positiv mit einer Vielzahl von Karzinomen. Die Daten in Tabelle 4 zeigen, dass die Core-1 spezifischen Erkennungsmoleküle einen großen Prozentsatz an Tumorpatienten einer Indikation erkennen, der von Indikation zu Indikation unterschiedlich ist.

**Tabelle 4: Reaktion von humanem Tumorgewebe mit dem Core-1 spezifischen Antikörper mIgM-Karo4**

| **Gewebetyp** | **Reaktivität** |
|---|---|
| Colonkarzinom | |
| primäres Karzinom | 31/52 |
| Lebermetastasen | 20/22 |
| Lungenkarzinom | |
| Großzelliges | 3/8 |
| Bronchoalveolar | 1/1 |
| Adenokarzinom | 6/6 |
| Blasenkarzinom | 5/9 |
| Magenkarzinom | |
| Intestinaler Typ | 8/8 |
| Diffuseer Typ | 3/3 |
| Prostatakarzinom | 9/9 |
| Mammakarzinom | |
| Intraductal/ductal | 8/10 |
| Schwach differenziert | 2/5 |
| Muzinöses | 1/1 |
| Schilddrüsenkarzinom | 0/10 |
| Nierenkarzinom | |
| Klarzelliges | 4/9 |
| Transitionalzelliges | 2/5 |
| Zervikalkarzinom | 1/2 |
| Ovarialkarzinom | |
| Adenokarzinom | 2/2 |
| Endometrioide | 2/2 |
| Teratom | 2/2 |
| Glioblastom | 0/3 |

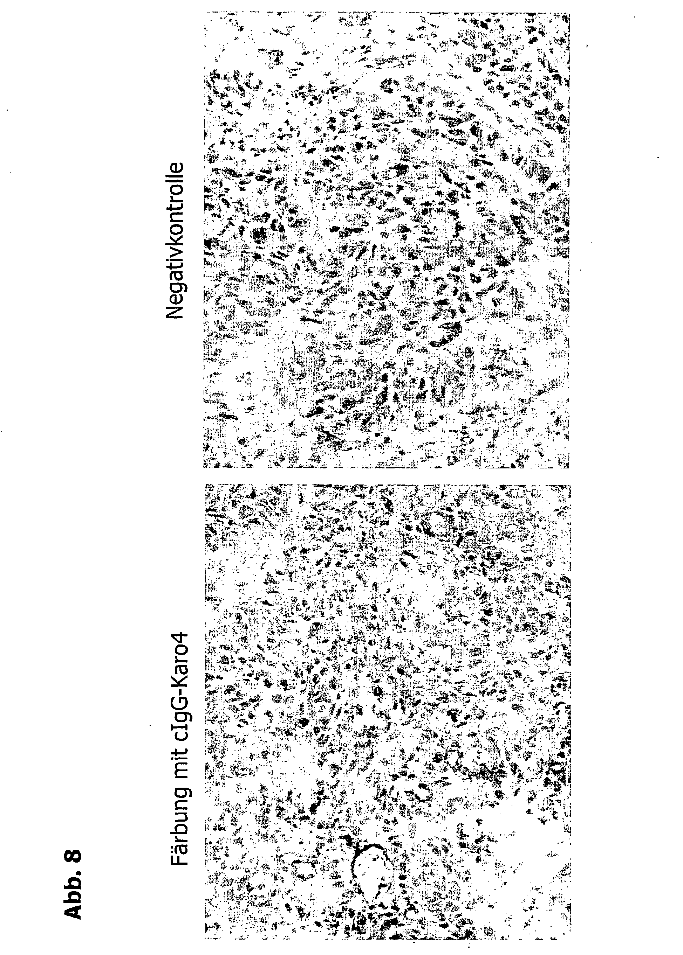

Für die Entwicklung eines Maus-Tumormodells wurden verschiedene Xenotransplantate untersucht. Bei den Xenotransplantaten handelt es sich um humanes Kolonkarzinomgewebe, das auf Nacktmäusen mehrfach passagiert wurde. Abbildung 8 zeigt beispielhaft eine immunhistochemische Färbung eines Xenotransplantat-Präparats mit dem Core-1 spezifischen Antikörper cIgG-Karo4.

Für die immunzytologischen Färbungen wurde die Immunfluoreszenz eingesetzt. Dafür wurden die entsprechenden Zellen auf Objektträger angetrocknet und mit 5% Formaldehyd 10 min fixiert. Nach Blockierung unspezifischer Bindungsstellen mit BSA (1% in PBS) wurden die Zellen mit dem Primärantikörper inkubiert. Anschließend wurde 3 mal mit PBS gewaschen und mit dem entsprechenden fluoreszenz-markierten Sekundärantikörper (anti-Maus oder anti-human IgG bzw. IgM für ganze Antikörper; anti-Myc-Tag oder anti-His-Tag Antikörper für die single chain Antikörperfragmente) inkubiert. Nach mehrmaligem Waschen mit PBS wurden die Zellen in Mowiol eingebettet.

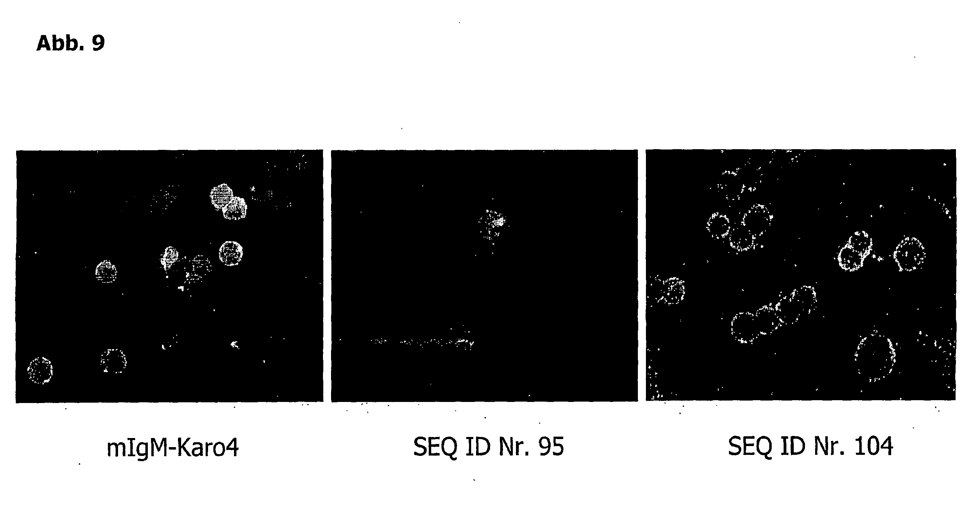

Es wurden verschiedene Zelllinien mit Core-1 spezifischen Erkennungsmolekülen in der Immunfluoreszenz getestet. Eine Reihe von Tumorzelllinien und auch einigen Leukämie-Zelllinien reagieren positiv (Tab. 5 und Abb. 9).

**Tabelle 5: Reaktivität verschiedener Zelllinien mit den Core-1 spezifischen Antikörpern mIgM-Karo1 bzw. mIgM-Karo4**

| **Zelllinien** | **Reaktivität** |
|---|---|
| KG-1 | positiv |
| ZR-75-1 | positiv |
| T47D | (positiv) wenige Zellen |
| U266 | negativ |
| LN78 | positiv |
| HT29 | positiv |
| HCT116 | negativ |
| HepG2 | negativ |
| K562 | negativ |
| NM-D4 | positiv |

Abbildung 9 zeigt beispielhaft eine Fluoreszenzmarkierung von KG-1 Zellen, einer akuten myeloischen Leukämie-Zelllinie, mit verschiedenen Antikörperkonstrukten, einem murinen IgM und zwei scFv-Antikörpern mit unterschiedlicher Linkerlänge (SEQ ID NO. 95 mit 18 Aminosäuren und SEQ ID NO. 104 mit einer Aminosäure als Linker). Alle drei Konstrukte zeigen eine spezifische Färbung der Tumorzelllinie, wobei das monovalente Antikörperfragment SEQ ID NO. 95 das schwächste Signal zeigt.

### 9. Chelatisierung und radioaktive Markierung von Antikörpern und Antikörperfragmenten

Durch Konjugation wurde an den Antikörper cIgG-Karo4 bzw. den Multibody mit der Sequenz SEQ ID NO. 104 ein Chelator kovalent gebunden, der die Bindung eines Radiometalls ermöglicht. Als Chelatoren kamen die kommerziellen Produkte der Firma Macrocyclics (Dallas, USA), p-isothiocyanatobenzyldiethylenetriaminepentaacetic acid (p-SCN-Bz-DTPA) und p-isothiocyanatobenzyl -1,4,7,10-tetraazacyclododecane-1,4,7,10-tetraacetic acid (p-SCN-Bz-DOTA) zum Einsatz. Beide Chelatoren eignen sich zur Kopplung an Antikörper zu deren Radioaktivmarkierung [Brechbiel et al., 1986; Kozak et al., 1989; Stimmel et al., 1995].

Die Konjugation erfolgte durch Reaktion der Isothiocyanatgruppe des Chelators mit einer freien s-Aminogruppe der Aminosäure Lysin am Antikörper. Es entsteht eine kovalente N-C-Bindung zwischen Chelator und Antikörper.

Der gereinigte Antikörper bzw. das gereinigte Antikörperfragment muß zunächst in Kopplungspuffer pH 8,7 umgepuffert werden. Hierzu wurde eine Ultrafiltration in einer Filtrationshülse (Centriprep YM50 (Amicon)) durchgeführt. Dies erfolgte durch mehrmalige Verdünnung mit 10-fachem Volumen und Filtration durch eine Membran mit definierter Porengröße durch Zentrifugation. Dadurch wurde PBS durch den alkalischen Kopplungspuffer (0,05 M Natrium-Carbonat, 0,15 M Natriumchlorid, pH 8,7) ersetzt.

Die Chelatisierung wurde mit den bifunktionellen Chelatoren p-SCN-Bz-DTPA- bzw. p-SCN-Bz-DOTA durchgeführt. Zur Chelatisierungsreaktion wurden Protein (1-10 mg/ml) in Kopplungspuffer und eine Lösung des Chelators von 1 mg/ml in 2% DMSO/Wasser so gemischt, dass ein molarer Überschuß des Chelators gewährleistet war. Es folgte eine Inkubation der Mischung von 1h bei 37°C. Anschließend wurde nicht gebundener Chelator durch Ultrafiltration im gleichen Gefäß (Centriprep YM50 (Amicon)) abgetrennt und wie oben beschrieben in den zur Radioaktivmarkierung notwendigen Beladungspuffer auf pH 4,2 umgepuffert (0,15 M Natriumacetat, 0,15 M Natriumclorid, pH 4,2). Die Proteinkonzentration während und nach diesem Schritt wurde wieder auf 1-10mg/ml mit Hilfe einer UV-Messung bei 280nm eingestellt.

Es waren Bedingungen für die Chelatisierungsreaktion zu finden, die eine Radiomarkierung des Antikörpers erlauben, ohne dessen Bioaktivität wesentlich zu mindern.

Der chelatisierte Antikörper wurde mit einem Radiometall beladen, wodurch der Radio-Antikörper erzeugt wurde. Zur Beladung wurden die Isotope ¹¹¹Indium und ⁹⁰Yttrium verwendet. Beide haben chemisch und physikochemisch vergleichbare Eigenschaften und werden durch den Chelator als dreiwertige Ionen gebunden (¹¹¹In³⁺, ⁹⁰Y³⁺). Der mit ¹¹¹Indium markierte Antikörper ist ein γ-Strahler und wird in der Klinik zur individuellen Dosisfindung für den Patienten verwendet, während ⁹⁰Yttrium ein β-Strahler ist, der therapeutisch zum Einsatz kommt. Die Halbwertzeiten betragen für ¹¹¹In 67 Stunden und für ⁹⁰Y 64 Stunden.

Zur Beladung wurde ¹¹¹Indiumchlorid der Firma NEN (Perkin Elmer, Belgien) verwendet. Die Lieferung des Radiometalls erfolgt in salzsaurer Lösung. Diese ¹¹¹InCl₃ Lösung wurde zunächst kurzzeitig auf eine HCl-Konzentration von 1M gebracht. Anschließend wurde mit 0,05M HCl auf eine spezifische Aktivität von 80-320mCi/ml verdünnt und davon ein Aliquot zum Einbau in den chelatisierten Antikörper verwendet, wobei das zugegebene Volumen HCl-saurer ¹¹¹InCl₃-Lösung gleich dem Volumen der vorgelegten Antikörperlösung im Kopplungspuffer pH 4,2 sein sollte, um die pH-Stabilität zu gewährleisten. Inkubationsdauer war 1h bei 37°C mit gelegentlichem vorsichtigem Mischen.

Im Anschluß daran wurde der Filtereinsatz wieder in die Filtrationshülse eingesetzt und wie oben beschrieben in Phosphatpuffer pH 7,2 mit physiologischem Gehalt an Kochsalz umgepuffert. Dabei erfolgte eine Trennung von hochmolekularem radioaktiv markiertem Antikörper und ungebundenem ¹¹¹InCl₃. Die Quantifizierung des Einbaus von ¹¹¹In in den chelatisierten Antikörper erfolgte dünnschichtchromatographisch. Die Einbaurate des Radiometalls lag bei 70-99% der eingesetzten Radioaktivität.

### 10. Nachweis des Core-1 positiven, sekretorischen MUC1 im Sandwich-ELISA

Core-1 positives, sekretorisches MUC1 kann im Sandwich-ELISA nachgewiesen werden. Dabei dient ein MUC1 spezifischer Antikörper als Fänger-Antikörper von MUC1 und ein Core-1 spezifischer Antikörper zum Nachweis des Core-1 Antigens. Ein dritter Enzym- oder Fluoreszenz-gekoppelter Antikörper muss zur Detektion des Zweitantikörpers eingesetzt werden.

Als Beispiel wurden die Überstände zweier Tumorzelllinien analysiert (K562 und T47D). Die Ergebnisse sind in Tabelle 6 dargestellt. 10⁵ Zellen pro ml Zellkulturmedium wurden ausgesät, 4 Tage ohne Mediumwechsel kultiviert, anschließend ein Aliquot abgenommen und der Zellkulturüberstand durch Zentrifugation vom Zellpellet getrennt. 50 µl dieser Überstände wurden unverdünnt im ELISA eingesetzt. Der anti-MUC1-anti-Core-1-Sandwich-ELISA wurde durchgeführt, indem die Mikrotiterplatte mit dem Fänger-Antikörper (1 µg/ml) in PBS über Nacht bei 4 °C beschichtet wurde. - Es wurden drei verschiedenen Konzentrationen des Antikörpers für die Beschichtung getestet (1 µg/ml, 2 µg/ml und 4 µg/ml). Die Beschichtung mit 1 µg/ml erwies sich im Sandwich ELISA als am empfindlichsten. - Anschließend wurden die beschichteten Platten zweimal mit PBS gewaschen und 1,5 h in 5% BSA, 0,05% Tween 20 in PBS bei Raumtemperatur blockiert. Der Blockierungspuffer wurde entfernt, die Platten erneut einmal mit 0,1% Tween 20 in PBS (Waschpuffer) gewaschen, die Proben dazugegeben und 1,5 h bei Raumtemperatur inkubiert. Als Negativkontrollen wurden das Zellkulturmedium oder 2% BSA in Waschpuffer (Verdünnungspuffer für Zweitantikörper) verwendet. Eine Positivkontrolle stand nicht zur Verfügung. Nach dreimaligem Waschen erfolgte die Neuraminidase Behandlung in den dafür vorgesehenen Wells. Zu diesem Zweck wurde die Neuraminidase-Lösung (DADE Behring, Deutschland) in Immidazolpuffer (0,68 g Immidazol, 0,19 g CaCl₂ und 0,4 g NaCl in 100 ml H₂O, pH 6,8) 1:5 verdünnt und 50 µl/well 30 min bei 37 °C inkubiert. Als Kontrolle wurde der Imidazolpuffer ohne Neuraminidase-Lösung in einem entsprechenden Well inkubiert. Anschließend wurden die Wells dreimal gewaschen und der mIgM-Karo4 Antikörper zum Nachweis des Core-1 Antigens in einer 1:500 Verdünnung in 2% BSA in Waschpuffer dazugegeben und eine weitere Stunde bei Raumtemperatur inkubiert. Nach erneutem dreimaligen Waschen folgte die Zugabe eines Peroxidasegekoppelten anti Maus IgM(µ) Antikörpers (Dianova) 1:5000 verdünnt in 2% BSA in Waschpuffer und eine Inkubation von 1 h bei Raumtemperatur. Abschließend wurden die Platten zweimal in Waschpuffer und einmal in PBS gewaschen. Die Färbereaktion erfolgte in 25 mM Zitronensäure, Phosphatpuffer pH 5,0 mit 0,04% H₂O₂ und 0,4 mg/ml o-Phenylendiamin (Sigma) im Dunkeln bei Raumtemperatur. Die Farbreaktion wurde durch Zugabe von 2,5 N Schwefelsäure (Endkonzentration 0,07 N) gestoppt und im ELISA-Reader bei 492 nm mit einem Referenzfilter von 620 nm vermessen.

**Tabelle 6: Analyse von Core-1 positivem MUC1 in Kulturüberständen zweier Zelllinien mit und ohne Neuraminidase Behandlung im Sandwich ELISA**

| Zelllinie | Signal | |
|---|---|---|
| | - NeuAcdase | +NeuAcdase |
| K562 | - | + |
| T47D | + | +++ |

### 11. Effektive Bindung von radioaktiv-markierten Core-1 spezifischen Erkennungsmolekülen an Tumorzellen

Die Core-1 positive Tumorzelllinie NM-D4 [DSMZ deposit NO. DSM ACC2605] (vergleiche Tabelle 5) wurden zur Testung der Bindungsfähigkeit der radiomarkierten Erkennungsmoleküle an Core-1 positive Tumorzellen herangezogen. Dabei wurde jeweils in Dublettbestimmungen eine bestimmte Anzahl Zellen in einem 1,5 ml Gefäß vorgelegt und mit steigenden Mengen Antikörper inkubiert. Nach dem Waschen wurde anhand der Zählrate bestimmt, wieviel Antikörper gebunden hat.
Pro Ansatz werden 2x10⁶ Zellen benötigt. Nach Vorinkubation der Zellen für eine Stunde auf Eis wurde die benötigte Menge Zellen in Reaktionsgefäße vorgelegt, zentrifugiert (5min 1000xg, 25°C) und der Überstand abgenommen. Dann wurde mit PBS/0,1% Tween20/1% BSA aufgefüllt bis zum Volumen 200µl abzüglich der noch zuzugebenen Menge Erkennungsmolekül. Anschließend wurde das entsprechende ¹¹¹In-markierte Erkennungsmolekül (siehe Beispiel 9) zum Endvolumen 200 µl zugegeben (ca. 0,5 bis 20 µg, je nach Erkennungsmolekül) und die Ansätze für eine Stunde bei 4-8°C inkubiert. Nach Zentrifugation (4min, 1000xg, 25°C) wurde der Überstand abgenommen und das Zellpellet in 400µl PBST/1%BSA vorsichtig resuspendiert. Nach nochmaligem Waschen wurde das Zellpellet im Gefäß am gamma-Counter gemessen. Von den Ausgangslösungen definierter Konzentration wurden die spezifischen Zählraten bestimmt, der Wert in cpm/ng wurde der Relativierung der Messwerte von gebundenem Antikörper zugrunde gelegt. Die freie Bindung ergibt sich aus der Differenz aus Gesamtmenge und gebundener Menge Antikörper. Diese Werte wurden als Verhältnis gebunden/nichtgebunden gegen die Menge gebunden in ein Diagramm aufgetragen und im linearen Bereich der Kurve die Steigung ermittelt und der Abzissen-Schnittpunkt bestimmt (Scatchard Analyse). Der Abzissen-Schnittpunkt bezeichnet die Anzahl der Bindungsplätze/Zelle. Aus der Steigung der Geraden ergibt sich die Assoziationskonstante Kass in [M⁻¹].

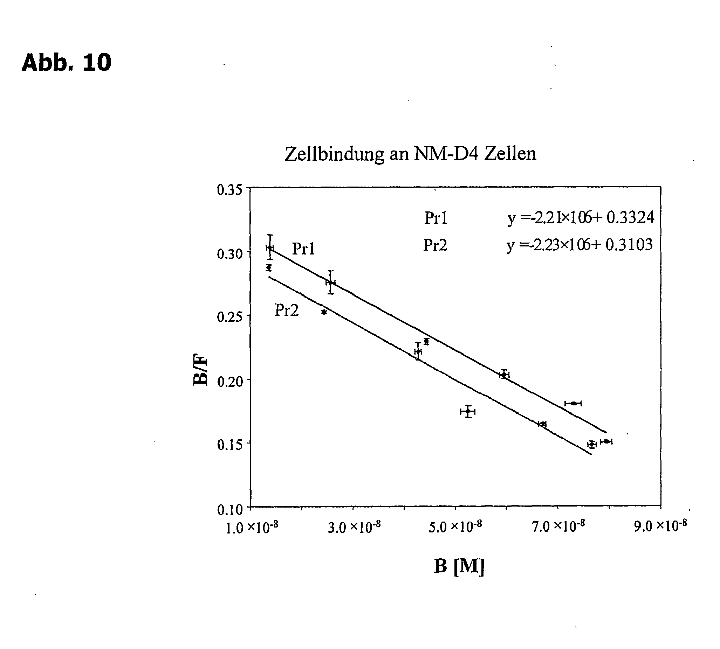

In Abbildung 10 ist beispielhaft die Scatchard-Analyse der Bindung des radioaktiv-markierten Erkennungsmoleküls im scFv-Format mit der Sequenz SEQ ID NO. 104 mit einer Aminosäure als Linker an NM-D4 Zellen dargestellt (zwei verschiedene Präparationen).
In Tabelle 7 sind die Assoziationskonstanten und die Anzahl der Zellbindungsplätze verschiedener Core-1 spezifischer Multibodies an NM-D4 Zellen zusammengefaßt.

**Tabelle 7: Zellbindungstest und Scatchard-Analyse mit ¹¹¹Inmarkierten Erkennungsmolekülen an NM-D4 Zellen.**

| **Antikörper** | **Kass [M-1]** | **Anzahl Bindungsplätze/Zelle** |
|---|---|---|
| SEQ ID NO. 105 | 1,1x10⁷ | 4,8x10⁶ |
| SEQ ID NO. 104 | 2,1x10⁶ | 8,1x10⁶ |
| SEQ ID NO. 103 | 1,2x10⁶ | 9,2x10⁶ |

### 12. Anreicherung der radioaktiv-markierten Core-1 spezifischen Erkennungsmoleküle an Core-1 positiven Tumoren in einem in vivo Tumormodell

Als Tumormodell wurden ZR-75-1 Zellen subkutan in Nacktmäuse (Ncr: nu/nu, weiblich) injiziert. Nach ca. 3-4 Wochen ist der Tumor unter der Haut tastbar. Den Tumor-tragenden Mäusen (pro Zeitpunkt n=4) wurden jeweils 5 µg ¹¹¹In-markierter Multibody (SEQ ID NO. 104 bzw. SEQ ID NO. 105) in 200 µl i.v. in die Schwanzvene verabreicht. Nach 24h wurden die Mäuse getötet und die Verteilung der Radioaktivität im Tumor, im Serum und den Organen ermittelt. Tabelle 8 zeigt die spezifische hohe Anreicherung der Multibodies im Tumor (in %ID/g Tumor bezogen auf die injizierte Dosis und das Tumorgeweicht) im Vergleich zum Serum und den Organen.

**Tabelle 8: Biodistribütion ¹¹¹In-markierter Erkennungsmoleküle in Tumor-tragenden Mäusen.**

| | **SEQ ID NO. 104** | **SEQ ID NO. 105** |
|---|---|---|
| Serum (%ID/ml) | 1,4 ± 0,16 | 1,0 ± 0,24 |
| Tumor (%ID/g) | 10,8 ± 2,88 | 8,1 ± 1,45 |
| Leber (%ID/g) | 3,7 ± 0,15 | 5,3 ± 0,92 |
| Lunge (%ID/g) | 1,7 ± 0,11 | 1,9 ± 0,19 |
| Herz (%ID/g) | 1,5 ± 0,06 | 1,9 ± 0,19 |
| Milz (%ID/g) | 5,4 ± 0,75 | 6,7 ± 1,07 |
| Gehirn (%ID/g) | 0,1 ± 0,01 | 0,1 ± 0,00 |
| Knochenmark (%ID/g) | 1,0 ± 0,16 | 1,7 ± 0,90 |

### 13. Therapiestudie zur Reduktion von Core-1 positiver Tumoren mit radioaktiv-markierten Core-1 spezifischen Erkennungsmolekülen in einem in vivo Tumormodell

Die Therapiestudien wurden unter. Verwendung des gleichen etablierten ZR-75-1-Tumormodells durchgeführt, wie für die Biodistributionstudien beschrieben (siehe Beispiel 12). Die chelatisierten Erkennungsmoleküle (siehe Beispiel 9) wurden hierfür mit ⁹⁰Yttrium (ein β-Strahler zur Zerstörung der Tumorzellen) beladen (pH 4,5, 37°C, 30 min; vergleiche Einbau von ¹¹¹Indium) und in der Dünnschichtchromatographie die Stabilität kontrolliert. Den Tumor-tragenden Mäusen (ca. drei Wochen nach der subkutanen Injektion der ZR-75-1 Zellen) wurden 200 µl i.v. in die Schwanzvene verabreicht. Die Injektionslösung enthielt den ⁹⁰Y-markierten Multibody (bis maximal 100 µCi pro Dosis) in Ca/Mg-PBS mit 0,2 bis 4% fötales Kälberserum zum Schutz vor Radiolyse. Kontrollgruppen erhielten die gleiche Injektion ohne radioaktiv-markiertem Erkennungsmolekül. Körpergewicht und Tumorgröße wurden zweimal wöchentlich gemessen und verglichen. Das relative Tumorwachstum wurde unter Berücksichtigung der jeweiligen Tumorgröße zu Beginn der Behandlung bestimmt. Eine zweite Injektion wurde drei Wochen nach der ersten Behandlung injiziert. Durch geeignete Behandlung konnte das Tumorwachstum signifikant zur Kontrollgruppe reduziert werden.

### Legende zu den Abbildungen

- **Abb. 1a:**: **Sequenzen der Linker in den verschiedenen Multibody single chain Antikörperfragmenten.**
- **Abb. 1b:**: **Klonierungsschema zur Herstellung von single chain Antikörperfragmenten mit unterschiedlicher Linkerlänge.**
- **Abb. 2:**: **Vektor zur Klonierung und bakteriellen Expression von single chain Antikörperfragmenten.**
- **Abb. 3:**: **Analyse von Multibodies im scFv Format mit unterschiedlicher Linkerlänge im ELISA.**

Multibodies mit den Aminosäuresequenzen SEQ ID NO. 95, 96, 97, 98, 99, 100, 101, 103, 104 und 105 wurden wie oben beschrieben in E.coli exprimiert und die Periplasmafraktionen gewonnen. Als Antigen für den ELISA Test wurde Asialoglykophorin, ein Core-1 tragendes Glykoprotein, eingesetzt. Eine schrittweise Linkerverkürzung führt zu einer Erhöhung der Bindung an Asialoglykophorin. Die besten Bindungseigenschaften zeigen die Varianten mit der SEQ ID NO. 104 und 105. Diese multivalenten Konstrukte im Dia/Triabody Format sind bevorzugte Ausführungsformen der Erfindung.
- **Abb. 4:**: **Vektorsystem zur Klonierung und eukaryontischen Expression von chimären Antikörpern im IgG1 oder IgM-Format.**
- **Abb. 5 und 6:**: **Spezifitätsanalyse im ELISA.**

Als Antigene wurden verschiedene Glykoproteine und Kohlenhydrat-PAA-Konjugate verwendet. Asialoglykophorin **[1]**; Glykophorin **[2];** Asialofetuine **[3];** Galβ1-3GalNAcα1-OC₃H₆NH-PAA **[4] ;** Galβ1-3GalNAcα1-*p*-OC₆H₄NH-PAA **[5];** Galα1-3GalNAcα1-OC₃H₆NH-PAA **[6];** Galβ1-3GalNAcβ1-OC₃H₆NH-PAA **[7];** Galα1-3GalNAcβ1-OC₃H₆NH-PAA **[8];** Galβ1-3(GlcNAcβ1-6) GalNAcα1-OC₃H₆NH-PAA **[9];** GalNAcα1-OC₃H₆NH-PAA **[10];** Neu5Acα2-3Galβ1-3GalNAcα1-OC₃H₆NH-PAA **[11];** Galβ1-3(Neu5Acα2-6)GalNAcα1-OC₃H₆NH-PAA **[12];** GlcNAcβ1-2Galβ1-3GalNAcα1-OC₃H₆NH-PAA **[13];** GlcNAca1-3Galβ1-3GalNAcα1-OC₃H₆NH-PAA [14]; GalNAcα1-3Galβ1-OC₃H₆NH-PAA **[15];** und 3'-O-Su-Galβ1-3GalNAcα1-OC₃H₆NH-PAA **[16]**. Als Kontrolle wurde BSA **[17]** verwendet. In **Abbildung 5** wurden zwei Antikörper im IgM-Format mit unterschiedlicher CDR-Sequenz-Zusammensetzung eingesetzt. **Abbildung 6** zeigt das Spezifitätsmuster von drei humanisierten Erkennungsmolekülen im scFv-Format mit variierenden Gerüstsequnezen.
- **Abb. 7:**: **Spezifische Bindung verschiedener bevorzugter Formate und Kombinationen erfindungsgemäßer Erkennungsmoleküle im ELISA, beispielhaft an den** Antigenen AGP, GP und/oder Core-1-PAA (Galβ1-3GalNAcα1-OC₃H₆NH-PAA).
- **Abb. 8:**: **Immunhistochemische Färbung von Xenotransplantat-Präparaten.**

Humanes Colonkarzinomgewebe wurde auf Nacktmäuse transplantiert und nach Erreichen einer bestimmten Größe passagiert. Das Tumorgewebe wurde eingebettet und geschnitten und für immunhistochemische Färbungen eingesetzt. In a) wurde das Gewebes mit cIgG-Karo4 als Primärantikörper und einem anti-human Fcγ Antikörper - POD gekoppelt als Sekundärantikörper markiert. Die braune Färbung kennzeichnet die Core-1-positiven Strukturen.
- **Abb. 9:**: **Fluoreszenzmarkierung von Zellen der Tumorzelllinie KG-1 mit verschiedenen Core-1 spezifischen Erkennungsmolekülen.**
- **Abb. 10:**: **Scatchard-Diagramm zur Analyse der Zellbindung radioaktiv markierter Core-1 spezifischer Erkennungsmoleküle.** Beispielhaft sind hier die Bindungsdaten des Multibodies SEQ ID NO. 104 mit einer Linkerlänge von einer Aminosäure dargestellt (Pr1 und Pr2 entsprechen zwei verschiedenen Präparationen). B: an die Zellen gebundener Anteil [M], F: freie Bindung als Differenz aus Gesamtmenge und gebundener Menge Antikörper [M]. Oben ist die entsprechende Geradengleichung angegeben, wobei der Geradenanstieg die Assoziationskonstante wiedergibt.

### Sequenzen

| CDR Sequenzen | |
|---|---|
| SEQ ID NO. 1 | NYWLG |
| | |
| SEQ ID NO. 2 | DIYPGGGYTNYNEKFKG |
| SEQ ID NO. 3 | DIYPGGSYTNYNEKFKG |
| | |
| SEQ ID NO. 4 | YDAAGPWFAY |
| SEQ ID NO. 5 | YDAAGPGFAY |
| SEQ ID NO. 6 | YDNHYFDY |
| | |
| SEQ ID NO. 7 | RSSQSIVHSNGNTYLE |
| SEQ ID NO. 8 | RSSQSLLHSNGNTYLH |
| SEQ ID NO. 9 | KSSQSLLHSDGKTYLY |
| | |
| SEQ ID NO. 10 | KVSNRFS |
| SEQ ID NO. 11 | EVSSRFS |
| | |
| SEQ ID NO. 12 | FQGSHVPYT |
| SEQ ID NO. 13 | SQSTHVPYT |

| CDR Sequenzen (canonical structure variants) | |
|---|---|
| SEQ ID NO. 14 | NYWIG |
| SEQ ID NO. 15 | NYWMG |
| SEQ ID NO. 16 | NYWWG |
| SEQ ID NO. 17 | NYWVG |
| | |
| SEQ ID NO. 18 | DIYPGGDYTNYNEKFKG |
| SEQ ID NO. 19 | DIYPGGNYTNYNEKFKG |
| SEQ ID NO. 20 | DIYTGGGYTNYNEKFKG |
| SEQ ID NO. 21 | DIYTGGDYTNYNEKFKG |
| SEQ ID NO. 22 | DIYTGGNYTNYNEKFKG |
| SEQ ID NO. 23 | DIYTGGSYTNYNEKFKG |
| SEQ ID NO. 24 | DIYAGGGYTNYNEKFKG |
| SEQ ID NO. 25 | DIYAGGDYTNYNEKFKG |
| SEQ ID NO. 26 | DIYAGGNYTNYNEKFKG |
| SEQ ID NO. 27 | DIYAGGSYTNYNEKFKG |
| | |
| SEQ ID NO. 28 | RPSQSIVHSNGNTYLE |
| SEQ ID NO. 29 | RSSQSLVHSNGNTYLE |
| SEQ ID NO. 30 | RSSQSIVHSNGNTYFE |
| SEQ ID NO. 31 | RPSQSLVHSNGNTYLE |
| SEQ ID NO. 32 | RPSQSIVHSNGNTYFE |
| SEQ ID NO. 33 | RSSQSLVHSNGNTYFE |
| SEQ ID NO. 34 | RPSQSLLHSNGNTYLH |
| SEQ ID NO. 35 | RSSQSILHSNGNTYLH |
| SEQ ID NO. 36 | RSSQSLLHSNGNTYFH |
| SEQ ID NO. 37 | RPSQSILHSNGNTYLH |
| SEQ ID NO. 38 | RPSQSLLHSNGNTYFH |
| SEQ ID NO. 39 | RSSQSILHSNGNTYFH |
| SEQ ID NO. 40 | KPSQSLLHSDGKTYLY |
| SEQ ID NO. 41 | KSSQSILHSDGKTYLY |
| SEQ ID NO. 42 | KSSQSLLHSDGKTYFY |
| SEQ ID NO. 43 | KPSQSILHSDGKTYLY |
| SEQ ID NO. 44 | KPSQSLLHSDGKTYFY |
| SEQ ID NO. 45 | KSSQSILHSDGKTYFY |

### variable schwere Ketten VH

### variable leichte Ketten

### VH/VL Paarungen

| Mausantikörper | |
|---|---|
| Karo1 | SEQ ID NO. 46 |
| | SEQ ID NO. 80 |
| | |
| Karo2 | SEQ ID NO. 47 |
| | SEQ ID NO. 81 |
| | |
| Karo3 | SEQ ID NO. 48 |
| | SEQ ID NO. 80 |
| | |
| Karo4 | SEQ ID NO. 50 |
| | SEQ ID NO. 80 |
| | |
| Karo5 | SEQ ID NO. 53 |
| | SEQ ID NO. 82 |
| | |
| Karo6 | SEQ ID NO. 52 |
| | SEQ ID NO. 83 |
| | |
| Karo7 | SEQ ID NO. 55 |
| | SEQ ID NO. 83 |
| | |
| Karo8 | SEQ ID NO. 54 |
| | SEQ ID NO. 80 |
| | |
| Karo9 | SEQ ID NO. 51 |
| | SEQ ID NO. 83 |
| | |
| Karo10 | SEQ ID NO. 49 |
| | SEQ ID NO. 80 |

| humanisierte Sequenzen | |
|---|---|
| Karo11 | SEQ ID NO. 56 |
| | SEQ ID NO. 90 |
| | |
| Karo12 | SEQ ID NO. 57 |
| | SEQ ID NO. 90 |
| | |
| Karo13 | SEQ ID NO. 57 |
| | SEQ ID NO. 86 |
| | |
| Karo14 | SEQ ID NO. 58 |
| | SEQ ID NO. 87 |
| | |
| Karo15 | SEQ ID NO. 56 |
| | SEQ ID NO. 91 |
| | |
| Karo16 | SEQ ID NO. 59 |
| | SEQ ID NO. 91 |
| | |
| Karo17 | SEQ ID NO. 60 |
| | SEQ ID NO. 87 |
| | |
| Karo18 | SEQ ID NO. 61 |
| | SEQ ID NO. 90 |
| | |
| Karo19 | SEQ ID NO. 56 |
| | SEQ ID NO. 88 |
| | |
| Karo20 | SEQ ID NO. 56 |
| | SEQ ID NO. 85 |
| | |
| Karo21 | SEQ ID NO. 59 |
| | SEQ ID NO. 90 |
| Karo22 | SEQ ID NO. 62 |
| | SEQ ID NO. 90 |
| | |
| Karo23 | SEQ ID NO. 59 |
| | SEQ ID NO. 86 |
| | |
| Karo24 | SEQ ID NO. 74 |
| | SEQ ID NO. 92 |
| | |
| Karo25 | SEQ ID NO. 63 |
| | SEQ ID NO. 87 |
| | |
| Karo26 | SEQ ID NO. 74 |
| | SEQ ID NO. 87 |
| | |
| Karo27 | SEQ ID NO. 74 |
| | SEQ ID NO. 89 |
| | |
| Karo28 | SEQ ID NO. 74 |
| | SEQ ID NO. 85 |
| | |
| Karo29 | SEQ ID NO. 64 |
| | SEQ ID NO. 86 |
| | |
| Karo30 | SEQ ID NO. 74 |
| | SEQ ID NO. 86 |
| | |
| Karo31 | SEQ ID NO. 63 |
| | SEQ ID NO. 86 |
| | |
| Karo32 | SEQ ID NO. 65 |
| | SEQ ID NO. 85 |
| Karo33 | SEQ ID NO. 65 |
| | SEQ ID NO. 86 |
| | |
| Karo34 | SEQ ID NO. 66 |
| | SEQ ID NO. 85 |
| | |
| Karo35 | SEQ ID NO. 67 |
| | SEQ ID NO. 87 |
| | |
| Karo36 | SEQ ID NO. 68 |
| | SEQ ID NO. 86 |
| | |
| Karo37 | SEQ ID NO. 72 |
| | SEQ ID NO. 88 |
| | |
| Karo38 | SEQ ID NO. 69 |
| | SEQ ID NO. 90 |
| | |
| Karo39 | SEQ ID NO. 70 |
| | SEQ ID NO. 90 |
| | |
| Karo40 | SEQ ID NO. 69 |
| | SEQ ID NO. 92 |
| | |
| Karo41 | SEQ ID NO. 73 |
| | SEQ ID NO. 86 |
| | |
| Karo42 | SEQ ID NO. 69 |
| | SEQ ID NO. 89 |
| | |
| Karo43 | SEQ ID NO. 71 |
| | SEQ ID NO. 92 |
| | |
| Karo44 | SEQ ID NO. 56 |
| | SEQ ID NO. 86 |
| | |
| Karo45 | SEQ ID NO. 65 |
| | SEQ ID NO. 92 |

### verschiedene single chain Fv Formate

### Murine Antikörper

| | |
|---|---|
| mIgM-Karo2 | SEQ ID NO. 109 |
| | SEQ ID NO. 107 |
| | |
| mIgM-Karo4 | SEQ ID NO. 110 |
| | SEQ ID NO. 108 |

### Chimäre Antikörper (Maus/Menscn)

### chimäre Antikörper

| | |
|---|---|
| cIgG-Karo4 | SEQ ID NO. 111 |
| | SEQ ID NO. 113 |
| | |

| | |
|---|---|
| cIgM-Karo4 | SEQ ID NO. 112 |
| | SEQ ID NO. 113 |

## Patentansprüche

1. Erkennungsmolekül, **dadurch gekennzeichnet, dass** es eine Aminosäuresequenz umfasst, die
(i) die Aminosäuresequenz SEQ ID NO. 1 und
(ii) die Aminosäuresequenz SEQ ID NO. 2 oder 3 und
(iii) die Aminosäuresequenz SEQ ID NO. 4, 5 oder 6 enthält und
das Antigen Core-1 spezifisch bindet.

2. Erkennungsmolekül nach Anspruch 1, **dadurch gekennzeichnet, dass** es weiterhin eine Aminosäuresequenz umfasst, die
(i) die Aminosäuresequenz SEQ ID NO. 7 oder 8 oder 9 und
(ii) die Aminosäuresequenz SEQ ID NO. 10 oder 11 und
(iii) die Aminosäuresequenz SEQ ID NO. 12 oder 13 enthält und
das Antigen Core-1 spezifisch bindet.

3. Erkennungsmolekül nach Anspruch 1 oder 2, **dadurch gekennzeichnet, dass** die Antikörpergerüst-sequenzen
a) FRH1, FRH2, FRH3 und FRH4 für die variable schwere Kette VH folgende Aminosäuresequenzen sind, wobei die Aminosäureposition der Nummerierung nach Kabat entspricht:
| | | |
|---|---|---|
| für FRH1 an Position | 1 | Q oder E |
| | 2 | V |
| | 3 | Q, K oder T |
| | 4 | L |
| | 5 | K oder V |
| | 6 | E oder Q |
| | 7 | S |
| | 8 | G |
| | 9 | A |
| | 10 | E |
| | 11 | L oder V |
| | 12 | V oder K |
| | 13 | R oder K |
| | 14 | P |
| | 15 | G |
| | 16 | T oder A |
| | 17 | S |
| | 18 | V |
| | 19 | K |
| | 20 | I oder V |
| | 21 | S oder P |
| | 22 | C |
| | 23 | K |
| | 24 | A, V, S oder T |
| | 25 | s |
| | 26 | G |
| | 27 | Y, F, S oder D |
| | 28 | T |
| | 29 | F, L oder I |
| | 30 | T |
| für FRH2 an Position | 36 | W |
| | 37 | V |
| | 38 | K oder R |
| | 39 | Q |
| | 40 | R oder A |
| | 41 | P |
| | 42 | G |
| | 43 | H oder Q |
| | 44 | G |
| | 45 | L |
| | 46 | E |
| | 47 | W oder R |
| | 48 | 1 oder M |
| | 49 | G |
| für FRH3 an Position | 66 | K oder R |
| | 67 | A oder V |
| | 68 | T |
| | 69 | L oder M |
| | 70 | T |
| | 71 | A, L oder T |
| | 72 | D |
| | 73 | T |
| | 74 | S |
| | 75 | S oder T |
| | 76 | S |
| | 77 | T |
| | 78 | A |
| | 79 | Y |
| | 80 | M |
| | 81 | Q oder E |
| | 82 | L |
| | 82a | S |
| | 82b | S oder R |
| | 82c | L |
| | 83 | T oder R |
| | 84 | S |
| | 85 | E |
| | 86 | D |
| | 87 | S oder T |
| | 88 | A |
| | 89 | V |
| | 90 | Y |
| | 91 | F oder Y |
| | 92 | C |
| | 93 | A |
| | 94 | Y, K oder R |
| für FRH4 an Position | 103 | W |
| | 104 | G |
| | 105 | Q |
| | 106 | G |
| | 107 | T |
| | 108 | T, S oder L |
| | 109 | V oder L |
| | 110 | T |
| | 111 | V |
| | 112 | S |
| | 113 | S oder A |
b) FRL1, FRL2, FRL3 und FRL4 für die variable leichte Kette VL folgende Aminosäuresequenzen sind, wobei die Aminosäureposition der Nummerierung nach Kabat entspricht:
| | | |
|---|---|---|
| für FRL1 an Position | 1 | D |
| | 2 | I, V oder L |
| | 3 | Q oder L |
| | 4 | M |
| | 5 | T |
| | 6 | Q |
| | 7 | T oder S |
| | 8 | P |
| | 9 | L |
| | 10 | S |
| | 11 | L |
| | 12 | P |
| | 13 | V |
| | 14 | S oder T |
| | 15 | L oder P |
| | 16 | G |
| | 17 | D oder E |
| | 18 | Q oder P |
| | 19 | A |
| | 20 | S |
| | 21 | I |
| | 22 | S |
| | 23 | C |
| für FRL2 an Position | 35 | W |
| | 36 | Y |
| | 37 | L |
| | 38 | Q |
| | 39 | K |
| | 40 | P |
| | 41 | G |
| | 42 | Q |
| | 43 | S |
| | 44 | P |
| | 45 | K oder Q |
| | 46 | L |
| | 47 | L |
| | 48 | I oder V |
| | 49 | Y |
| für FRL3 an Position | 57 | G |
| | 58 | V |
| | 59 | P |
| | 60 | D |
| | 61 | R |
| | 62 | F |
| | 63 | S |
| | 64 | G |
| | 65 | S |
| | 66 | G |
| | 67 | S |
| | 68 | G |
| | 69 | T |
| | 70 | D |
| | 71 | F |
| | 72 | T |
| | 73 | L |
| | 74 | K |
| | 75 | I |
| | 76 | S |
| | 77 | R |
| | 78 | V |
| | 79 | E |
| | 80 | A |
| | 81 | E |
| | 82 | D |
| | 83 | L oder V |
| | 84 | G |
| | 85 | V |
| | 86 | Y |
| | 87 | Y |
| | 88 | C |
| für FRL4 an Position | 98 | F |
| | 99 | G |
| | 100 | G oder Q |
| | 101 | G |
| | 102 | T |
| | 103 | K |
| | 104 | L |
| | 105 | E |
| | 106 | I oder L |
| | 106a | K |
| | 107 | R |
| | 108 | A. |

4. Erkennungsmolekül nach dem vorhergehenden Anspruch, **dadurch gekennzeichnet, dass** das Erkennungsmolekül eine Kombination der Sequenzen SEQ ID NO. 46 und 80, oder SEQ ID NO. 47 und 81, oder SEQ ID NO. 48 und 80, oder SEQ ID NO. 50 und 80, oder SEQ ID NO. 53 und 82, oder SEQ ID NO. 52 und 83, oder SEQ ID NO. 55 und 83, oder SEQ ID NO. 54 und 80, oder SEQ ID NO. 51 und 83, oder SEQ ID NO. 49 und 80, oder SEQ ID NO. 56 und 90, oder SEQ ID NO. 57 und 90, oder SEQ ID NO. 57 und 86, oder SEQ ID NO. 58 und 87, oder SEQ ID NO. 56 und 91, oder SEQ ID NO. 59 und 91, oder SEQ ID NO. 60 und 87, oder SEQ ID NO. 61 und 90, oder SEQ ID NO. 56 und 88, oder SEQ ID NO. 56 und 85, oder SEQ ID NO. 59 und 90, oder SEQ ID NO. 62 und 90, oder SEQ ID NO. 59 und 86, oder SEQ ID NO. 74 und 92, oder SEQ ID NO. 63 und 87, oder SEQ ID NO. 74 und 87, oder SEQ ID NO. 74 und 89, oder SEQ ID NO. 74 und 85, oder SEQ ID NO. 64 und 86, oder SEQ ID NO. 74 und 86, oder SEQ ID NO. 63 und 86, oder SEQ ID NO. 65 und 85, oder SEQ ID NO. 65 und 86, oder SEQ ID NO. 66 und 85, oder SEQ ID NO. 67 und 87, oder SEQ ID NO. 68 und 86, oder SEQ ID NO. 72 und 88, oder SEQ ID NO. 69 und 90, oder SEQ ID NO. 70 und 90, oder SEQ ID NO. 69 und 92, oder SEQ ID NO. 73 und 86, oder SEQ ID NO. 69 und 89, oder SEQ ID NO. 71 und 92, oder SEQ ID NO. 56 und 86, oder SEQ ID NO. 65 und 92 umfasst.

5. Erkennungsmolekül nach einem der Ansprüche 1 bis 4, **dadurch gekennzeichnet, dass** es ein single chain Antikörperfragment, ein Multibody, ein Fab-Fragment, ein Fusionsprotein aus einem Antikörperfragment mit Peptiden oder Proteinen und/oder ein Immunglobulin der Isotypen IgG, IgM, IgA, IgE, IgD und/oder deren Subklassen ist.

6. Konstrukt umfassend die Erkennungsmoleküle nach einem der Ansprüche 1 bis 5, **dadurch gekennzeichnet, dass** die Erkennungsmoleküle mit (i) Immunglobulindomänen verschiedener Spezies, (ii) Enzymmolekülen, (iii) Interaktionsdomänen, (iv) Domänen zur Stabilisierung, (v) Signalsequenzen, (vi) Fluoreszenzfarbstoffen, (vii) Toxinen, (viii) katalytischen Antikörpern, (ix) einem oder mehreren Antikörpern oder Antikörperfragmenten mit anderer Spezifität, (x) zytolytischen Komponenten, (xi) Immunmodulatoren, (xii) Immuneffektoren, (xiii) MHC-Klasse I oder Klasse II Antigenen, (xiv) Chelatoren zur radioaktiven Markierung, (xv) Radioisotopen, (xvi) Liposomen, (xvii) Transmembrandomänen, (xviii) Viren und/oder (xix) Zellen fusioniert, chemisch gekoppelt, kovalent oder nicht-kovalent assoziiert sind.

7. Verfahren zur Herstellung von Erkennungsmolekülen nach einem der Anspruche 1 bis 5 umfassend
(i) Einbringen eines oder mehrerer Nukleinsäuremoleküle, die die Aminosäuresequenz von mindestens einem Erkennungsmolekül nach einem der Ansprüche 1 bis 5 kodiert, in einen Virus oder in eine Wirtszelle;
(ii) Kultivierung der Wirtszellen oder des Virus unter geeigneten Bedingungen; und
(iii) Gewinnung des Erkennungsmoleküls, der Erkennungsmolekül tragenden Effektorzelle oder des Virus, das ein Core-1 Antigen spezifisch erkennt.

8. Verwendung eines Erkennungsmoleküls nach einem der Ansprüche 1 bis 5 oder eines Konstruktes nach Anspruch 6 zur Herstellung eines Arzneimittels und/oder Diagnostikums zur Prophylaxe, Prävention, Diagnose, Verminderung, Therapie, Verlaufskontrolle und/oder Nachbehandlung von Tumorerkrankungen und/oder Metastasen.

9. Verwendung nach Anspruch 8, wobei das Erkennungsmolekül ein nicht markiertes Erkennungsmolekül ist, das einem IgM oder IgG entspricht oder davon abgeleitet wurde.

10. Verwendung nach Anspruch 8, wobei die Erkennungsmoleküle Multibodies sind.

## Claims

1. A recognition molecule, **characterized in that** it comprises an amino acid sequence which contains
(i) the amino acid sequence SEQ ID NO. 1 and
(ii) the amino acid sequence SEQ ID NO. 2 or 3 and
(iii) the amino acid sequence SEQ ID NO. 4, 5 or 6 and specifically binds the core 1 antigen.

2. The recognition molecule according to claim 1,
**characterized in that** it further comprises an amino acid sequence which contains
(i) the amino acid sequence SEQ ID NO. 7 or 8 or 9 and
(ii) the amino acid sequence SEQ ID NO. 10 or 11 and
(iii) the amino acid sequence SEQ ID NO. 12 or 13 and specifically binds the core 1 antigen.

3. The recognition molecule according to claim 1 or 2, **characterized in that** the antibody framework sequences
a) FRH1, FRH2, FRH3 and FRH4 for the variable heavy chain V_{H} are the following amino acid sequences, the amino acid position corresponding to the numbering according to Kabat:
| | | |
|---|---|---|
| for FRH1 in position | 1 | Q or E |
| | 2 | V |
| | 3 | Q, K or T |
| | 4 | L |
| | 5 | K or V |
| | 6 | E or Q |
| | 7 | S |
| | 8 | G |
| | 9 | A |
| | 10 | E |
| | 11 | L or V |
| | 12 | V or K |
| | 13 | R or K |
| | 14 | P |
| | 15 | G |
| | 16 | T or A |
| | 17 | S |
| | 18 | V |
| | 19 | K |
| | 20 | I or V |
| | 21 | S or P |
| | 22 | C |
| | 23 | K |
| | 24 | A, V, S or T |
| | 25 | S |
| | 26 | G |
| | 27 | Y, F, S or D |
| | 28 | T |
| | 29 | F, L or I |
| | 30 | T |
| for FRH2 in position | 36 | W |
| | 37 | V |
| | 38 | K or R |
| | 39 | Q |
| | 40 | R or A |
| | 41 | P |
| | 42 | G |
| | 43 | H or Q |
| | 44 | G |
| | 45 | L |
| | 46 | E |
| | 47 | W or R |
| | 48 | I or M |
| | 49 | G |
| for FRH3 in position | 66 | K or R |
| | 67 | A or V |
| | 68 | T |
| | 69 | L or M |
| | 70 | T |
| | 71 | A, L or T |
| | 72 | D |
| | 73 | T |
| | 74 | S |
| | 75 | S or T |
| | 76 | S |
| | 77 | T |
| | 78 | A |
| | 79 | Y |
| | 80 | M |
| | 81 | Q or E |
| | 82 | L |
| | 82a | S |
| | 82b | S or R |
| | 82c | L |
| | 83 | T or R |
| | 84 | S |
| | 85 | E |
| | 86 | D |
| | 87 | S or T |
| | 88 | A |
| | 89 | V |
| | 90 | Y |
| | 91 | F or Y |
| | 92 | C |
| | 93 | A |
| | 94 | Y, K or R |
| for FRH4 in position | 103 | W |
| | 104 | G |
| | 105 | Q |
| | 106 | G |
| | 107 | T |
| | 108 | T, S or L |
| | 109 | V or L |
| | 110 | T |
| | 111 | V |
| | 112 | S |
| | 113 | S or A |
b) FRL1, FRL2, FRL3 and FRL4 for the variable light chain V_{L} are the following amino acid sequences, the amino acid position corresponding to the numbering according to Kabat:
| | | |
|---|---|---|
| for FRL1 in position | 1 | D |
| | 2 | I, V or L |
| | 3 | Q or L |
| | 4 | M |
| | 5 | T |
| | 6 | Q |
| | 7 | T or S |
| | 8 | P |
| | 9 | L |
| | 10 | S |
| | 11 | L |
| | 12 | P |
| | 13 | V |
| | 14 | S or T |
| | 15 | L or P |
| | 16 | G |
| | 17 | D or E |
| | 18 | Q or P |
| | 19 | A |
| | 20 | S |
| | 21 | I |
| | 22 | S |
| | 23 | C |
| for FRL2 in position | 35 | W |
| | 36 | Y |
| | 37 | L |
| | 38 | Q |
| | 39 | K |
| | 40 | P |
| | 41 | G |
| | 42 | Q |
| | 43 | S |
| | 44 | P |
| | 45 | K or Q |
| | 46 | L |
| | 47 | L |
| | 48 | I or V |
| | 49 | Y |
| for FRL3 in position | 57 | G |
| | 58 | V |
| | 59 | P |
| | 60 | D |
| | 61 | R |
| | 62 | F |
| | 63 | S |
| | 64 | G |
| | 65 | S |
| | 66 | G |
| | 67 | S |
| | 68 | G |
| | 69 | T |
| | 70 | D |
| | 71 | F |
| | 72 | T |
| | 73 | L |
| | 74 | K |
| | 75 | I |
| | 76 | S |
| | 77 | R |
| | 78 | V |
| | 79 | E |
| | 80 | A |
| | 81 | E |
| | 82 | D |
| | 83 | L or V |
| | 84 | G |
| | 85 | V |
| | 86 | Y |
| | 87 | Y |
| | 88 | C |
| for FRL4 in position | 98 | F |
| | 99 | G |
| | 100 | G or Q |
| | 101 | G |
| | 102 | T |
| | 103 | K |
| | 104 | L |
| | 105 | E |
| | 106 | I or L |
| | 106a | K |
| | 107 | R |
| | 108 | A |

4. The recognition molecule according to the preceding claim, **characterized in that** the recognition molecule comprises a combination of sequences SEQ ID Nos. 46 and 80, or SEQ ID Nos. 47 and 81, or SEQ ID Nos. 48 and 80, or SEQ ID Nos. 50 and 80, or SEQ ID Nos. 53 and 82, or SEQ ID Nos. 52 and 83, or SEQ ID Nos. 55 and 83, or SEQ ID Nos. 54 and 80, or SEQ ID Nos. 51 and 83, or SEQ ID Nos. 49 and 80, or SEQ ID Nos. 56 and 90, or SEQ ID Nos. 57 and 90, or SEQ ID Nos. 57 and 86, or SEQ ID Nos. 58 and 87, or SEQ ID Nos. 56 and 91, or SEQ ID Nos. 59 and 91, or SEQ ID Nos. 60 and 87, or SEQ ID Nos. 61 and 90, or SEQ ID Nos. 56 and 88, or SEQ ID Nos. 56 and 85, or SEQ ID Nos. 59 and 90, or SEQ ID Nos. 62 and 90, or SEQ ID Nos. 59 and 86, or SEQ ID Nos. 74 and 92, or SEQ ID Nos. 63 and 87, or SEQ ID Nos. 74 and 87, or SEQ ID Nos. 74 and 89, or SEQ ID Nos. 74 and 85, or SEQ ID Nos. 64 and 86, or SEQ ID Nos. 74 and 86, or SEQ ID Nos. 63 and 86, or SEQ ID Nos. 65 and 85, or SEQ ID Nos. 65 and 86, or SEQ ID Nos. 66 and 85, or SEQ ID Nos. 67 and 87, or SEQ ID Nos. 68 and 86, or SEQ ID Nos. 72 and 88, or SEQ ID Nos. 69 and 90, or SEQ ID Nos. 70 and 90, or SEQ ID Nos. 69 and 92, or SEQ ID Nos. 73 and 86, or SEQ ID Nos. 69 and 89, or SEQ ID Nos. 71 and 92, or SEQ ID Nos. 56 and 86, or SEQ ID Nos. 65 and 92.

5. The recognition molecule according to any of claims 1 to 4, **characterized in that** it is a single-chain antibody fragment, a multibody, a Fab fragment, a fusion protein of an antibody fragment with peptides or proteins and/or an immunoglobulin of the IgG, IgM, IgA, IgE, IgD isotypes and/or subclasses thereof.

6. A construct comprising the recognition molecules according to any of the claims 1 to 5, **characterized in that** the recognition molecules are fused, chemically coupled, covalently or non-covalently associated with (i) immunoglobulin domains of various species, (ii) enzyme molecules, (iii) interaction domains, (iv) domains for stabilization, (v) signal sequences, (vi) fluorescent dyes, (vii) toxins, (viii) catalytic antibodies, (ix) one or more antibodies or antibody fragments with different specificity, (x) cytolytic components, (xi) immunomodulators, (xii) immunoeffectors, (xiii) MHC class I or class II antigens, (xiv) chelating agents for radioactive labelling, (xv) radioisotopes, (xvi) liposomes, (xvii) transmembrane domains, (xviii) viruses and/or (xix) cells.

7. A method for the production of recognition molecules according to any of claims 1 to 5, comprising:
(i) incorporating one or more nucleic acid molecules encoding the amino acid sequences of at least one recognition molecule according to any of claims 1 to 5 in a virus or in a host cell;
(ii) culturing the host cells or the virus under suitable conditions; and
(iii) obtaining the recognition molecule, the effector cell bearing the recognition molecule, or the virus specifically recognizing a core 1 antigen.

8. Use of a recognition molecule according to any of claims 1 to 5 or of a construct according to claim 6 for the manufacture of a medicament and/or diagnostic agent for the prophylaxis, prevention, diagnosis, reduction, therapy, follow-up and/or aftercare of tumor diseases and/or metastases.

9. The use according to claim 8, wherein the recognition molecule is a non-labelled recognition molecule, which corresponds to an IgM or IgG or has been derived therefrom.

10. The use according to claim 8, wherein the recognition molecules are multibodies.

## Revendications

1. Molécule de reconnaissance, **caractérisée en ce qu'**elle comprend une séquence d'acides aminés, qui contient
(i) la séquence d'acides aminés SEQ ID NO 1 et
(ii) la séquence d'acides aminés SEQ ID NO 2 ou 3 et
(iii) la séquence d'acides aminés SEQ ID NO 4, 5 ou 6 et se fie spécifiquement à l'antigène Core-1.

2. Molécule de reconnaissance selon la revendication 1, **caractérisée en ce qu'**elle comprend en plus une séquence d'acides aminés, qui contient
(i) la séquence d'acides aminés SEQ ID NO 7 ou 8 ou 9, et
(ii) la séquence d'acides aminés SEQ ID NO 10 ou 11 et
(iii) la séquence d'acides aminés SEQ ID NO 12 ou 13, et se lie spécifiquement à l'antigène Core-1.

3. Molécule de reconnaissance selon la revendication 1 ou 2, **caractérisée en ce que** les séquences du squelette d'anticorps
a) FRH1, FRH2, FRH3 et FRH4 pour la chaîne lourde variable VH sont les séquences d'acides aminés suivantes, dans lesquelles la position d'acide aminé correspond à la numération selon Kabat :
| | | |
|---|---|---|
| pour FRH1 en position | 1 | Q au E |
| | 2 | V |
| | 3 | Q, K ou T |
| | 4 | L |
| | 5 | K ou V |
| | 6 | E ou Q |
| | 7 | S |
| | 8 | G |
| | 9 | A |
| | 10 | E |
| | 11 | LouV |
| | 12 | V ou K |
| | 13 | R ou K |
| | 14 | P |
| | 15 | G |
| | 16 | T ou A |
| | 17 | S |
| | 18 | V |
| | 19 | K |
| | 20 | I ou V |
| | 21 | S ou P |
| | 22 | C |
| | 23 | K |
| | 24 | A, V, S ou T |
| | 25 | S |
| | 26 | G |
| | 27 | Y, F, S ou D |
| | 28 | T |
| | 29 | F, L, ou I |
| | 30 | T |
| pour FRH2 en position | 36 | W |
| | 37 | V |
| | 38 | K ou R |
| | 39 | Q |
| | 40 | R ou A |
| | 41 | P |
| | 42 | G |
| | 43 | H ou Q |
| | 44 | G |
| | 45 | L |
| | 46 | E |
| | 47 | W ou R |
| | 48 | I ou M |
| | 49 | G |
| pour FRH3 en position | 66 | K ou R |
| | 67 | A ou V |
| | 68 | T |
| | 69 | L ou M |
| | 70 | T |
| | 71 | A, LouT |
| | 72 | D |
| | 73 | T |
| | 74 | S |
| | 75 | SouT |
| | 76 | S |
| | 77 | T |
| | 78 | A |
| | 79 | Y |
| | 80 | M |
| | 81 | QouE |
| | 82 | L |
| | 82a | S |
| | 82b | S ou R |
| | 82c | L |
| | 83 | T ou R |
| | 84 | S |
| | 85 | E |
| | 86 | D |
| | 87 | SouT |
| | 88 | A |
| | 89 | V |
| | 90 | Y |
| | 91 | F ou Y |
| | 92 | C |
| | 93 | A |
| | 94 | Y, K ou R |
| pour FRH4 en position | 103 | W |
| | 104 | G |
| | 105 | Q |
| | 106 | G |
| | 107 | T |
| | 108 | T, S ou L |
| | 109 | V ou L |
| | 110 | T |
| | 111 | V |
| | 112 | S |
| | 113 | S ou A |
b) FRL1, FRL2, FRL3 et FRL4 pour la chaîne légère variable VL sont les séquences d'acides aminés suivantes, dans lesquelles la position d'acide aminé correspond à la numérotation selon Kabat :
| | | |
|---|---|---|
| pour FRL1 en position | 1 | D |
| | 2 | I, V ou L |
| | 3 | Q ou L |
| | 4 | M |
| | 5 | T |
| | 6 | Q |
| | 7 | T ou S |
| | 8 | P |
| | 9 | L |
| | 10 | S |
| | 11 | L |
| | 12 | P |
| | 13 | V |
| | 14 | SouT |
| | 15 | L ou P |
| | 16 | G |
| | 17 | D ou E |
| | 18 | QouP |
| | 19 | A |
| | 20 | S |
| | 21 | I |
| | 22 | S |
| | 23 | C |
| pour FRL2 en position | 35 | W |
| | 36 | Y |
| | 37 | L |
| | 38 | Q |
| | 39 | K |
| | 40 | P |
| | 41 | G |
| | 42 | Q |
| | 43 | S |
| | 44 | P |
| | 45 | K ou Q |
| | 46 | L |
| | 47 | L |
| | 48 | I ou V |
| | 49 | Y |
| pour FRL3 en position | 57 | G |
| | 58 | V |
| | 59 | P |
| | 60 | D |
| | 61 | R |
| | 62 | F |
| | 63 | S |
| | 64 | G |
| | 65 | S |
| | 66 | G |
| | 67 | S |
| | 68 | G |
| | 69 | T |
| | 70 | D |
| | 71 | F |
| | 72 | T |
| | 73 | L |
| | 74 | K |
| | 75 | I |
| | 76 | S |
| | 77 | R |
| | 78 | V |
| | 79 | E |
| | 80 | A |
| | 81 | E |
| | 82 | D |
| | 83 | LouV |
| | 84 | G |
| | 85 | V |
| | 86 | Y |
| | 87 | Y |
| | 88 | C |
| pour FRL4 en position | 98 | F |
| | 99 | G |
| | 100 | G ou Q |
| | 101 | G |
| | 102 | T |
| | 103 | K |
| | 104 | L |
| | 105 | E |
| | 106 | I ou L |
| | 106a | K |
| | 107 | R |
| | 108 | A. |

4. Molécule de reconnaissance selon la revendication précédente, **caractérisée en ce que** la molécule de reconnaissance comprend une combinaison des séquences SEQ ID NO 46 et 80, ou SEQ ID NO 47 et 81, ou SEQ ID NO 48 et 80, ou SEQ ID NO 50 et 80, ou SEQ ID NO 53 et 82, ou SEQ ID NO 52 et 83, ou SEQ ID NO 55 et 83, ou SEQ ID NO 54 et 80, ou SEQ ID NO 51 et 83, ou SEQ ID NO 49 et 80, ou SEQ ID NO 56 et 90, ou SEQ ID NO 57 et 90, ou SEQ ID NO 57 et 86, ou SEQ ID NO 58 et 87, ou SEQ ID NO 56 et 91, ou SEQ ID NO 59 et 91, ou SEQ ID NO 60 et 87, ou SEQ ID NO 61 et 90, ou SEQ ID NO 56 et 88, ou SEQ ID NO 56 et 85, ou SEQ ID NO 59 et 90, ou SEQ ID NO 62 et 90, ou SEQ ID NO 59 et 86, ou SEQ ID NO 74 et 92, ou SEQ ID NO 63 et 87, ou SEQ ID NO 74 et 87, ou SEQ ID NO 74 et 89, ou SEQ ID NO 74 et 85, ou SEQ ID NO 64 et 86, ou SEQ ID NO 74 et 86, ou SEQ ID NO 63 et 86, ou SEQ ID NO 65 et 85, ou SEQ ID NO 65 et 86, ou SEQ ID NO 66 et 85, ou SEQ ID NO 67 et 87, ou SEQ ID NO 68 et 86, ou SEQ ID NO 72 et 88, ou SEQ ID NO 69 et 90, ou SEQ ID NO 70 et 90, ou SEQ ID NO 69 et 92, ou SEQ ID NO 73 et 86, ou SEQ ID NO 69 et 89, ou SEQ ID NO 71 et 92, ou SEQ ID NO 56 et 86, ou SEQ ID NO 65 et 92.

5. Molécule de reconnaissance selon l'une des revendications 1 à 4, **caractérisée en ce que** c'est un fragment d'anticorps à une seule chaîne, un multicorps, un fragment Fab, une protéine de fusion d'un fragment d'anticorps avec des peptides ou des protéines et/ou une immunoglobuline des classes IgG, IgM, IgA, IgE, IgD et/ou de leurs sous-classes.

6. Construction comprenant les molécules de reconnaissance selon l'une des revendications 1 à 5, **caractérisé en ce que** les molécules de reconnaissance sont fusionnées, couplées chimiquement et/ou associées par une liaison covalente ou non covalente avec (i) des domaines d'immunoglobuline de différente espèce, (ii) des molécules d'enzyme, (iii) des domaines d'interaction, (iv) des domaines de stabilisation (v), des séquences signal, (vi) des colorants de fluorescence, (vii) des toxines, (viii) des anticorps catalytiques, (ix) un ou plusieurs anticorps ou fragments d'anticorps ayant une autre spécificité, (x) des composants cytolytiques, (xi) des immunomodulateurs, (xii) des immunoeffecteurs, (xiii), des antigènes MHC de classe I ou de classe II, (xiv) des chélateurs de marquage radioactif, (xv) des radioisotopes, (xvi) des liposomes, (xvii) des domaines transmembranaires, (xviii) des virus et/ou (xix) des cellules.

7. Procédé de préparation de molécules de reconnaissance selon l'une des revendications 1 à 5, comprenant :
(i) l'incorporation d'une ou plusieurs molécule(s) d'acide nucléique, qui code la séquence d'acides aminés d'au moins une molécule de reconnaissance selon l'une des revendications 1 à 5, dans un virus ou une cellule hôte,
(ii) la culture des cellules hôtes ou du virus dans des conditions appropriées, et
(iii) l'obtention de la molécule de reconnaissance, de la cellule effectrice ou du virus portant la molécule de reconnaissance, qui reconnaît spécifiquement l'antigène Core-1.

8. Utilisation d'une molécule de reconnaissance selon l'une des revendications 1 à 5 ou d'une construction selon la revendication 6 pour préparer un médicament et/ou un diagnostic destiné à la prophylaxie, la prévention, le diagnostic, la diminution, la thérapie, le contrôle de suivi et/ou le traitement consécutif des maladies tumorales et/ou des métastases.

9. Utilisation selon la revendication 8, dans laquelle la molécule de reconnaissance est une molécule de reconnaissance non marquée, qui correspond à une IgM ou une IgG, ou qui a été dérivée d'entre elles,

10. Utilisation selon la revendication 8, dans laquelle les molécules de reconnaissance sont des multicorps.
